# EUROPÄISCHE PATENTSCHRIFT

(11) **EP 0 696 584 B1**
(45) Veröffentlichungstag und Bekanntmachung des Hinweises auf die Patenterteilung: **20.01.1999**
(21) Anmeldenummer: 95112277.9
(22) Anmeldetag: 04.08.1995
(51) Int. Cl.: C07D 263/14, A01N 43/76, C07C 43/205, C07C 49/84, C07C 251/48, C07C 233/67

(54) **Substituierte Biphenyloxazoline und ihre Verwendung zur Bekämpfung von tierischen Schädlingen**
Substituted biphenyloxazolines and their use as fungicides
Biphenyloxazolines substitués et leur application comme pesticides

(30) Priorität: 12.08.1994 DE 4428536; 12.12.1994 DE 4444108
(43) Veröffentlichungstag der Anmeldung: 14.02.1996
(73) Patentinhaber: BAYER AG, 51368 Leverkusen (DE); YASHIMA CHEMICAL INDUSTRY CO., LTD., Kawasaki-shi, Kanagawa 213 (JP)
(72) Erfinder: Lantzsch, Reinhard, Dr., D-42115 Wuppertal (DE); Marhold, Albrecht, Dr., D-51373 Leverkusen (DE); Krämer, Wolfgang, Dr., D-51399 Burscheid (DE); Erdelen, Christoph, Dr., D-42799 Leichlingen (DE); Wachendorff-Neumann, Ulrike, Dr., D-53177 Bonn (DE); Turberg, Andreas, Dr., D-40699 Erkrath (DE); Mencke, Norbert, Dr., D-51381 Leverkusen (DE)
(74) Vertreter: Linkenheil, Dieter

(56) Entgegenhaltungen:
- EP-A- 0 432 661
- EP-A- 0 645 085
- US-A- 4 960 884
- SYNTHESIS, Nr. 5, 1985 Seiten 499-500, H. SUZUKI ET AL. 'Copper(I)-Assisted Synthesis of Aryl 2,2,2-Trifluoroethyl Ethers'
- SYNLETT, Bd. 4, 1994 Seiten 251-252, M. KUROBOSHI; T HIYAMA 'A Facile Synthesis of alpha-alpha-Difluoroalkyl Ethers and Carbonyl Fluoride Acetals by Oxidative Desulfurisation-Fluorination'

## Beschreibung

Die Erfindung betrifft neue substituierte Biphenyloxazoline, mehrere Verfahren zu ihrer Herstellung, neue Zwischenprodukte und die Verwendung der substituierten Biphenyloxazoline zur Bekämpfung von tierischen Schädlingen.

Es ist bekannt, daß bestimmte substituierte Biphenyloxazoline, wie 2-(2,6-Difluorphenyl)-4-(4'-chlorbiphenyl-4)-2-oxazolin, insektizid und akarizid wirksam sind (vgl. EP-A-0 432 661 und EP-A-0 645 085).

Die Wirkungshöhe und/oder Wirkungsdauer dieser bekannten Verbindungen ist jedoch, insbesondere gegen bestimmte Organismen oder bei niedrigen Anwendungskonzentrationen nicht in allen Anwendungsgebieten völlig zufriedenstellend.

Es wurden neue substituierte Biphenyloxazoline der Formel (I) 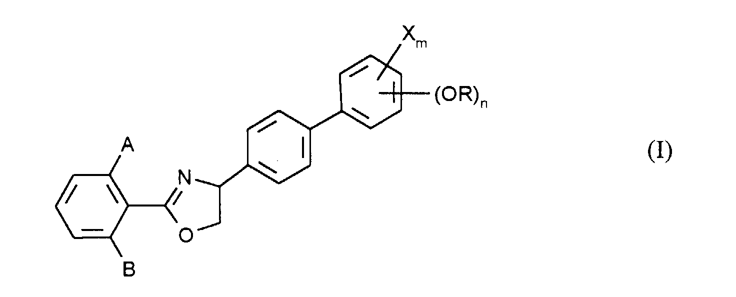 gefunden,
in welcher
- A: für Wasserstoff, Fluor oder Chlor steht,
- B: für Fluor oder Chlor steht,
- R: für R¹ oder R² steht, wobei
- R¹: für C₁-C₆-Halogenalkyl oder C₃-C₆-Halogencycloalkyl mit jeweils mindestens einem Fluoratom und zusätzlich mindestens einem Wasserstoff- oder Chloratom oder für C₄-C₆-Halogencycloalkenyl mit mindestens einem Fluoratom steht,
- R²: für Wasserstoff, C₃-C₁₂-Alkenyl, C₃-C₁₂-Alkinyl, für gegebenenfalls durch C₁-C₄-Alkyl, C₂-C₄-Alkenyl, C₂-C₄-Halogenalkenyl, Phenyl, Halogenphenyl, Styryl oder Halogenstyryl substituiertes C₃-C₆-Cycloalkyl,
für gegebenenfalls durch Halogen, C₁-C₄-Alkyl, C₂-C₄-Alkenyl, C₂-C₄-Halogenalkenyl, Phenyl, Halogenphenyl, Styryl oder Halogenstyryl substituiertes C₃-C₆-Cycloalkyl-C₁-C₄-alkyl,
für gegebenenfalls durch Halogen oder C₁-C₄-Alkyl substituiertes C₄-C₆-Cycloalkenyl-C₁-C₄-alkyl,
für gegebenenfalls durch C₁-C₄-Alkyl substituiertes C₄-C₆-Cycloalkenyl,
für jeweils gegebenenfalls einfach bis vierfach, gleich oder verschieden durch Nitro, Halogen, C₁-C₁₂-Alkyl, C₁-C₁₂-Halogenalkyl, C₁-C₁₂-Alkoxy oder C₁-C₁₂-Halogenalkoxy substituiertes Phenyl-C₁-C₆-alkyl, Naphthyl-C₁-C₃-alkyl oder Tetrahydronaphthyl-C₁-C₃-alkyl,
für gegebenenfalls einfach oder zweifach, gleich oder verschieden durch Nitro, Halogen, C₁-C₁₂-Alkyl, C₁-C₁₂-Halogenalkyl, C₁-C₁₂-Alkoxy oder C₁-C₁₂-Halogenalkoxy substituiertes 5- oder 6-gliedriges Heteroaryl-C₁-C₄-alkyl mit 1 oder 2 gleichen oder verschiedenen Heteroatomen aus der Reihe Stickstoff, Sauerstoff und Schwefel
oder für den Rest COR³ steht,
- R³: für C₁-C₁₂-Alkyl, C₁-C₁₂-Alkoxy, C₃-C₁₂-Alkenyl, C₃-C₁₂-Alkenyloxy, für jeweils gegebenenfalls durch Halogen, C₁-C₄-Alkyl, C₁-C₄-Halogenalkyl, C₂-C₄-Alkenyl, C₂-C₄-Halogenalkenyl substituiertes C₃-C₁₀-Cycloalkyl, C₃-C₁₀-Cycloalkyloxy oder C₃-C₁₀-Cycloalkyl-C₁-C₆-alkyloxy,
für jeweils gegebenenfalls einfach bis vierfach, gleich oder verschieden durch Halogen, C₁-C₁₂-Alkyl, C₁-C₁₂-Halogenalkyl, C₁-C₁₂-Alkoxy oder C₁-C₁₂-Halogenalkoxy substituiertes Phenyl oder Naphthyl oder für den Rest NR⁴R⁵ steht,
- R⁴: für Wasserstoff oder C₁-C₁₂-Alkyl steht,
- R⁵: für C₁-C₁₂-Alkyl, C₁-C₁₂-Halogenalkyl, für jeweils gegebenenfalls durch Halogen, C₁-C₄-Alkyl, C₁-C₄-Halogenalkyl oder C₂-C₄-Halogenalkenyl substituiertes C₃-C₁₀-Cycloalkyl oder C₃-C₁₀-Cycloalkyl-C₁-C₆-alkyl oder für jeweils gegebenenfalls einfach bis vierfach, gleich oder verschieden durch Halogen, C₁-C₁₂-Alkyl, C₁-C₁₂-Halogenalkyl, C₁-C₁₂-Alkoxy oder C₁-C₁₂-Halogenalkoxy substituiertes Phenyl oder Phenyl-C₁-C₆-alkyl steht,
- X: für Halogen, C₁-C₆-Alkyl oder C₁-C₆-Alkoxy steht,
- m: für 0, 1 oder 2 steht und
- n: für 1 oder 2 steht,
ausgenommen 2-(2,6-Difluorphenyl)-4-[4'-(2,2,2-trifluorethoxy)-biphenyl-4]-2-oxazolin und 2-(2-Chlor-6-fluorphenyl)-4-[4'-(2,2,2-trifluorethoxy)-biphenyl-4]-2-oxazolin.

Aufgrund eines oder mehrerer Chiralitätszentren fallen die Verbindungen der Formel (I) im allgemeinen als Stereoisomerengemische an. Sie können sowohl in Form ihrer Diastereomerengemische als auch als reine Diastereomere oder Enantiomere verwendet werden.

Weiter wurde gefunden, daß man die neuen substituierten Biphenyloxazoline der Formel (I) erhält, wenn man
A) in einer ersten Stufe zum Erhalt von Verbindungen der Formel (II) 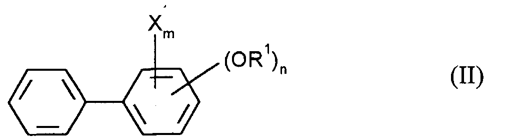 in welcher
   - R¹, X, m und n: die oben angegebene Bedeutung haben,

   α) zum Erhalt von Verbindungen der Formel (IIa) 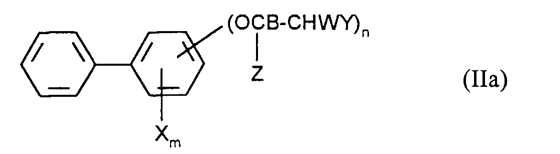 in welcher
      - W und Y: unabhängig voneinander für Fluor, Chlor oder Trifluormethyl stehen,
      - B: für Wasserstoff oder Fluor steht,
      - Z: für Fluor steht oder
      - W und Z: gemeinsam für -(CF₂)₁ stehen,
      worin
      - l: für 2, 3 oder 4 steht,
      - n: für 1 oder 2 steht und
      - m: für 0, 1 oder 2 steht,
      ein Hydroxybiphenyl der Formel (III) 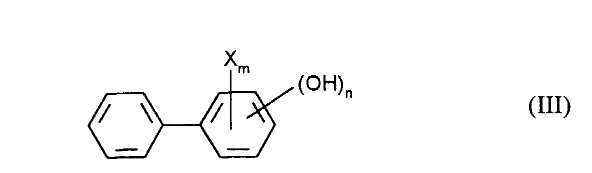 in welcher
      - X, m und n: die oben angegebene Bedeutung haben,
      mit einer Verbindung der Formel (IV) 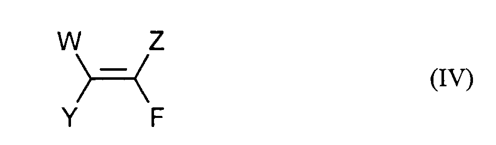 in welcher
      - W, Y und Z: die oben angegebene Bedeutung haben,
      gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert,
      oder
   β) zum Erhalt von Verbindungen der Formel (IIb) 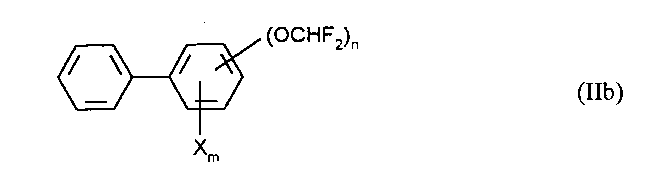 in welcher
      - X, m und n: die oben angegebene Bedeutung haben,
      Hydroxybiphenyle der obengenannten Formel (III) mit einem Difluorhalogenmethan der Formel (V)

      CHF₂Hal (V)

      in welcher
      - Hal: für Chlor oder Brom steht,
      gegebenenfalls in Gegenwart einer Base, gegebenenfalls in Gegenwart eines Katalysators und gegebenenfalls in Gegenwart eines Verdünnungsmittels umsetzt,
      oder
   γ) zum Erhalt von Verbindungen der Formel (IIc) 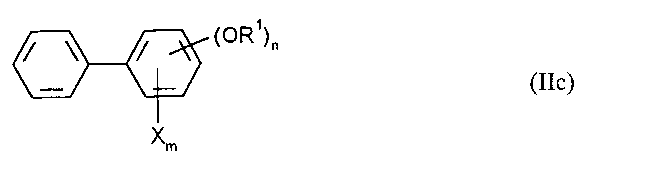 in welcher
      - X, R¹, m und n: die oben angegebene Bedeutung haben,
      Aminophenolderivate der Formel (VI) 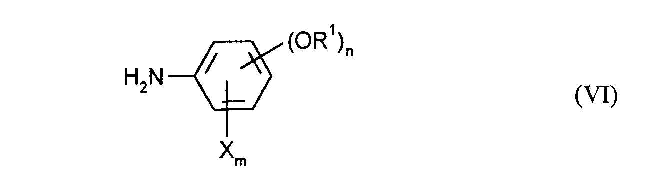 in welcher
      - X, R¹, m und n: die oben angegebene Bedeutung haben,
      diazotiert und das entstandene Diazoniumsalz mit Benzol in Gegenwart von Säure und Eisenpulver oder in Gegenwart einer Base und jeweils gegebenenfalls in Gegenwart eines Verdünnungsmittels umsetzt,
      oder
   δ) zum Erhalt von Verbindungen der Formel (IId) 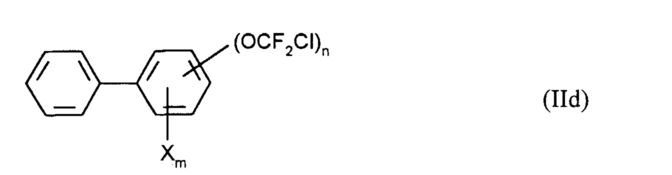 in welcher
      - X, m und n: die oben angegebene Bedeutung haben,
      ein Hydroxybiphenyl der obengenannten Formel (III) mit Tetrachlorkohlenstoff in Gegenwart von Fluorwasserstoffsäure, gegebenenfalls in Gegenwart eines Verdünnungmittels umsetzt,
B) in einer zweiten Stufe zum Erhalt von Verbindungen der Formel (VII) 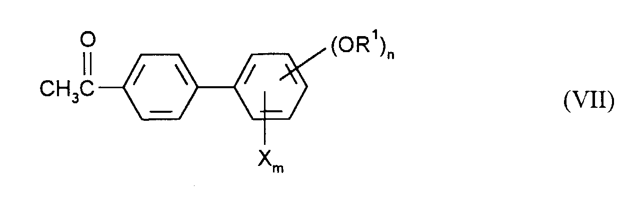 in welcher
   - X, R¹, m und n: die oben angegebene Bedeutung haben,

   α) die nach Verfahren A) erhältlichen Verbindungen der Formel (II) in Gegenwart einer Säure oder Lewis-Säure und in Gegenwart eines Verdünnungsmittels mit Acetylchlorid umsetzt,
      oder
   β) zum Erhalt von Verbindungen der Formel (VIIa) 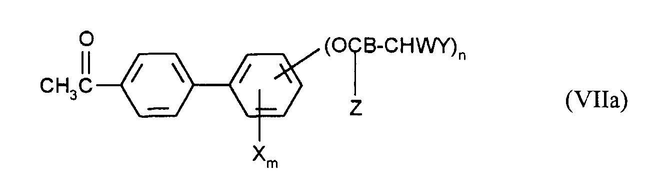 in welcher
      - B, X, m, n, W, Y und Z: die oben angegebene Bedeutung haben,
      ein Hydroxybiphenylderivat der Formel (IIIa) 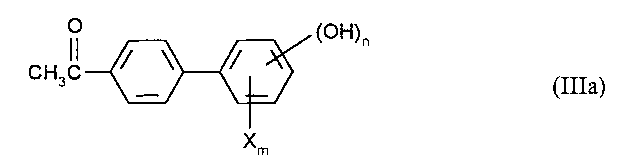 in welcher
      - X, m und n: die oben angegebene Bedeutung haben,
      mit einer Verbindung der Formel (IV) 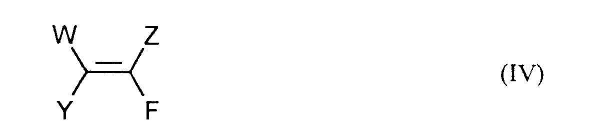 in welcher
      - W, Y und Z: die oben angegebene Bedeutung haben,
      gegebenenfalls in Gegenwart einer Base, gegebenenfalls in Gegenwart eines Katalysators und gegebenenfalls in Gegenwart eines Lösungsmittels umsetzt und gegebenenfalls anschließend hydriert,
      oder
   γ) zum Erhalt von Verbindungen der Formel (VIIb) 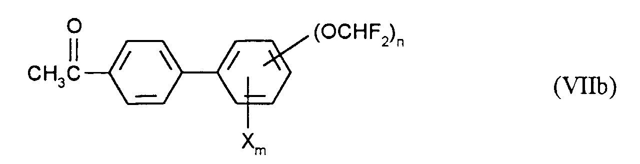 in welcher
      - X, m und n: die oben angegebene Bedeutung haben,
      ein Hydroxybiphenylderivat der oben gezeigten Formel (IIIa) mit einem Difluorhalogenmethan der oben gezeigten Formel (V) gegebenenfalls in Gegenwart einer Base, gegebenenfalls in Gegenwart eines Katalysators und gegebenenfalls in Gegenwart eines Verdünnungsmittels umsetzt,
C) in einer dritten Stufe zum Erhalt von Verbindungen der Formel (VIII) 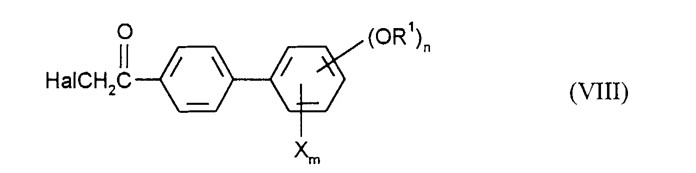 in welcher
   - X, R¹, m und n: die oben angegebene Bedeutung haben und
   - Hal: für Chlor oder Brom steht,

   α) die nach Verfahren B) erhältlichen Verbindungen der oben gezeigten Formel (VII) gegebenenfalls in Gegenwart eines Verdünnungsmittels chloriert oder bromiert,
      oder
   β) die nach Verfahren A) erhältlichen Verbindungen der oben gezeigten Formel (II) mit Halogenacetylchloriden der Formel (IX)

      HalCH₂COCl (IX)

      in welcher
      - Hal: für Chlor oder Brom steht,
      in Gegenwart einer Säure oder Lewis-Säure und in Gegenwart eines Verdünnungsmittels umsetzt,
D) in einer vierten Stufe zum Erhalt von Verbindungen der Formel (X) 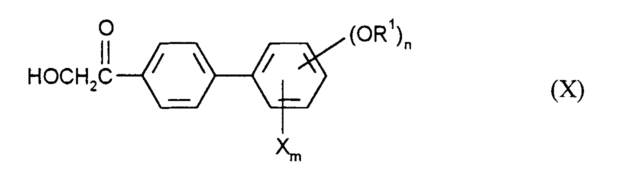 in welcher
   - X, R¹, m und n: die oben angegebene Bedeutung haben,
   die nach Verfahren C) erhältlichen Verbindungen der oben gezeigten Formel (VIII) mit einem Salz der Ameisensäure, in Gegenwart eines Verdünnungsmittels und gegebenenfalls in Gegenwart eines Katalysators umsetzt,
E) in einer fünften Stufe zum Erhalt von Verbindungen der Formel (XI) 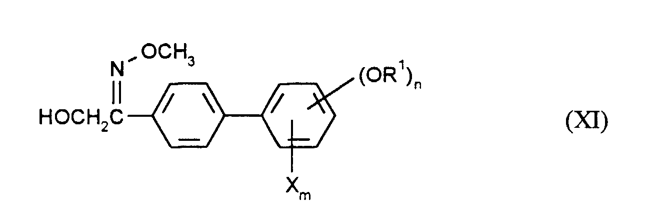 in welcher
   - X, R¹, m und n: die oben angegebene Bedeutung haben,
   die nach Verfahren D) erhältlichen Verbindungen der oben gezeigten Formel (X) mit der Verbindung der Formel (XII)

   H₂N-OCH₃ (XII)

   gegebenenfalls in Gegenwart eines Verdünnungsmittels umsetzt,
F) in einer sechsten Stufe zum Erhalt von Verbindungen der Formel (XIII) 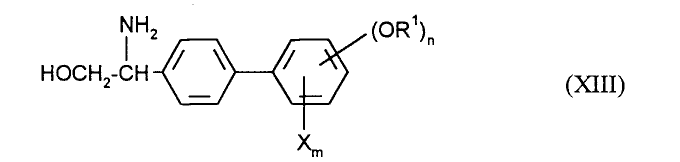 in welcher
   - R¹, X, m und n: die oben angegebene Bedeutung haben,
   die nach Verfahren E) erhältlichen Verbindungen der oben gezeigten Formel (XI) mit einem Reduktionsmittel in Gegenwart einer Säure und gegebenenfalls in Gegenwart eines Verdünnungsmittels reduziert,
G) in einer siebten Stufe zum Erhalt von Verbindungen der Formel (XIV) 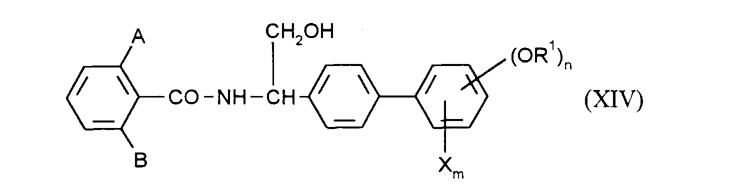 in welcher
   - A, B, R¹, X, m und n: die oben angegebene Bedeutung haben,
   entweder
   α) die nach Verfahren F) erhältlichen Verbindungen der oben gezeigten Formel (XIII) mit einem 2-A,6-B-Benzoylchlorid, gegebenenfalls in Gegenwart einer Base und gegebenenfalls in Gegenwart eines Verdünnungsmittels umsetzt
      oder
   β) zunächst die nach Verfahren A) erhältlichen Verbindungen der oben gezeigten Formel (II) mit einer Verbindung der Formel (XV) 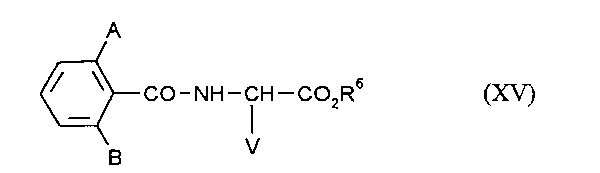 in welcher
      - A und B: die oben angegebene Bedeutung haben,
      - V: für Chlor, Hydroxy oder C₁-C₄-Alkoxy steht und
      - R⁶: für Wasserstoff oder Alkyl, bevorzugt Wasserstoff oder C₁-C₆-Alkyl, steht,
      in Gegenwart eines sauren Katalysators und gegebenenfalls in Gegenwart eines Verdünnungsmittels umsetzt und
      die so erhaltenen Verbindungen der Formel (XVI) 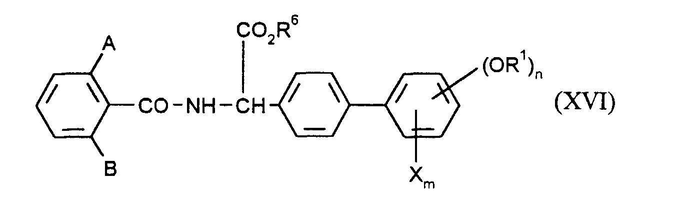 in welcher
      - A, B, R¹, X, m, n und R⁶: die oben angegebene Bedeutung haben,
      mit einem Reduktionsmittel in Gegenwart eines Verdünnungsmittels reduziert,
H) in einer achten Stufe zum Erhalt von Verbindungen der Formel (XVII) 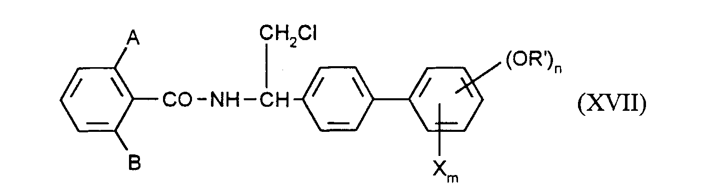 in welcher
   - A, B, X, m und n: die oben angegebene Bedeutung haben,
   - R': für R¹ oder COR³ steht, worin
   R¹ und R³ die obengenannte Bedeutung haben,
   entweder
   α) die nach Verfahren G) erhältlichen Verbindungen der oben gezeigten Formel (XIV) mit einem Chlorierungsmittel, gegebenenfalls in Gegenwart eines Verdünnungsmittels umsetzt
      oder
   β) die nach Verfahren A) erhältlichen Verbindungen der oben gezeigten Formel (II) oder eine Verbindung der Formel (IIe) 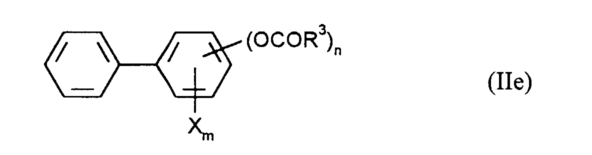 in welcher
      - R³, X, n und m: die oben angegebene Bedeutung haben,
      mit einer Verbindung der Formel (XVIII) 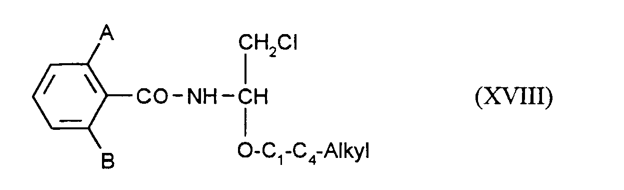 in welcher
      - A und B: die oben angegebene Bedeutung haben,
      in Gegenwart eines sauren Katalysators und gegebenenfalls in Gegenwart eines Verdünnungsmittels umsetzt,
      und
I) in einer neunten Stufe die nach Verfahren H) erhältlichen Verbindungen der oben gezeigten Formel (XVII) in Gegenwart einer Base, gegebenenfalls in Gegenwart eines Katalysators und gegebenenfalls in Gegenwart eines Verdünnungsmittels cyclisiert,
   und gegebenenfalls
J) die dabei für R = COR³ erhaltenen Verbindungen der Formel (Ia) 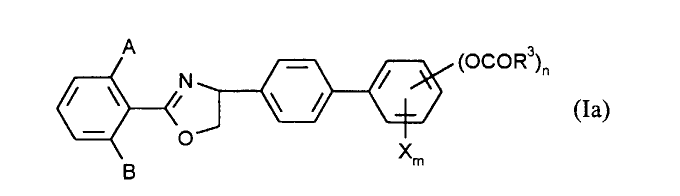 in welcher
   - A, B, R³, X, n und m: die oben angegebene Bedeutung haben,
   zu Verbindungen der Formel (Ib) 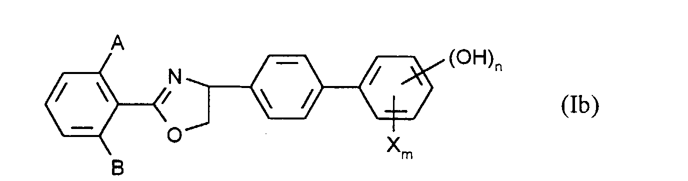 in welcher
   - A, B, X, n und m: die oben angegebene Bedeutung haben,
   verseift und diese anschließend gegebenenfalls
K
   α) mit einer Verbindung der Formel (XIX)

      HalCOR³ (XIX)

      in welcher
      - R³: die obengenannte Bedeutung hat und
      - Hal: für Halogen, bevorzugt Chlor oder Brom steht, gegebenenfalls in Gegenwart eines Verdünnungsmittels und gegebenenfalls in Gegenwart einer Base umsetzt, oder
   β) mit einer Verbindung der Formel (IV) 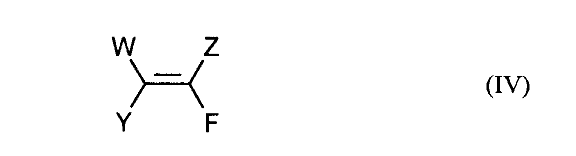 in welcher
      - W, Y und Z: die oben angegebene Bedeutung haben,
      gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert,
      oder
   γ) mit einem Difluorhalogenmethan der Formel (V)

      CHF₂Hal (V)

      in welcher
      - Hal: für Chlor oder Brom steht,
      gegebenenfalls in Gegenwart einer Base, gegebenenfalls in Gegenwart eines Katalysators und gegebenenfalls in Gegenwart eines Verdünnungsmittels umsetzt,
      oder
   δ) mit Tetrachlorkohlenstoff in Gegenwart von Fluorwasserstoffsäure, gegebenenfalls in Gegenwart eines Verdünnungsmittels umsetzt, oder
   ε) mit einer Verbindung der Formel (XX)

      M-R² (XX)

      in welcher
      - R²: die obengenannte Bedeutung hat und
      - M: für eine Abgangsgruppe steht, gegebenenfalls in Gegenwart eines Verdünnungsmittels und gegebenenfalls in Gegenwart einer Base umsetzt.

Weiter wurde gefunden, daß die neuen substituierten Biphenyloxazoline der Formel (I) sehr gut zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- A: steht bevorzugt für Wasserstoff, Fluor oder Chlor.
- B: steht bevorzugt für Fluor oder Chlor.
- R: steht bevorzugt für R¹ oder R², wobei
- R¹: für C₁-C₃-Halogenalkyl oder C₄-C₅-Halogencycloalkyl mit jeweils mindestens einem Fluoratom und zusätzlich mindestens einem Wasserstoff- oder Chloratom oder für C₄-C₆-Halogencycloalkenyl mit mindestens einem Fluoratom steht und
- R²: für Wasserstoff, C₃-C₁₂-Alkenyl, C₃-C₅-Alkinyl, für gegebenenfalls durch C₁-C₄-Alkyl, C₂-C₄-Alkenyl, C₂-C₃-Halogenalkenyl, Phenyl, Halogenphenyl, Styryl oder Halogenstyryl substituiertes C₃-C₆-Cycloalkyl,
für gegebenenfalls durch Halogen, C₁-C₄-Alkyl, C₂-C₄-Alkenyl, C₂-C₃-Halogenalkenyl, Phenyl, Halogenphenyl, Styryl oder Halogenstyryl substituiertes C₃-C₆-Cycloalkyl-C₁-C₄-alkyl,
für gegebenenfalls durch Halogen substituiertes C₄-C₆-Cycloalkenylmethyl, für C₄-C₆-Cycloalkenyl,
für jeweils gegebenenfalls einfach oder zweifach, gleich oder verschieden durch Nitro, Halogen, C₁-C₄-Alkyl, C₁-C₄-Halogenalkyl, C₁-C₄-Alkoxy oder C₁-C₄-Halogenalkoxy substituiertes Phenyl-C₁-C₆-alkyl, Naphthylmethyl, Tetrahydronaphthylmethyl, Pyridyl-, Furanyl-, Thiazolyl-, Oxazolyl- oder Isoxazolyl-C₁-C₃-alkyl oder für den Rest COR³ steht.
- R³: steht bevorzugt für C₁-C₆-Alkyl, C₁-C₆-Alkoxy, C₃-C₆-Alkenyl, C₃-C₆-Alkenyloxy, für jeweils gegebenenfalls durch Fluor, Chlor, C₁-C₃-Alkyl, C₁-C₂-Halogenalkyl, C₂-C₃-Halogenalkenyl substituiertes C₃-C₆-Cycloalkyl, C₃-C₆-Cycloalkyloxy oder C₃-C₆-Cycloalkyl-C₁-C₂-alkyloxy,
für gegebenenfalls einfach oder zweifach, gleich oder verschieden durch Halogen, C₁-C₄-Alkyl, C₁-C₃-Halogenalkyl, C₁-C₄-Alkoxy oder C₁-C₄-Halogenalkoxy substituiertes Phenyl oder für den Rest NHR⁵.
- R⁵: steht bevorzugt für C₁-C₄-Alkyl oder für jeweils gegebenenfalls einfach oder zweifach, gleich oder verschieden durch Fluor, Chlor, Brom, C₁-C₄-Alkyl, C₁-C₄-Halogenalkyl oder C₁-C₄-Halogenalkoxy substituiertes Phenyl oder Benzyl.
- X: steht bevorzugt für Fluor, Chlor oder Brom.
- m: steht bevorzugt für 0 oder 1.
- n: steht bevorzugt für 1.
- A: steht besonders bevorzugt für Wasserstoff, Fluor oder Chlor.
- B: steht besonders bevorzugt für Fluor oder Chlor.
- R: steht besonders bevorzugt für R¹ oder R², wobei
- R¹: für eine der Gruppen -CHF₂, -CClF₂,
-CF₂CHFCl, -CF₂CH₂F, -CF₂CHF₂, -CF₂CCl₃, -CF₂CHFCF₃, -CH₂CF₃, -CH₂CF₂CHF₂, -CH₂CF₂CF₃, 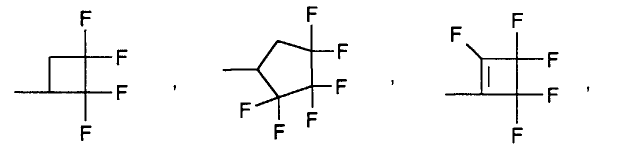 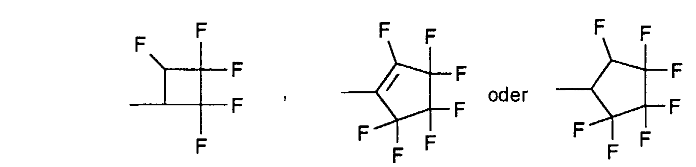 steht und
- R²: für Wasserstoff, für Propenyl, Butenyl, Pentenyl, Hexenyl, Propinyl, Butinyl, Pentinyl,
für eine der Cycloalkylalkylgruppierungen: 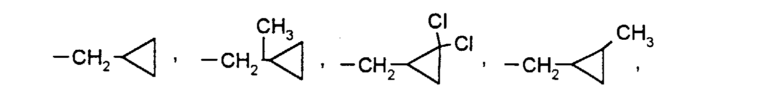 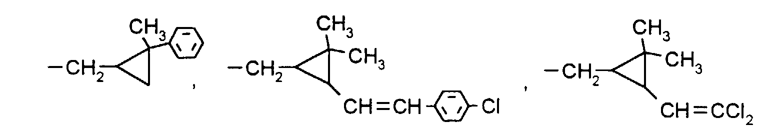 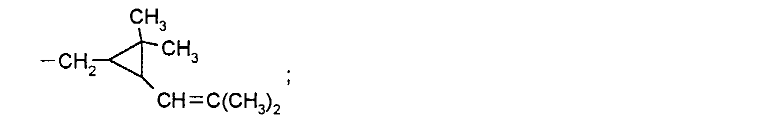 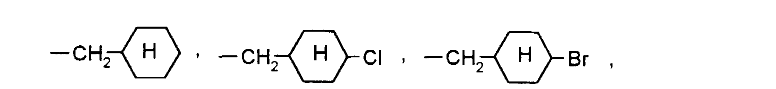 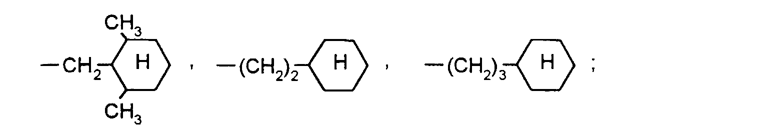 für die Cycloalkenylalkylgruppierung: 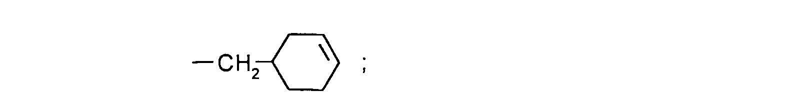 für eine der Phenylalkylgruppierungen: 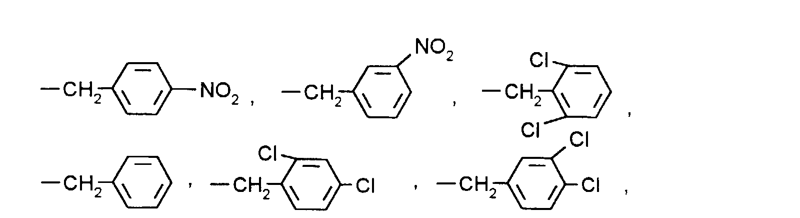 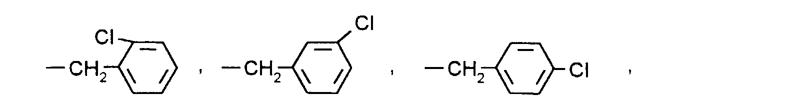 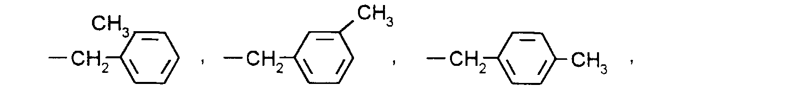 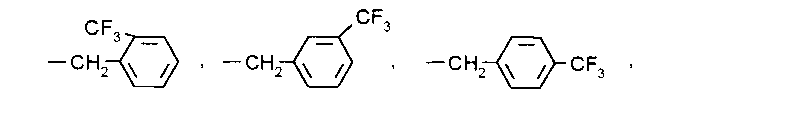 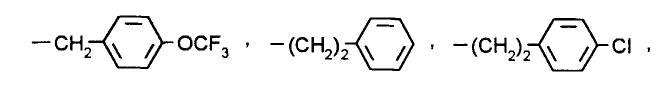 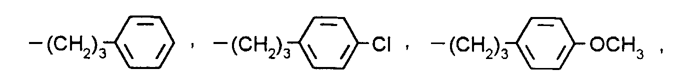 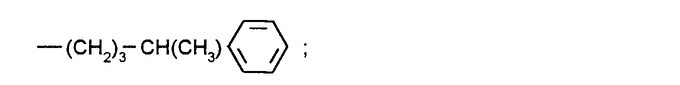 für 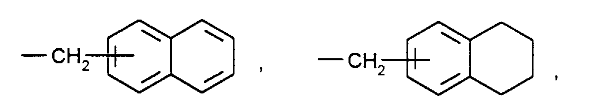 für eine der Heteroarylalkylgruppierungen: 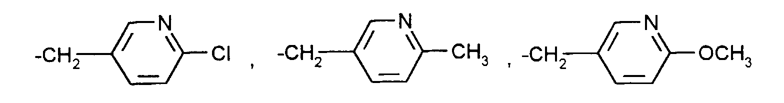 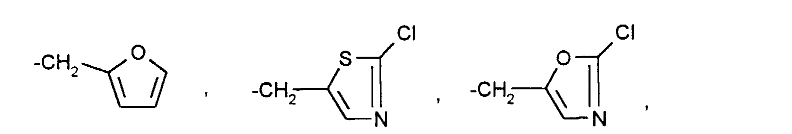 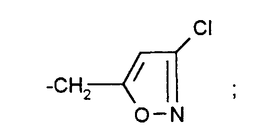 oder für den Rest -COR³ steht.
R³ steht besonders bevorzugt
für Methyl, Ethyl, Propyl;
für Methoxy, Ethoxy, Propoxy, Butoxy;
für Cyclopropyl, Cyclohexyl;
für Cyclohexyloxy;
für Phenyl, 2-Chlorphenyl, 3-Chlorphenyl, 2,6-Difluorphenyl, 2-Trifluormethoxyphenyl, 4-Trifluormethoxyphenyl; 2,4-Dichlorphenyl; 3,4-Dichlorphenyl;
oder für den Rest -NHR⁵.
R⁵ steht besonders bevorzugt für Methyl, Ethyl oder gegebenenfalls einfach durch Chlor substituiertes Phenyl.
- X: steht besonders bevorzugt für Fluor, Chlor oder Brom.
- m: steht besonders bevorzugt für 0 oder 1.
- n: steht besonders bevorzugt für 1.

Bevorzugt sind weiterhin Verbindungen der Formel (Ic) 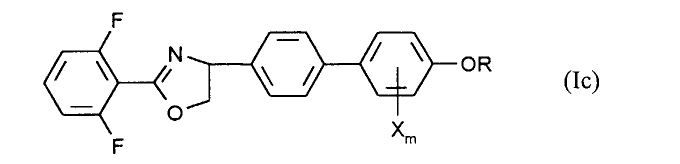 in welcher
- R, X und m: die obengenannten allgemeinen, bevorzugten oder besonders bevorzugten Bedeutungen haben.

Eine bevorzugte Gruppe von Verbindungen sind solche der Formel (Id) 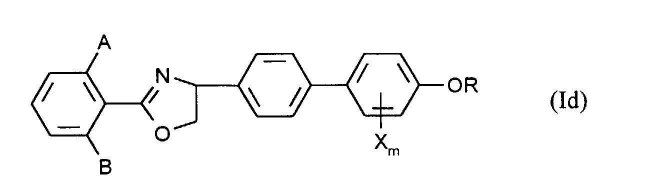 in welcher
- A: für Wasserstoff steht,
- B: für Fluor steht und
- R, X und m: die obengenannten allgemeinen, bevorzugten und besonders bevorzugten Bedeutungen haben.

Eine weitere bevorzugte Gruppe von Verbindungen sind solche der obengenannten Formel (Id), in welcher
- A: für Wasserstoff steht,
- B: für Chlor steht und
- R, X und m: die obengenannten allgemeinen, bevorzugten und besonders bevorzugten Bedeutungen haben.

Dabei sind jeweils 2-(2,6-Difluorphenyl)-4-[4'-(2,2,2-trifluorethoxy)-biphenyl-4]-2-oxazolin und 2-(2-Chlor-6-fluorphenyl)-4-[4'-(2,2,2-trifluorethoxy)-biphenyl-4]-2-oxazolin ausgenommen.

Die oben bei der Definition der erfindungsgemäßen Verbindungen genannten Kohlenwasserstoffreste, wie Alkyl oder Alkenyl, sind - auch in Verbindung mit Heteroatomen, wie in Alkoxy - soweit möglich jeweils geradkettig oder verzweigt.

Die oben aufgeführten allgemeinen oder in Vorzugsbereichen aufgeführten Reste-definitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Verwendet man gemäß Verfahren Aα) z.B. 4-Hydroxybiphenyl und Trifluorchlorethylen als Ausgangsstoffe, so kann der Verlauf des erfindungsgemäßen Verfahrens durch folgendes Reaktionsschema wiedergegeben werden:

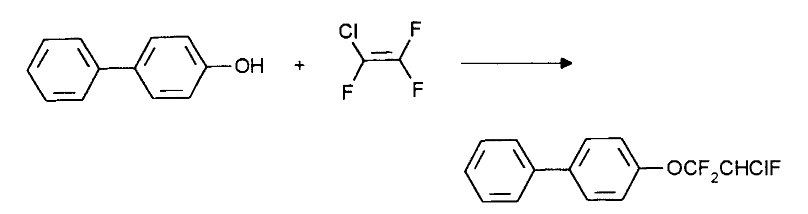

Verwendet man gemäß Verfahren Aβ) z.B. 4-Hydroxybiphenyl und Difluorbrommethan als Ausgangsstoffe, so kann der Verlauf des erfindungsgemäßen Verfahrens durch folgendes Reaktionsschema wiedergegeben werden:

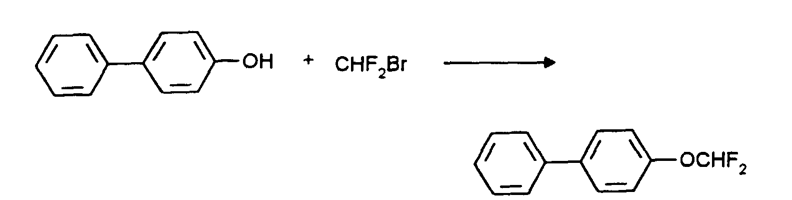

Verwendet man gemäß Verfahren Aγ) z.B. 4-Difluormethoxyanilin als Ausgangsstoff, so kann der Verlauf des erfindungsgemäßen Verfahrens durch das folgende Reaktionsschema wiedergegeben werden:

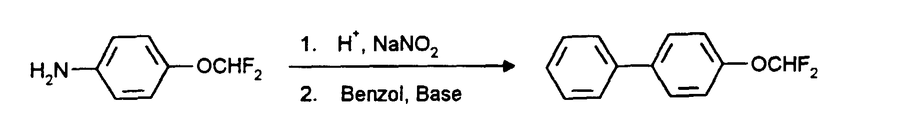

Verwendet man gemäß Verfahren Aδ) z.B. 4-Hydroxybiphenyl als Ausgangsstoff, so kann der Verlauf des erfindungsgemäßen Verfahrens durch das folgende Reaktionsschema dargestellt werden:

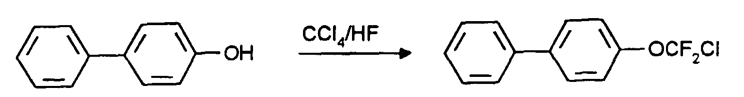

Verwendet man gemäß Verfahren Bα) z.B. 4-Difluorchlormethoxybiphenyl als Ausgangsstoff, so kann der Verlauf des erfindungsgemäßen Verfahrens durch das folgende Reaktionsschema dargestellt werden:

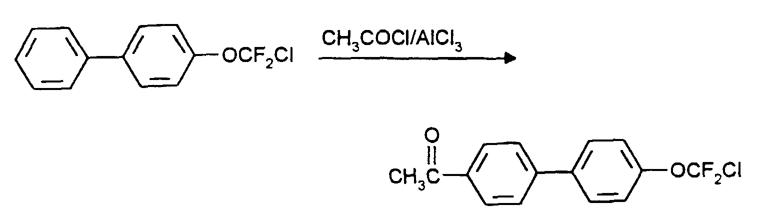

Verwendet man gemäß Verfahren Bβ) z.B. 4-Acetyl-4'-hydroxybiphenyl und Hexafluorcyclobuten als Ausgangsstoffe, so kann der Verlauf des erfindungsgemäßen Verfahrens durch das folgende Reaktionsschema dargestellt werden:

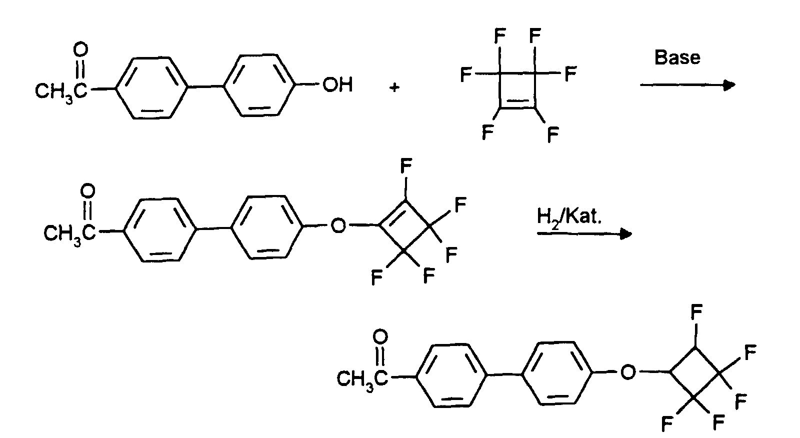

Verwendet man gemäß Verfahren Bγ) z.B. 4-Acetyl-4'-hydroxybiphenyl und Chlordifluormethan als Ausgangsstoffe, so kann der Verlauf des erfindungsgemäßen Verfahrens durch das folgende Formelschema wiedergegeben werden:

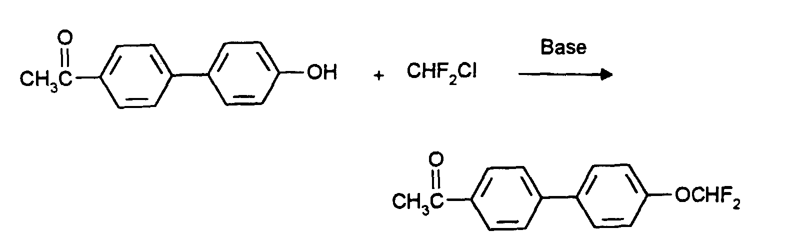

Verwendet man gemäß Verfahren Cα) z.B. 4-Acetyl-4'-difluormethoxybiphenyl als Ausgangsstoff, so kann der Verlauf des erfindungsgemäßen Verfahrens durch das folgende Formelschema wiedergegeben werden:

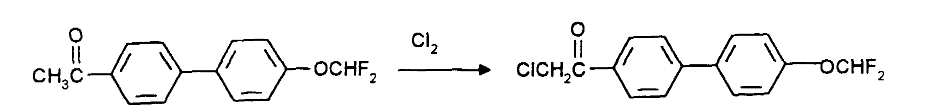

Verwendet man gemäß Verfahren Cβ) z.B. 4-Difluorchlormethoxybiphenyl und Chloracetylchlorid als Ausgangsstoffe, so kann der Verlauf des erfindungsgemäßen Verfahrens durch das folgende Formelschema wiedergegeben werden:

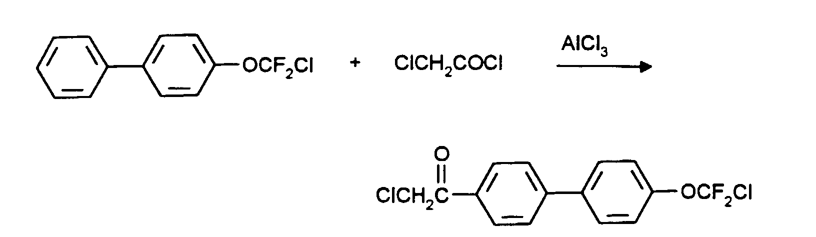

Verwendet man gemäß Verfahren D) 4-Chloracetyl-4'-difluormethoxybiphenyl als Ausgangsstoff, so kann der Verlauf des erfindungsgemäßen Verfahrens durch das folgende Formelschema wiedergegeben werden:

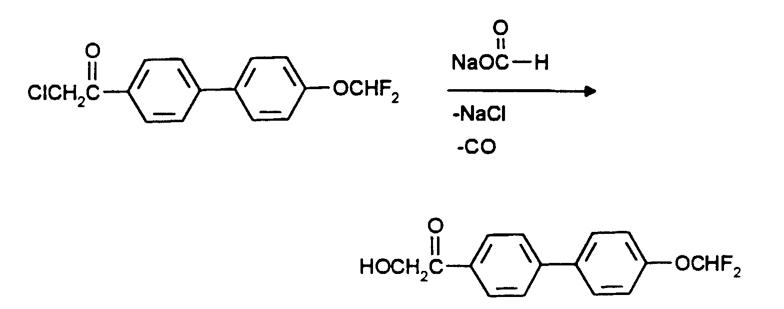

Verwendet man gemäß Verfahren E) z.B. 4-Hydroxyacetyl-4'-difluorchlormethoxybiphenyl als Ausgangsstoff, so kann der Verlauf des erfindungsgemäßen Verfahrens durch das folgende Reaktionsschema wiedergegeben werden:

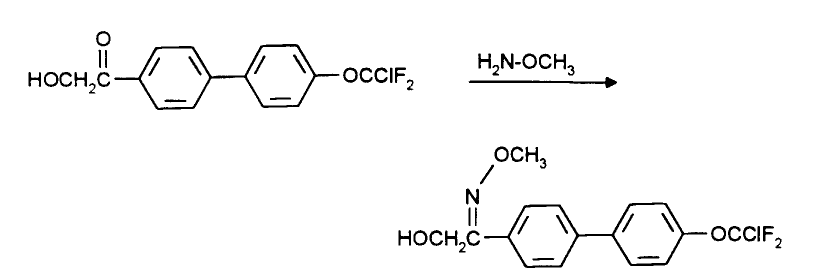

Verwendet man gemäß Verfahren F) z.B. 4-Hydroxyacetyl-oxim-O-methylether-4'-trifluorchlorethoxy-biphenyl als Ausgangsstoff, so kann der Verlauf des erfindungsgemäßen Verfahrens durch das folgende Reaktionsschema wiedergegeben werden:

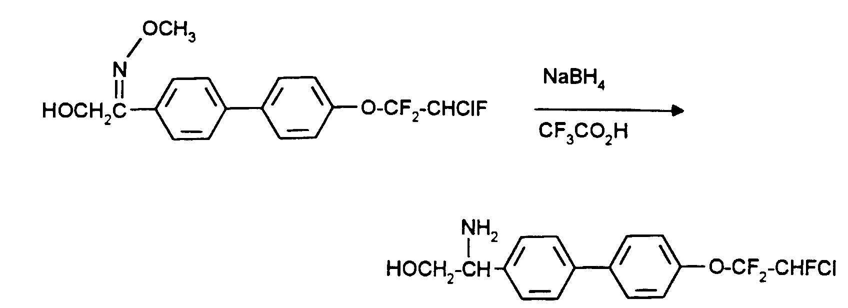

Verwendet man gemäß Verfahren Gα) z.B. 2-Amino-2-(4'-difluorchlormethoxybiphenyl)-4)-ethan-1-ol als Ausgangsmaterial, so kann der Verlauf des erfindungsgemäßen Verfahrens durch das folgende Reaktionsschema wiedergegeben werden:

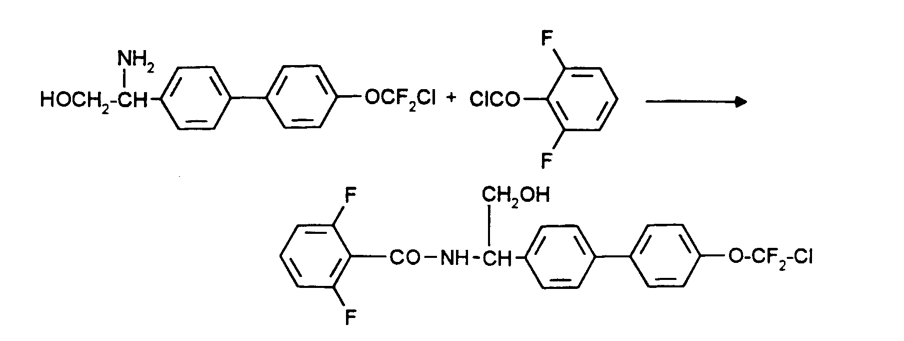

Verwendet man gemäß Verfahren Gβ) z.B. N-(Carboxymethylchlormethyl)-2,6-difluorbenzamid und 4-Difluorchlormethoxybiphenyl als Ausgangsstoffe, so kann der Verlauf des erfindungsgemäßen Verfahrens durch das folgende Reaktionsschema wiedergegeben werden:

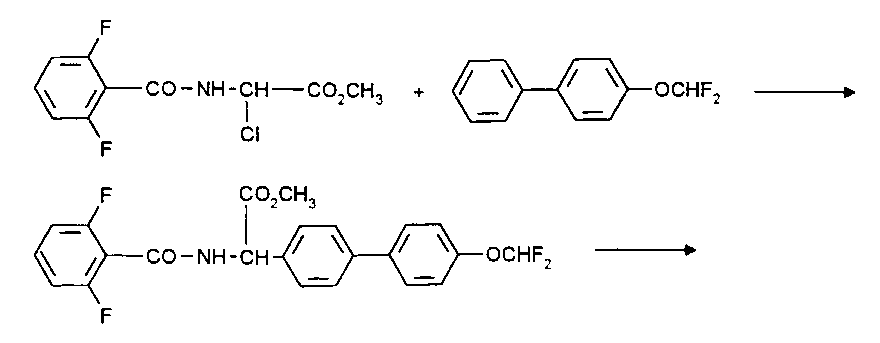

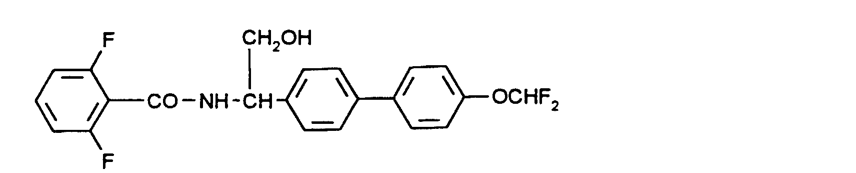

Verwendet man gemäß Verfahren Hα) z.B. N-(1-(4'-Difluormethoxybiphenyl-4)-ethyl-2-ol)-2,6-difluorbenzamid und Thionylchlorid als Ausgangsstoffe, so kann der Verlauf des erfindungsgemäßen Verfahrens durch das folgende Reaktionsschema wiedergegeben werden:

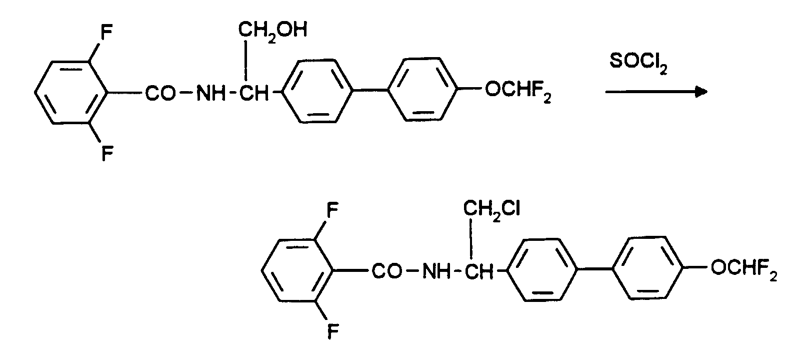

Verwendet man gemäß Verfahren Hβ) z.B. N-1-Methoxy-2-chlorethyl)-2,6-difluorbenzamid und 4-Difluormethoxybiphenyl als Ausgangsstoffe, so kann der Verlauf des erfindungsgemäßen Verfahrens durch das folgende Reaktionsschema wiedergegeben werden:

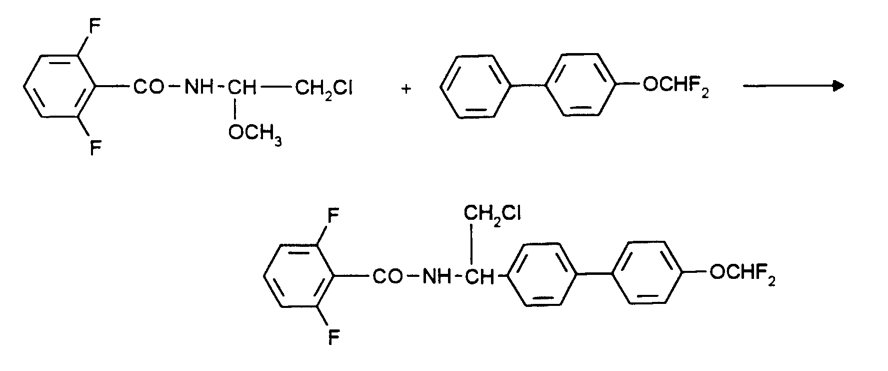

Verwendet man gemäß Verfahren I) z.B. N-(1-(4'-Difluormethoxybiphenyl-4-)2-chlorethyl)-2,6-difluorbenzamid als Ausgangsstoff, so kann der Verlauf des erfindungsgemäßen Verfahrens durch das folgende Reaktionsschema wiedergegeben werden:

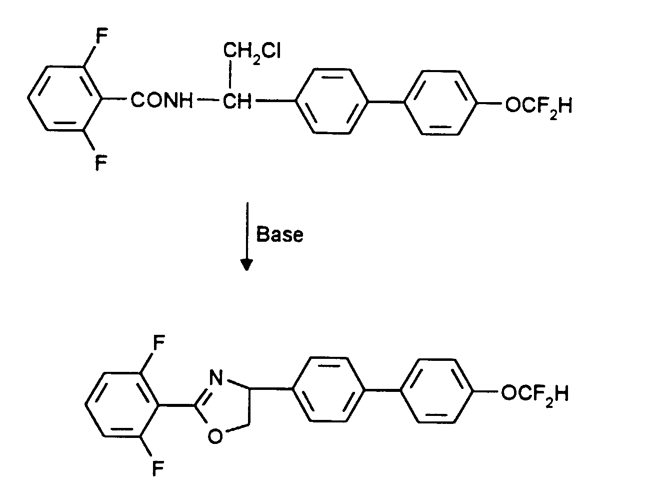

Verwendet man gemäß Verfahren J) z.B. 2-(2,6-Difluorphenyl)-4-(4'-tert.-butylcarbonyloxybiphenyl-4)-2-oxazolin als Ausgangsverbindung, so kann der Verlauf des erfindungsgemäßen Verfahrens durch das folgende Formelschema wiedergegeben werden:

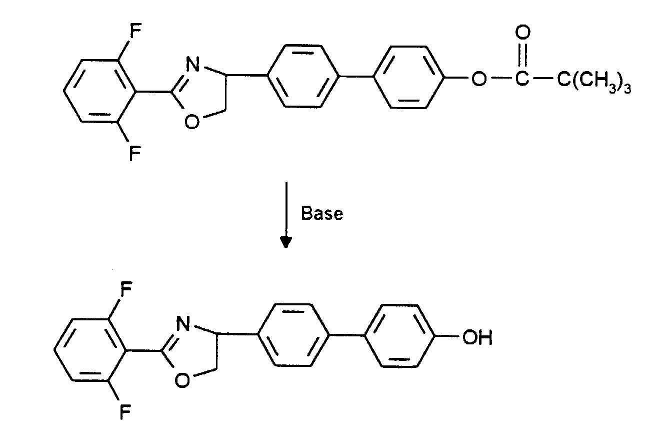

Verwendet man gemäß Beispiel Kα) z.B. 2-(2,6-Difluorphenyl)-4-(4'-hydroxybiphenyl-4)-2-oxazolin und Chlorameisensäureethylester als Ausgangsverbindungen, so läßt sich der Verlauf des erfindungsgemäßen Verfahrens durch das folgende Reaktionsschema darstellen:

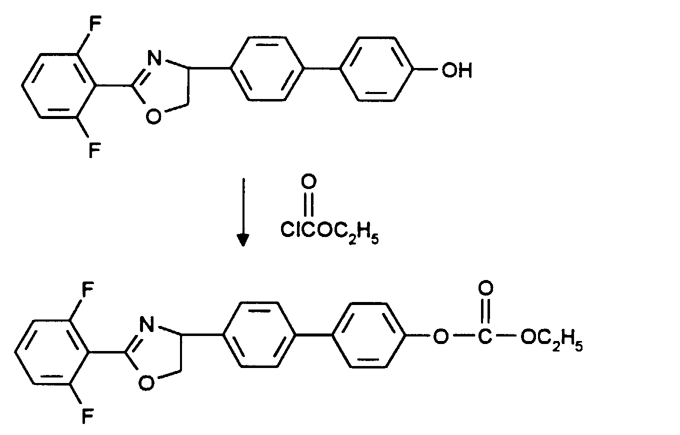

Verwendet man gemäß Beispiel Kβ) z.B. 2-(2,6-Difluorphenyl)-4-(4'-hydroxybiphenyl-4)-2-oxazolin und Trifluorchlorethylen als Ausgangsstoffe, so läßt sich der Verlauf des erfindungsgemäßen Verfahrens durch das folgende Reaktionsschema darstellen:

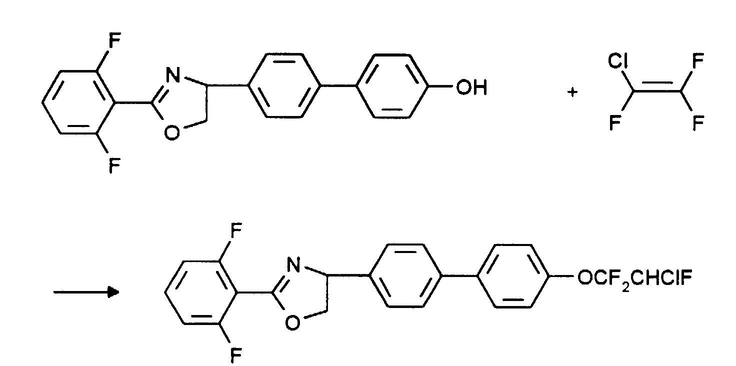

Verwendet man gemäß Beispiel Kγ) z.B. 2-(2,6-Difluorphenyl)-4-(4'-hydroxybiphenyl-4)-2-oxazolin und Difluorchlormethan als Ausgangsstoffe, so läßt sich der Verlauf des erfindungsgemäßen Verfahrens durch das folgende Reaktionsschema darstellen:

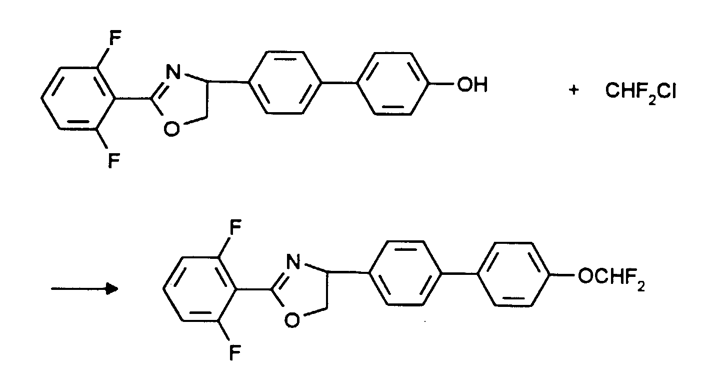

Verwendet man gemäß Beispiel Kδ) z.B. 2-(2,6-Difluorphenyl)4-(4'-hydroxybiphenyl-4)-2-oxazolin und Tetrachlorkohlenstoff als Ausgangsstoffe, so läßt sich der Verlauf des erfindungsgemäßen Verfahrens durch das folgende Reaktionsschema darstellen:

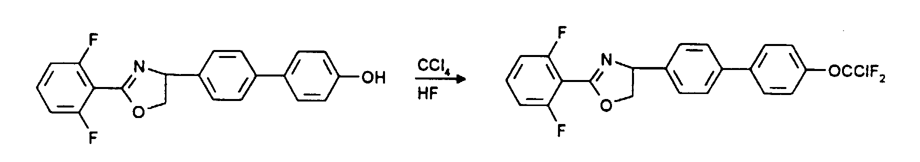

Verwendet man gemäß Beispiel Kε) z.B. 2-(2,6-Difluorphenyl)-4-(4'-hydroxybiphenyl-4)-2-oxazolin und Allylbromid als Ausgangsstoffe, so läßt sich der Verlauf des erfindungsgemäßen Verfahrens durch das folgende Reaktionsschema darstellen:

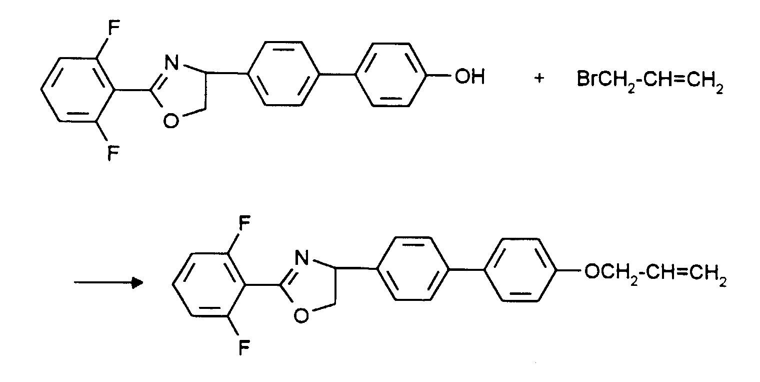

Die zur Durchführung des erfindungsgemäßen Verfahrens Aα) als Ausgangsstoffe benötigten Verbindungen der Formel (III) sind bekannt und/oder lassen sich nach bekannten Methoden in einfacher Weise herstellen.

Man erhält die Verbindungen der Formel (III) beispielsweise, indem man gegebenenfalls substituierte Biphenyle sulfoniert und dann mit Alkalihydroxiden zu den Hydroxybiphenylen umsetzt oder Aminobiphenyle diazotiert und verkocht. (vgl. z.B. Houben-Weyl, Band VI/1c (1976), Seite 216 und 251)

Die zur Durchführung der erfindungsgemäßen Verfahren Aα), Bβ) und Kβ) weiter als Ausgangsstoffe benötigten Verbindungen der Formel (IV) sind bekannt und/oder lassen sich nach bekannten Methoden in einfacher Weise herstellen (vgl. z.B. Houben-Weyl, Band V/3 (1962), Seite 187 ff, 317, 346 ff und 377 ff.)

Die zur Durchführung der erfindungsgemäßen Verfahren Aβ) und Kγ) als Ausgangsstoffe benötigten Difluorhalogenmethane der Formel (V) sind allgemein bekannte Verbindungen der Organischen Chemie.

Die zur Durchführung des erfindungsgemäßen Verfahrens Aγ) als Ausgangsstoffe benötigten Verbindungen der Formel (VI) sind bekannt und/oder lassen sich nach bekannten Methoden in einfacher Weise herstellen.

Man erhält die Verbindungen der Formel (VI) beispielsweise, indem man die entsprechenden Nitroaromaten reduziert oder die entsprechenden Carbonsäureamide z.B. einem Hofmann-Abbau unterwirft.

Die zur Durchführung des erfindungsgemäßen Verfahrens Cβ) als Ausgangsstoffe benötigten Halogenacetylchloride der Formel (IX) sind gängige, allgemein bekannte Chemikalien der Organischen Chemie.

Die zur Durchführung des erfindungsgemäßen Verfahrens E) benötigte Verbindung (XII) ist eine allgemein bekannte Chemikalie der Organischen Chemie.

Die zur Durchführung des erfindungsgemäßen Verfahrens Gβ) als Ausgangsstoffe benötigten Verbindungen der Formel (XV) sind bekannt und/oder lassen sich nach bekannten Methoden in einfacher Weise herstellen (vgl. z.B. WO 93/24 470).

Die zur Durchführung des erfindungsgemäßen Verfahrens Hβ) als Ausgangsstoffe benötigten Verbindungen der Formel (XVIII) sind bekannt und/oder lassen sich nach bekannten Methoden in einfacher Weise herstellen (vgl. z.B. EP-A-0 594 179). Die ebenfalls zur Durchführung des erfindungsgemäßen Verfahrens Hβ) als Ausgangsstoffe benötigten Verbindungen der Formel (IIe) sind bekannt und/oder lassen sich in einfacher Weise z.B. durch Acylierung der entsprechenden Hydroxybiphenyle erhalten.

Die zur Durchführung des erfindungsgemäßen Verfahrens Kα) als Ausgangsstoffe benötigten Verbindungen der Formel (XIX) sind allgemein bekannte Verbindungen der organischen Chemie.

Die zur Durchführung des erfindungsgemäßen Verfahrens Kε) als Ausgangsstoffe benötigten Verbindungen der Formel (XX) sind allgemein bekannte Verbindungen der organischen Chemie.

M steht für eine übliche Abgangsgruppe; wenn R² die Bedeutung -COR³ hat, steht M vorzugsweise für Halogen, insbesondere Chlor oder Brom, Anhydrid oder Imidazolid; wenn R² die übrigen oben genannten Bedeutungen hat, steht M vorzugsweise für Halogen, insbesondere Chlor oder Brom; Alkylsulfonyloxy, insbesondere Methylsulfonyloxy; oder gegebenenfalls substituiertes Arylsulfonyloxy, insbesondere Phenylsulfonyloxy, p-Chlorphenylsulfonyloxy oder Tolylsulfonyloxy.

Die Zwischenprodukte der Formeln (Ia), (Ib), (IIa), (IIb), (IIc), (IId), (VII), (VIIa), (VIIb), (VIII), (X), (XI), (XIII), (XIV), (XVI) und (XVII) sind neu und ebenfalls Gegenstand der Erfindung. Sie weisen z.T. selbst insektizide und akarizide Eigenschaften auf, so beispielsweise die Verbindungen der Formel (Ia), (Ib), (XIV) und (XVII).

Die Zwischenprodukte der Formel (II) sind neu mit Ausnahme der Verbindungen 4-(2,2,2-Trifluorethoxybiphenyl), 4-(1,1-Difluorethoxybiphenyl), 4-(1,1-Difluor-n-propoxy)biphenyl und 4-(2-Fluorethoxy)biphenyl (bekannt aus Synthesis, Nr. 5, 1985, 499-500 und US-A-4 960 884).

Das Verfahren Aα) zur Herstellung von Verbindungen der Formel (IIa) ist dadurch gekennzeichnet, daß man Verbindungen der Formel (III) mit Verbindungen der Formel (IV), gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert.

Als Verdünnungsmittel können alle üblichen Lösungsmittel eingesetzt werden. Vorzugsweise verwendbar sind beispielsweise Nitrile, wie Acetonitril und Butyronitril oder Ether, wie Methyl-tert.-butylether, Methyl-tert.-amylether, Diisopropylether, 1,2-Dimethoxyethan, Tetrahydrofuran und Dioxan.

Als Basen können alle üblichen Protonenakzeptoren eingesetzt werden. Genannt seien z.B. tertiäre Amine, wie Triethylamin, Pyridin, Diazabicyclooctan (DABCO), Diazabicycloundecan (DBU), und Alkali- und Erdalkalihydroxide. Vorzugsweise verwendbar sind Alkalihydroxide, insbesondere Natriumhydroxid oder Kaliumhydroxid, die auch als wäßrige Lösung eingesetzt werden können.

Als Katalysatoren können Phasentransferkatalysatoren eingesetzt werden. Genannt seien beispielsweise quartäre Ammoniumsalze wie Tetraoctylammoniumbromid und Benzyltriethylammoniumchlorid.

Die Reaktionstemperatur kann innerhalb eines größeren Bereiches variiert werden. Im allgemeinen arbeitet man bei Temperaturen zwischen 20°C und 100°C, bevorzugt zwischen 25°C und 70°C.

Die Umsetzung wird im allgemeinen unter Normaldruck oder erhöhtem Druck durchgeführt. Im allgemeinen arbeitet man bei einem Druck zwischen 1 bar und 25 bar, bevorzugt zwischen 1 bar und 6 bar.

Das Verhältnis von Base zu Ausgangsstoff der Formel (III) kann in einem größeren Bereich variiert werden. Grundsätzlich genügen katalytische Mengen an Base, sie kann jedoch auch äquimolar oder im Überschuß eingesetzt werden. Im allgemeinen verwendet man pro mol an Verbindung der Formel (III) 0,1 bis 5 mol, bevorzugt 0,5 bis 2 mol an Base.

Im allgemeinen geht man so vor, daß die Verbindung der Formel (III) und die Base in einem Verdünnungsmittel vorgelegt werden, die gewünschte Reaktionstemperatur wird eingestellt und eine etwa äquimolare Menge des Olefins der Formel (IV) wird zudosiert.

Arbeitet man im geschlossenen Kessel oder Autoklav, stellt sich ein gewisser Eigendruck ein, der durch das Einleiten eines Inertgases, wie z.B. Stickstoff, erhöht werden kann.

Das Reaktionsgemisch wird in üblicher Weise beispielsweise durch Extraktion oder Filtration aufgearbeitet.

Offenkettige Verbindungen der Formel (IV) reagieren mit den Verbindungen der Formel (III) unter Addition.

Cyclische Verbindungen der Formel (IV) reagieren mit den Verbindungen der Formel (III) an der Doppelbindung durch Substitution. Die dann verbleibende Doppelbindung kann gegebenenfalls anschließend hydriert werden, beispielsweise mit Wasserstoff (gegebenenfalls unter erhöhtem Druck) in Gegenwart eines Edelmetallkatalysators wie z.B. Platin oder Palladium. Die Hydrierung wird vorzugsweise in einem Verdünnungsmittel durchgeführt, wobei alle üblichen Lösungsmittel wie z.B. (Halogen)Kohlenwasserstoffe, Ether, Alkohole, in Frage kommen. Wenn Y für Fluor oder Chlor steht, werden bevorzugt aprotische Lösungsmittel verwendet.

Das Verfahren Aβ) zur Herstellung von Verbindungen der Formel (IIb) ist dadurch gekennzeichnet, daß man Verbindungen der Formel (III) mit einem Difluorhalogenmethan der Formel (V) gegebenenfalls in Gegenwart einer Base, gegebenenfalls in Gegenwart eines Katalysators und gegebenenfalls in Gegenwart eines Verdünnungsmittels umsetzt.

Als Verdünnungsmittel können alle üblichen Lösungsmittel eingesetzt werden. Vorzugsweise verwendbar sind beispielsweise Nitrile, wie Acetonitril und Butyronitril, Ether, wie Methyl-tert.-butylether, Methyl-tert.-amylether, Diisopropylether, 1,2-Dimethoxyethan, Tetrahydrofuran und Dioxan, oder Alkohole, wie Ethanol, die isomeren Propanole oder die isomeren Butanole.

Als Basen können alle üblichen Protonenakzeptoren eingesetzt werden. Vorzugsweise verwendbar sind Alkalihydroxide wie Natriumhydroxid oder Kaliumhydroxid, die auch als wäßrige Lösung eingesetzt werden können.

Als Katalysatoren können Phasentransferkatalysatoren eingesetzt werden. Genannt seien beispielsweise quartäre Ammoniumsalze, wie Tetraoctylammoniumbromid und Benzyltriethylammoniumchlorid und Phosphoniumsalze wie Tetrabutylphosphoniumbromid.

Die Reaktionstemperatur kann innerhalb eines größeren Bereichs variiert werden. Im allgemeinen arbeitet man bei einer Temperatur zwischen 20°C und 150°C, bevorzugt zwischen 40°C und 100°C.

Die Umsetzung wird im allgemeinen unter Normaldruck oder erhöhtem Druck durchgeführt. Im allgemeinen arbeitet man bei einem Druck zwischen 1 bar und 25 bar, bevorzugt zwischen 1 bar und 10 bar.

Das Verhältnis von Base zu Ausgangsstoff der Formel (III) kann in einem größeren Bereich variiert werden. Im allgemeinen setzt man pro mol an Verbindung der Formel (III) 1 bis 10 mol, bevorzugt 1 bis 5 mol an Base ein.

Das Difluorhalogenmethan der Formel (V) wird im allgemeinen in einem bis zu 5-fachen Überschuß eingesetzt.

Das Verfahren Aγ) zur Herstellung von Verbindungen der Formel (IIc) ist dadurch gekennzeichnet, daß man Verbindungen der Formel (VI) diazotiert und in Gegenwart von Säure und Eisenpulver oder in Gegenwart einer Base und gegebenenfalls in Gegenwart eines Verdünnungsmittels mit Benzol umsetzt.

Als Verdünnungsmittel kommen alle inerten Lösungsmittel in Frage. Es kann aber auch ein größerer Überschuß des Reaktionspartners Benzol, vorzugsweise bis zu 30 mol, besonders bevorzugt bis zu 5 mol, bezogen auf ein mol der Verbindung der Formel (VI), als Verdünnungsmittel verwendet werden.

Wird die Umsetzung in Gegenwart von Säure und Eisenpulver durchgeführt, kommen als Säure bevorzugt organische Säuren, wie z.B. Trichloressigsäure, in Frage.

Wird die Umsetzung in Gegenwart einer Base durchgeführt, kommen als Base z.B. Salze organischer Säuren, wie Alkaliacetate, insbesondere Natriumacetat oder Kaliumacetat, in Frage.

Im allgemeinen werden zwei Äquivalente an Base eingesetzt.

Die Reaktionstemperatur kann innerhalb eines größeren Bereichs variiert werden. Im allgemeinen arbeitet man bei einer Temperatur zwischen -40°C und 140°C, bevorzugt zwischen -20°C und 80°C.

Die Umsetzung wird im allgemeinen unter Normaldruck durchgeführt.

Das Diazoniumsalz wird im allgemeinen in Gegenwart einer Säure wie Salzsäure oder Schwefelsäure aus der Verbindung der Formel (VI) in üblicher Weise durch Umsetzung mit einem Alkalinitrit wie Natriumnitrit oder einem Alkylnitrit wie Pentylnitrit oder Methylnitrit oder durch Umsetzung mit Nitrosylchlorid hergestellt.

Das das Produkt der Formel (IIc) enthaltende Reaktionsgemisch wird mit Hilfe üblicher Methoden aufgearbeitet.

Das Verfahren Aδ) zur Herstellung von Verbindungen der Formel (IId) ist dadurch gekennzeichnet, daß man Verbindungen der Formel (III) in Gegenwart von wasserfreier Fluorwasserstoffsäure, gegebenenfalls in Gegenwart eines Verdünnungsmittels, mit Tetrachlorkohlenstoff umsetzt.

Als Verdünnungsmittel kommen inerte organische Lösungsmittel in Frage. Bevorzugt wird ein 2- bis 3-facher Überschuß Tetrachlorkohlenstoff als Verdünnungsmittel verwendet.

Die Menge an wasserfreier Fluorwasserstoffsäure kann in einem größeren Bereich variiert werden. Im allgemeinen setzt man pro mol der Verbindung der Formel (III) 2 bis 40 mol, bevorzugt 5 bis 20 mol wasserfreie Fluorwasserstoffsäure ein.

Die Reaktionstemperatur kann innerhalb eines größeren Bereichs variiert werden. Im allgemeinen arbeitet man bei einer Temperatur zwischen 60°C und 150°C, bevorzugt zwischen 80°C und 125°C.

Im allgemeinen geht man so vor, daß man in einem Autoklav die Verbindung der Formel (III) mit Tetrachlorkohlenstoff und wasserfreier Fluorwasserstoffsäure mischt, die Mischung auf die gewünschte Temperatur heizt und bei dem sich einstellenden Druck rührt.

Zur Aufarbeitung wird das Reaktionsgemisch beispielsweise durch Destillation von leichterflüchtigen Komponenten wie Fluorwasserstoffsäure, Frigenen und überschüssigem Tetrachlorkohlenstoff befreit.

Der Rückstand wird in einem geeigneten inerten Lösungsmittel aufgenommen, mit Alkali gewaschen und destillativ gereinigt.

Das Verfahren Bα) zur Herstellung von Verbindungen der Formel (VII) ist dadurch gekennzeichnet, daß man Verbindungen der Formel (II) in Gegenwart einer Säure oder Lewis-Säure und in Gegenwart eines Verdünnungsmittels mit Acetylchlorid umsetzt.

Als Verdünnungsmittel kommen alle üblichen, für Friedel-Crafts-Reaktionen geeigneten Lösungsmittel in Frage. Bevorzugt verwendet werden chlorierte Kohlenwasserstoffe wie z.B. Methylenchlorid oder Dichlorethan.

Als Säure oder Lewis-Säure kommen alle für Friedel-Crafts-Reaktionen geeigneten in Frage. Bevorzugt verwendet werden Aluminiumchlorid, wasserfreie Flußsäure, Tetrafluoroborsäure oder BF₃-Etherat.

Die Reaktionstemperatur kann innerhalb eines größeren Bereichs variiert werden. Im allgemeinen arbeitet man bei Temperaturen zwischen -30°C und 80°C, bevorzugt zwischen -15°C und 50°C.

Die Umsetzung wird im allgemeinen unter Normaldruck oder unter erhöhtem Druck durchgeführt.

Acetylchlorid und die Verbindungen der Formel (II) werden im allgemeinen in etwa äquimolaren Mengen eingesetzt.

Nach beendeter Umsetzung wird mit Hilfe üblicher Methoden aufgearbeitet.

Das Verfahren Bβ) zur Herstellung von Verbindungen der Formel (VIIa) ist dadurch gekennzeichnet, daß man Verbindungen der Formel (IIIa) mit einer Verbindung der Formel (IV) gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert.

Hinsichtlich Base, Katalysator, Verdünnungsmittel und den anderen Reaktionsbedingungen gilt das gleiche wie bei Verfahren Aα).

Das Verfahren Bγ) zur Herstellung von Verbindungen der Formel (VIIb) ist dadurch gekennzeichnet, daß man eine Verbindung der Formel (IIIa) mit einer Verbindung der Formel (V) gegebenenfalls in Gegenwart einer Base, gegebenenfalls in Gegenwart eines Katalysators und gegebenenfalls in Gegenwart eines Verdünnungsmittels umsetzt.

Hinsichtlich Base, Katalysator, Verdünnungsmittel und den anderen Reaktionsbedingungen gilt das gleiche wie bei Verfahren Aβ).

Das Verfahren Cα zur Herstellung von Verbindungen der Formel (VIII) ist dadurch gekennzeichnet, daß man Verbindungen der Formel (VII) gegebenenfalls in Gegenwart eines Verdünnungsmittels chloriert oder bromiert.

Als Verdünnungsmittels kommen alle gegenüber Chlor und Brom inerten Lösungsmittel in Frage. Bevorzugt verwendet werden beispielsweise Chlorkohlenwasserstoffe wie Methylenchlorid, Chloroform oder Tetrachlorkohlenstoff oder Alkohole wie Methanol oder Ethanol.

Die Reaktionstemperatur kann innerhalb eines größeren Bereichs variiert werden. Im allgemeinen arbeitet man bei einer Temperatur zwischen -30°C und 50°C, bevorzugt zwischen -10°C und 25°C.

Die Umsetzung wird im allgemeinen unter Normaldruck durchgeführt.

Im allgemeinen geht man so vor, daß die Verbindung der Formel (VII) in einem geeigneten Verdünnungsmittel vorgelegt wird und dann bei der gewünschten Temperatur eine etwa äquimolare Menge Chlor oder Brom zudosiert wird. Man kann auch einen geringen Über- oder Unterschuß Halogen einsetzen.

Das Verfahren Cβ) zur Herstellung von Verbindungen der Formel (VIII) ist dadurch gekennzeichnet, daß man Verbindungen der Formel (II) mit Halogenacetylchloriden der Formel (IX) in Gegenwart einer Säure oder Lewis-Säure und in Gegenwart eines Verdünnungsmittels umsetzt.

Als Verdünnungsmittel kommen alle üblichen, für Friedel-Crafts-Reaktionen geeigneten Lösungsmittel in Frage. Bevorzugt verwendet werden chlorierte Kohlenwasserstoffe wie z.B. Methylenchlorid oder Dichlorethan.

Als Säuren oder Lewis-Säuren kommen alle für Friedel-Crafts-Reaktionen geeigneten in Frage. Bevorzugt verwendet werden Aluminiumchlorid oder Tetrafluoroborsäure.

Die Temperatur kann innerhalb eines größeren Bereichs variiert werden. Im allgemeinen arbeitet man zwischen -30°C und 50°C, bevorzugt zwischen -15°C und 30°C.

Die Umsetzung wird im allgemeinen unter Normaldruck durchgeführt.

Das Halogenacetylchlorid der Formel (IX) und die Verbindung der Formel (II) werden im allgemeinen in etwa äquimolaren Mengen eingesetzt.

Nach beendeter Umsetzung wird mit Hilfe üblicher Methoden aufgearbeitet.

Das Verfahren D) zur Herstellung von Verbindungen der Formel (X) ist dadurch gekennzeichnet, daß man Verbindungen der Formel (VIII) mit einem Salz der Ameisensäure gegebenenfalls in Gegenwart eines Katalysators umsetzt.

Als Verdünnungsmittel kommen alle üblichen, unter den Reaktionsbedingungen inerten Lösungsmittel in Frage. Vorzugsweise verwendbar sind Kohlenwasserstoffe wie Toluol, Xylol, Mesitylen, Cyclohexan, Methylcyclohexan, Chlorkohlenwasserstoffe wie Chlorbenzol, o-Dichlorbenzol, Tetrachlorkohlenstoff, Alkohole wie Methanol, Ethanol, die isomeren Propanole, die isomeren Butanole und Pentanole, Ether wie Diisopropylether, Tetrahydrofuran, Dioxan, Nitrile wie Acetonitril und Butyronitril, Amide wie Dimethylformamid und auch stark polare Solventien wie Dimethylsulfoxid und Sulfolan.

Die genannten Verdünnungsmittel können gegebenenfalls auch im Gemisch mit Wasser verwendet werden, gegebenenfalls in Gegenwart eines Phasentransferkatalysators, z.B. quartären Ammoniumsalzen, wie Tetraoctylammoniumbromid oder Benzyltriethylammoniumchlorid.

Vorzugsweise verwendbare Salze der Ameisensäure sind Natriumformiat und Kaliumformiat.

Die Reaktionstemperatur kann in einem größeren Bereich variiert werden. Im allgemeinen arbeitet man bei Temperaturen zwischen 50°C und 200°C, bevorzugt zwischen 80°C und 160°C.

Im allgemeinen geht man so vor, daß man die Verbindung der Formel (VIII) mit 1 bis 20 mol, bevorzugt 1 bis 5 mol Formiat in einem Verdünnungsmittel erhitzt, gegebenenfalls Wasser zugibt, die Phasen trennt und das Verdünnungsmittel abdestilliert.

Das Verfahren E) zur Herstellung von Verbindungen der Formel (XI) ist dadurch gekennzeichnet, daß man Verbindungen der Formel (X) mit der Verbindung der Formel (XII) gegebenenfalls in Gegenwart eines Verdünnungsmittels umsetzt.

Als Verdünnungsmittel kommen alle üblichen Lösungsmittel in Frage. Vorzugsweise verwendet werden beispielsweise Alkohole wie Methanol, Ethanol, die isomeren Propanole, Butanole, Pentanole, oder Ether wie Diisopropylether, Tetrahydrofuran, Dioxan, die gegebenenfalls im Gemisch mit Wasser eingesetzt werden können.

O-Methylhydroxylamin der Formel (XII) kann als freie Base oder auch als Salz einer Säure eingesetzt werden. Im letzteren Fall arbeitet man in Gegenwart einer Base, vorzugsweise Natriumacetat. Die Verbindung der Formel (XII) wird im allgemeinen in äquimolaren Mengen eingesetzt.

Die Reaktionstemperatur kann in einem größeren Bereich variiert werden. Im allgemeinen arbeitet man bei Temperaturen zwischen -20°C und 100°C, bevorzugt zwischen 0°C und 60°C.

Die Umsetzung wird im allgemeinen unter Normaldruck durchgeführt.

Die Aufarbeitung erfolgt in üblicher Weise z.B. durch Filtration oder Extraktion.

Das Verfahren F) zur Herstellung von Verbindungen der Formel (XIII) ist dadurch gekennzeichnet, daß man die Verbindung der Formel (XI) mit einem Reduktionsmittel in Gegenwart einer Säure und gegebenenfalls in Gegenwart eines Verdünnungsmittels umsetzt.

Als Verdünnungsmittel kommen alle gegenüber den Reaktionsteilnehmern inerten Lösungsmittel in Frage. Vorzugsweise verwendet werden Ether, wie z.B. Diisopropylether, Methyl-tert.-butylether, Tetrahydrofuran, 1,2-Dimethoxyethan oder Dioxan.

Als Reduktionsmittel wird bevorzugt Natriumboranat in einer äquimolaren Menge oder im Überschuß verwendet.

Als Säure wird bevorzugt Trifluoressigsäure in einer äquimolaren Menge oder im Überschuß verwendet.

Die Reaktionstemperatur kann in einem größeren Bereich variiert werden. Im allgemeinen arbeitet man zu Beginn der Umsetzung bei Temperaturen zwischen 0°C und 50°C und erhöht die Temperatur im Verlauf der Umsetzung gegebenenfalls auf bis zu 120°C.

Die Umsetzung wird im allgemeinen unter Normaldruck durchgeführt.

Die Aufarbeitung erfolgt mit Hilfe üblicher Methoden.

Das Reaktionsprodukt der Formel (XIII) wird vorzugsweise in Form von Salzen, beispielsweise der Hydrochloride, isoliert.

Das Verfahren Gα) ist dadurch gekennzeichnet, daß man die Verbindung der Formel (XIII) gegebenenfalls in Gegenwart einer Base und gegebenenfalls in Gegenwart eines Verdünnungsmittels mit einem 2-A,6-B-Benzoylchlorid umsetzt.

Als Verdünnungsmittel können alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Base kommen bei der Umsetzung alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat und Alkali- oder Erdalkalihydroxide wie Natriumhydroxid oder Kaliumhydroxid.

Die Reaktionstemperaturen können innerhalb eines größeren Bereiches variiert werden. Im allgemeinen arbeitet man bei Temperaturen zwischen -20°C und +100°C, vorzugsweise zwischen 0°C und 30°C.

Die Umsetzung wird im allgemeinen unter Normaldruck durchgeführt.

Bei der Durchführung werden die Ausgangsstoffe der Formel (XIII) und das Benzoylchlorid im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren Gβ) zur Herstellung der Verbindung der Formel (XIV) ist dadurch gekennzeichnet, daß man zunächst (1. Stufe) die Verbindung der Formel (II) mit einer Verbindung der Formel (XV) in Gegenwart eines sauren Katalysators und gegebenenfalls in Gegenwart eines Verdünnungsmittels umsetzt und anschließend (2. Stufe) die so erhaltene Verbindung der Formel (XVI) mit einem Reduktionsmittel in Gegenwart eines Verdünnungsmittels umsetzt.

Als Verdünnungsmittel kommen in der ersten Stufe alle gegenüber den Reaktionsteilnehmern inerten Lösungsmittel in Frage.

Vorzugsweise verwendet werden Kohlenwasserstoffe wie Toluol, Xylol, Tetralin, Halogenkohlenwasserstoffe wie Methylenchlorid, Chloroform, Chlorbenzol, o-Dichlorbenzol, Ether wie Diisopropylether, Methyl-tert.-butylether, Tetrahydrofuran, Dioxan, Dimethoxyethan, Ketone wie Aceton oder Methylethylketon, Alkohole wie Methanol, Ethanol, Propanol, Amide wie Dimethylformamid, Sulfoxie wie Dimethylsulfoxid.

Als saurer Katalysator kommen prinzipiell alle anorganischen oder organischen Säuren oder Lewis-Säuren in Frage. Bevorzugt verwendet werden beispielsweise Schwefelsäure, Methansulfonsäure, Benzolsulfonsäure, Aluminiumchlorid, Titantetrachlorid, Phosphoroxychlorid, Bortrifluorid-Etherat. Ein Überschuß an Säure kann gegebenenfalls auch als Verdünnungsmittel dienen.

Die Reaktionstemperatur kann innerhalb eines größeren Bereichs variiert werden. Im allgemeinen arbeitet man bei Temperaturen zwischen -20°C und 150°C, bevorzugt zwischen 0°C und 50°C.

Die Umsetzung wird im allgemeinen unter Normaldruck durchgeführt.

Die Verbindung der Formel (II) und die Verbindung der Formel (XV) werden im allgemeinen in äquimolaren Mengen eingesetzt, es ist jedoch auch möglich, einen Überschuß der einen oder anderen Verbindung zu verwenden.

Als Verdünnungsmittel kommen in der zweiten Stufe insbesondere Alkohole und Ether in Frage. Genannt seien beispielsweise Methanol, Ethanol, die isomeren Propanole, Butanole und Pentanole, ferner Diethylether, Diisopropylether, Methyltert.-butylether, Tetrahydrofuran, Dioxan und Dimethoxyethan.

Als Reduktionsmittel wird bevorzugt Natriumborhydrid in einer Menge von 1 bis 5 mol, bezogen auf 1 mol der Verbindung der Formel (XVI), verwendet.

Wenn die Verbindung der Formel (XVI) als Säure vorliegt (R⁶ = H), ist diese vor der Umsetzung mit Natriumborhydrid in einen Alkylester zu überführen.

Die Reaktionstemperatur kann innerhalb eines größeren Bereichs variiert werden. Im allgemeinen arbeitet man bei Temperaturen zwischen 20°C und 150°C, bevorzugt zwischen 50°C und 100°C.

Die Umsetzung wird im allgemeinen unter Normaldruck durchgeführt.

Es wird mit Hilfe üblicher Methoden aufgearbeitet.

Das Verfahren Hα) zur Herstellung von Verbindungen der Formel (XVII) ist dadurch gekennzeichnet, daß man Verbindungen der Formel (XIV) mit einem Chlorierungsmittel gegebenenfalls in Gegenwart eines Verdünnungsmittels umsetzt.

Als Verdünnungsmittel kommen alle inerten organischen Lösungsmittel in Frage. Bevorzugt verwendet werden Kohlenwasserstoffe wie Toluol, Xylol, Hexan, Cyclohexan, Halogenkohlenwasserstoffe wie Chlorbenzol, Chloroform, Methylenchlorid und Ether wie Diethylether, Diisopropylether, Dimethoxyethan, Tetrahydrofuran, Dioxan.

Als Chlorierungsmittel kommen alle für diesen Zweck üblicherweise verwendbaren Reagenzien in Frage. Genannt seien beispielsweise Thionylchlorid, Phosgen und Phosphoroxychlorid, die im allgemeinen in mindestens äquimolarer Menge eingesetzt werden.

Die Reaktionstemperatur kann in einem größeren Bereich variiert werden. Im allgemeinen arbeitet man bei Temperaturen zwischen 0°C und 120°C, bevorzugt zwischen 20°C und 100°C.

Die Umsetzung wird gegebenenfalls in Gegenwart einer Base, insbesondere eines tertiären Amins, wie z.B. Triethylamin oder Pyridin, durchgeführt.

Das Verfahren Hβ) zur Herstellung von Verbindungen der Formel (XVII) ist dadurch gekennzeichnet, daß man Verbindungen der Formeln (II) bzw. (IIe) mit Verbindungen der Formel (XVIII) in Gegenwart eines sauren Katalysators und gegebenenfalls in Gegenwart eines Verdünnungsmittels umsetzt.

Als Verdünnungsmittel kommen alle gegenüber den Reaktionsteilnehmern inerten Lösungsmittel in Frage.

Vorzugsweise verwendet werden Kohlenwasserstoffe, wie Toluol, Xylol, Tetralin, Halogenkohlenwasserstoffe wie Methylenchlorid, Chloroform, Chlorbenzol, o-Dichlorbenzol, Ether wie Diisopropylether, Methyl-tert.-butylether, Tetrahydrofuran, Dioxan, Dimethoxyethan, Ketone wie Aceton oder Methylethylketon, Alkohole wie Methanol, Ethanol, Propanol, Amide wie Dimethylformamid, Sulfoxide wie Dimethylsulfoxid.

Als saurer Katalysator kommen prinzipiell alle anorganischen oder organischen Säuren oder Lewis-Säuren in Frage. Bevorzugt verwendet werden beispielsweise Schwefelsäure, Methansulfonsäure, Benzolsulfonsäure, wasserfreie Flußsäure, Aluminiumchlorid, Titantetrachlorid, Phosphoroxychlorid, Bortrifluorid-Etherat. Ein Überschuß an Säure kann gegebenenfalls auch als Verdünnungsmittel dienen.

Die Reaktionstemperatur kann innerhalb eines größeren Bereichs variiert werden. Im allgemeinen arbeitet man bei Temperaturen zwischen -20°C und 150°C, bevorzugt zwischen 0°C und 80°C.

Die Umsetzung wird im allgemeinen unter Normaldruck oder unter erhöhtem Druck durchgeführt.

Die Verbindungen der Formeln (II) bzw. (IIe) und die Verbindung der Formel (XVIII) werden im allgemeinen in äuqimolaren Mengen eingesetzt; es ist jedoch auch möglich, einen Überschuß der einen oder anderen Verbindung zu verwenden.

Das Verfahren I) zur Herstellung der Verbindungen der Formel (I) ist dadurch gekennzeichnet, daß man die Verbindungen der Formel (XVII) in Gegenwart einer Base, gegebenenfalls in Gegenwart eines Katalysators und gegebenenfalls in Gegenwart eines Verdünnungsmittels cyclisiert.

Als Verdünnungsmittel kommen alle inerten organischen Lösungsmittel in Frage. Sie können gegebenenfalls in Mischung mit Wasser verwendet werden. Bevorzugt verwendet werden Kohlenwasserstoffe wie Toluol, Xylol, Tetralin, Hexan, Cyclohexan, Halogenkohlenwasserstoffe wie Methylenchlorid, Chloroform, Chlorbenzol, o-Dichlorbenzol, Alkohole wie Methanol, Ethanol, Glykol, die isomeren Propanole, Butanole, Pentanole, Ether wie Diethylether, Diisopropylether, Dimethoxyethan, Tetrahydrofuran, Dioxan, Nitrile wie Acetonitril oder Butyronitril, Amide wie Dimethylformamid, Sulfoxide wie Dimethylsulfoxid, ferner Sulfolan. Besonders bevorzugt werden Alkohole verwendet.

Als Base kommen alle üblichen Säureakzeptoren in Frage.

Vorzugsweise verwendbar sind tertiäre Amine wie Triethylamin, Pyridin, DABCO, DBU, DBN, N,N-Dimethylanilin, ferner Erdalkalimetalloxide wie Magnesium-und Calciumoxid, außerdem Alkali- und Erdalkalimetallcarbonate wie Natriumcarbonat, Kaliumcarbonat und Calciumcarbonat, Alkalihydroxide wie Natrium- und Kaliumhydroxid, ferner Alkoholate wie Natriumethanolat oder Kalium-tert.-butylat.

Gegebenenfalls wird in Gegenwart eines Phasentransferkatalysators gearbeitet. Als Phasentransferkatalysator kommen beispielsweise Ammoniumverbindungen wie Tetraoctylammoniumbromid oder Benzyltriethylarnmoniumchlorid in Frage.

Die Reaktionstemperatur kann in einem größeren Bereich variiert werden. Im allgemeinen arbeitet man bei Temperaturen zwischen -10°C und 150°C, bevorzugt zwischen 0°C und 100°C.

Die Umsetzung wird im allgemeinen unter Normaldruck durchgeführt.

Im allgemeinen wird eine äquimolare Menge an Base eingesetzt. Es ist aber auch möglich, mit einem Basenüberschuß zu arbeiten.

Es wird in üblicher Weise aufgearbeitet.

Das Verfahren J) zur Herstellung von Verbindungen der Formel (Ib) ist dadurch gekennzeichnet, daß man die Verbindungen der Formel (Ia) verseift.

Das Verfahren J) wird vorzugsweise in Gegenwart eines Verdünnungsmittels durchgeführt. Als solches werden vorzugsweise Wasser/Alkohol-Gemische verwendet, wie beispielsweise Wasser/Methanol, Wasser/Ethanol oder Wasser/Propanol oder Wasser/Amidgemische wie beispielsweise Wasser/Dimethylformamid (DMF) oder Wasser/Dimethylacetamid.

Das Verfahren J) wird in Gegenwart einer Base durchgeführt. Als solche kommen anorganische und organische Basen in Frage, insbesondere Alkalihydroxide wie Natriumhydroxid oder Kaliumhydroxid oder Ammoniak.

Die Reaktionstemperatur kann in einem größeren Bereich variiert werden. Im allgemeinen arbeitet man bei Temperaturen zwischen -10°C und 60°C, bevorzugt zwischen 0°C und 40°C. Die Umsetzung wird im allgemeinen unter Normaldruck durchgeführt.

Das Verfahren Kα) ist dadurch gekennzeichnet, daß man eine Verbindung der Formel (Ib) mit einer Verbindung der Formel (XIX) gegebenenfalls in Gegenwart eines Verdünnungsmittels und gegebenenfalls in Gegenwart einer Base umsetzt.

Als Verdünnungsmittel können alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat und Alkali- oder Erdalkalihydroxide wie Natriumhydroxid oder Kaliumhydroxid.

Die Reaktionstemperaturen können innerhalb eines größeren Bereiches variiert werden. Im allgemeinen arbeitet man bei Temperaturen zwischen -20°C und +100°C, vorzugsweise zwischen 0°C und 30°C.

Die Umsetzung wird im allgemeinen unter Normaldruck durchgeführt.

Bei der Durchführung werden die Ausgangsstoffe der Formeln (Ib) und (XIX) im allgemeinen in angenähert äquivalenten Mengen verwendet. Es ist jedoch auch möglich, die Verbindung der Formel (XIX) in einem größeren Überschuß (bis zu 5 mol) einzusetzen. Die Aufarbeitung erfolgt nach üblichen Methoden.

Das Verfahren Kβ) ist dadurch gekennzeichnet, daß man eine Verbindung der Formel (Ib) mit einer Verbindung der Formel (IV) gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert.

Hinsichtlich Verdünnungsmittel, Base, Katalysator, Temperatur, Druck, Verhältnis von Base zum Ausgangsstoff der Formel (Ib), Durchführung und Aufarbeitung gilt das oben für Verfahren Aα) gesagte entsprechend.

Das Verfahren Kγ) ist dadurch gekennzeichnet, daß man eine Verbindung der Formel (Ib) mit einem Difluorhalogenmethan der Formel (V) gegebenenfalls in Gegenwart einer Base, gegebenenfalls in Gegenwart eines Katalysators und gegebenenfalls in Gegenwart eines Verdünnungsmittels umsetzt.

Hinsichtlich Verdünnungsmittel, Base, Katalysator, Temperatur, Druck und Mengenverhältnis der beteiligten Stoffe gilt das oben für Verfahren Aβ) gesagte entsprechend.

Das Verfahren Kδ) ist dadurch gekennzeichnet, daß man eine Verbindung der Formel (Ib) mit Tetrachlorkohlenstoff in Gegenwart von Fluorwasserstoffsäure, gegebenenfalls in Gegenwart eines Verdünnungsmittels umsetzt.

Hinsichtlich Verdünnungsmittel, Menge der Fluorwasserstoffsäure, Temperatur, Durchführung und Aufarbeitung gilt das oben für Verfahren Aδ) gesagte entsprechend.

Das Verfahren Kε) ist dadurch gekennzeichnet, daß man eine Verbindung der Formel (Ib) mit einer Verbindung der Formel (XX) gegebenenfalls in Gegenwart eines Verdünnungsmittels und gegebenenfalls in Gegenwart einer Base umsetzt.

Als Verdünnungsmittel kommen alle üblichen Lösungsmittel in Frage. Vorzugsweise verwendbar sind gegebenenfalls halogenierte, aromatische oder aliphatische Kohlenwasserstoffe, Ketone, Nitrile und Amide. Genannt seien beispielsweise Toluol, Aceton, Acetonitril Dimethylformamid und Dimethylacetamid.

Als Base kommen alle üblichen anorganischen und organischen Basen in Frage. Genannt seien beispielsweise tertiäre Amine wie Triethylamin, DBN, DBU, DABCO, Alkali- und Erdalkalihydroxide wie z.B. Natriumhydroxid, Kaliumhydroxid und Calciumhydroxid sowie Alkali- und Erdalkalicarbonate wie z.B. Natriumcarbonat oder Kaliumcarbonat.

Die Reaktionstemperatur kann innerhalb eines größeren Bereiches variiert werden. Im allgemeinen arbeitet man bei Temperaturen zwischen -20°C und 100°C, bevorzugt zwischen 0°C und 60°C.

Die Umsetzung wird im allgemeinen unter Normaldruck durchgeführt.

Die Verbindungen der Formel (Ib) und die Verbindungen der Formel (XX) werden im allgemeinen in angenähert äquimolaren Mengen eingesetzt. Man kann jedoch auch einen Überschuß der Verbindungen der Formel (XX) verwenden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz, der Veterinärmedizin sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria, Supella spp..

Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Phthirus spp., Pediculus spp., Haematopinus spp., Linognathus spp., Solenopotes spp..
Aus der Ordnung der Mallophaga z.B. Trichodectes spp., Damalinea spp., Trimenopon spp., Monopon spp., Trinoton spp., Bovicola spp., Werneckiella spp., Lepikentron spp., Felicola spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp., Panstrongylus spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinaniensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora spp., Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Hydrotaea spp., Stomoxys spp., Haematobia spp., Morellia spp., Fannia spp., Glossina spp., Calliphora spp., Wohlfahrtia spp., Sarcophaga spp., Oestrus spp., Hypoderma spp., Gasterophilus spp., Hippobosca spp., Lipoptena spp., Melophagus spp., Muscina spp..

Aus der Ordnung der Siphonapterida z.B. Xenopsylla spp., Ceratophyllus spp., Pulex spp., Ctenocephalides spp..
Aus der Ordnung der Arachnida z.B. Scorpio maurus, Latrodectus mactans.

Aus der Ordnung der Acarina z.B. Myocoptes spp., Otodectes spp., Acarus siro, Argas spp., Ornithodoros spp., Ornithonyssus spp., Dermanyssus spp., Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Dermacentor spp., Haemaphysalis spp., Raillietia spp., Pneumonyssus spp., Sternostorma spp., Varroa spp..

Aus der Ordnung der Actinedida (Prostigmata) und Acaridida (Astigmata) z.B.: Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alpha-methrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxyphenoxy-alkansäureester, wie z.B. Diclofop-methyl, Fenoxaprop-ethyl, Fluazifopbutyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise TaKapseln, Tränken, Drenchen, Granulaten, Pasten, Boli, des feed-through-Verfahrens, von Zäpfchen, durch parenterale Verabreichung, wie zum Beispiel durch Injektion (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Haltern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Wirkstoffe geht aus den nachfolgenden Beispielen hervor.

### Herstellungsbeispiele

### Beispiel (I-1)

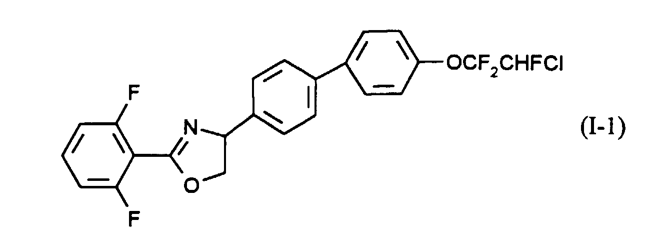

6,1 g (0,012 mol) der Verbindung gemäß Beispiel (XVII-1) werden in 50 ml trockenem Methanol suspendiert. Man tropft ohne zu Kühlen 6,4 g (0,048 mol) 30 %ige Natronlauge zu, wobei die Temperatur bis auf 27°C steigt. Man erhitzt ca. 20 Minuten zum Sieden und kühlt ab. Man filtriert ab, wäscht mit Wasser und trocknet.
Ausbeute: 4,8 g weiße Kristalle (84 % d.Th.)
Fp.: 101-103°C

### Beispiel (I-2)

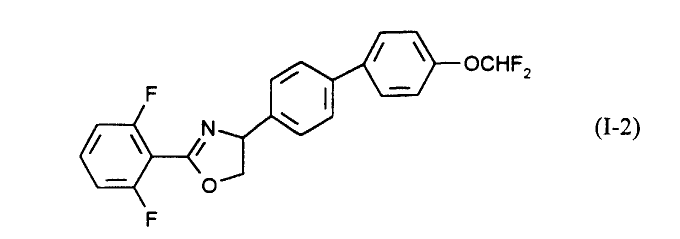

Man suspendiert 17,6 g (0,042 mol) 2,6-Difluor-N-[2-hydroxyethyl-1-phenyl-4-(4'-difluormethoxyphenyl)]-benzoesäureamid gemäß Beispiel (XIV)-1 in 150 ml Toluol, tropft 20 g (0,168 mol) Thionylchlorid zu und erhitzt auf 70°C. Man hält 1,5 Stunden bei dieser Temperatur, läßt abkühlen und destilliert das Toluol im Vakuum ab. Der Rückstand wird in 150 ml Methanol gelöst. Dann gibt man 22,4 g (0,168 mol) 30 %ige Natronlauge zu und erhitzt 20 Minuten auf 70°C. Nach Abkühlen engt man ein, nimmt in Methylenchlorid auf und wäscht dreimal mit Wasser. Nach Trocknen und Einengen bleiben 14,9 g gelbe Kristalle zurück, die über Kieselgel (Petrolether/Essigester 1:1) gereinigt werden. Man erhält 13,0 g weiße Kristalle vom Fp.: 112°C.
Ausbeute: 82 % d.Th.

Analog zu Beispiel I-1 oder I-2 und gemäß den allgemeinen Angaben zur Herstellung erhält man die folgenden Verbindungen der Formel (I).

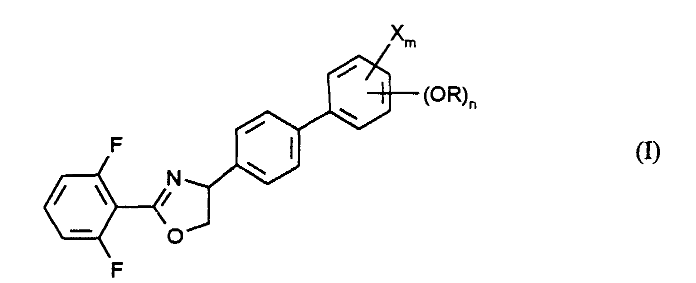

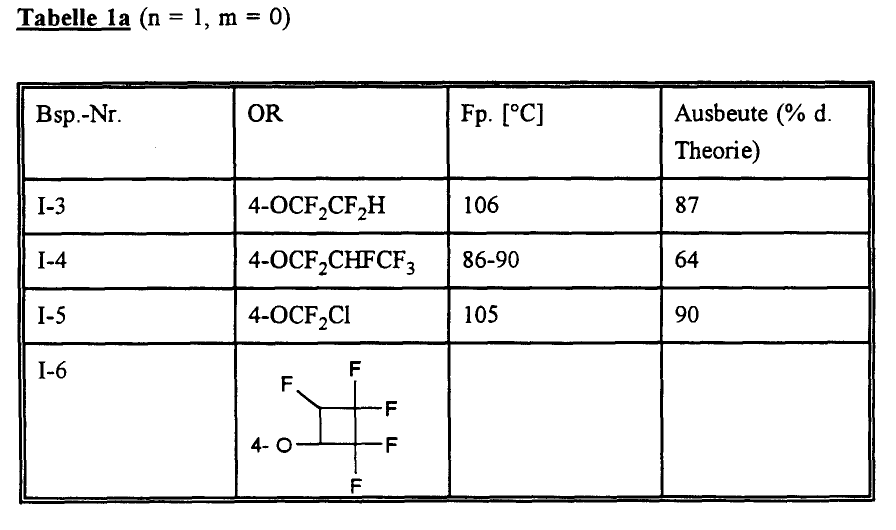

### Beispiel (I-7)

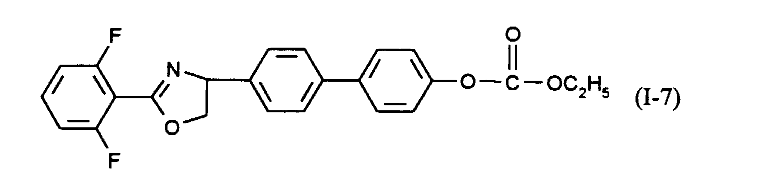

Zu 19,8 g (0,043 mol) der Verbindung gemäß Beispiel (XVII-3) in 120 ml Dimethylformamid werden bei 5°C 4 ml (0,045 mol) 50%ige Natronlauge getropft. Nach 2 Stunden bei Raumtemperatur rührt man in 500 ml Eiswasser ein und saugt die ausgefallenen Kristalle ab.
Ausbeute: 15,1 g (83 % der Theorie), Fp.: 98 bis 100°C.

### Beispiel (I-8)

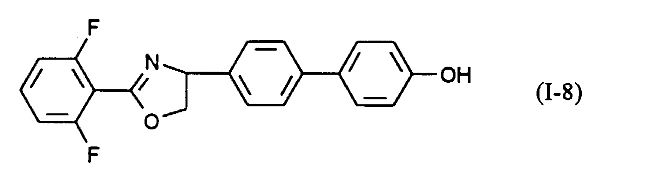

10,9 g (0,026 mol) der Verbindung gemäß Beispiel (I-7) werden in 50 ml Methanol suspendiert, dann werden bei Raumtemperatur 35,4 ml (0,52 mol) 25%ige wässrige Ammoniaklösung zugetropft. Nach 28 Stunden bei Raumtemperatur wird der Niederschlag abgesaugt und mit wenig Methanol gewaschen.
Ausbeute 7,6 g (97,4 % der Theorie), Fp. 180 bis 182°C.

### Beispiel (I-9) (Eintopfreaktion in DMF)

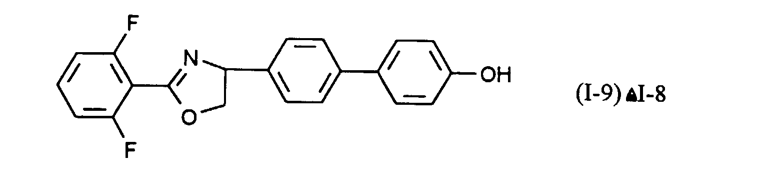

9,6 g der Verbindung gemäß Beispiel (XVII-3) werden wie in Beispiel (I-7) in DMF mit Natronlauge behandelt, dann wird bei 5°C mit verdünnter Salzsäure neutralisiert. Nach Zugabe der entsprechenden Menge Ammoniaklösung (vgl. Beispiel I-8) wird 15 Stunden gerührt, in Eiswasser eingetragen und abgesaugt.
Ausbeute 7 g (94,5 % der Theorie).

### Beispiel (I-10)

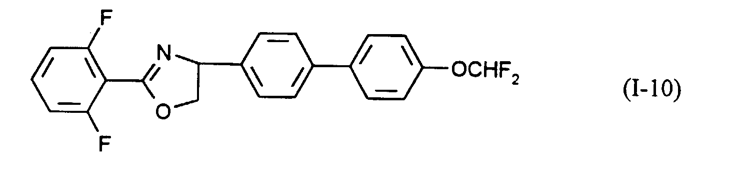

In eine Lösung von 1,2 g (3,4 mmol) der Verbindung gemäß Beispiel (I-8) und 2,7 g (50 mmol) KOH in 20 ml Isopropanol werden 3 Stunden lang bei 40 bis 50°C Difluorchlormethan eingeleitet. Nach dem Abkühlen gibt man auf 100 ml Eiswasser, extrahiert 2 mal mit je 50 ml Methylenchlorid, trocknet und destilliert das Lösungsmittel ab. Nach Chromatographie an Kieselgel mit Petrolether/Essigester 1/1 als Laufmittel erhält man 0,8 g (50 % der Theorie) farblose Kristalle vom Fp. 112°C.

### Beispiel (I-11)

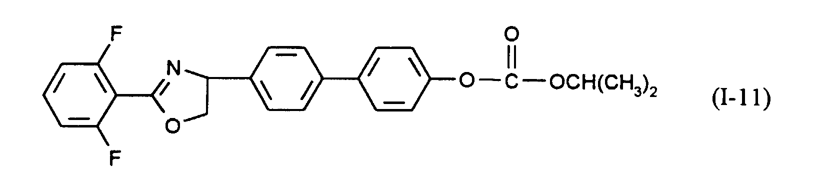

Zu 3,5 g (0,01 mol) der Verbindung gemäß Beispiel (I-8) suspendiert in 20 ml Essigsäureethylester gibt man 1,4 ml (0,01 mol) Triethylamin und tropft dann 1,2 g (0,01 mol) Chlorameisensäureisopropylester innerhalb von 15 Minuten zu, dabei erwärmt sich das Reaktionsgemisch auf 30°C. Man rührt 18 Stunden bei Raumtemperatur, wäscht mit 20 ml Wasser, trocknet die organische Phase und chromatographiert nach Einengen an Kieselgel mit Methylenchlorid als Laufmittel. Ausbeute 2,1 g (48 % der Theorie), Fp. 104°C.

Analog bzw. gemäß den allgemeinen Angaben zur Herstellung erhält man die folgenden Verbindungen der Formel (I):

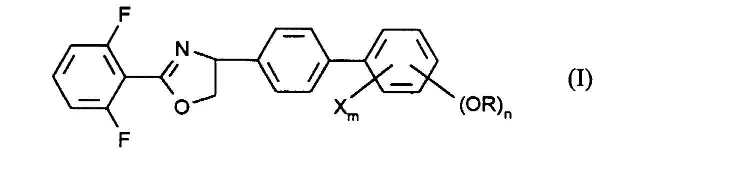

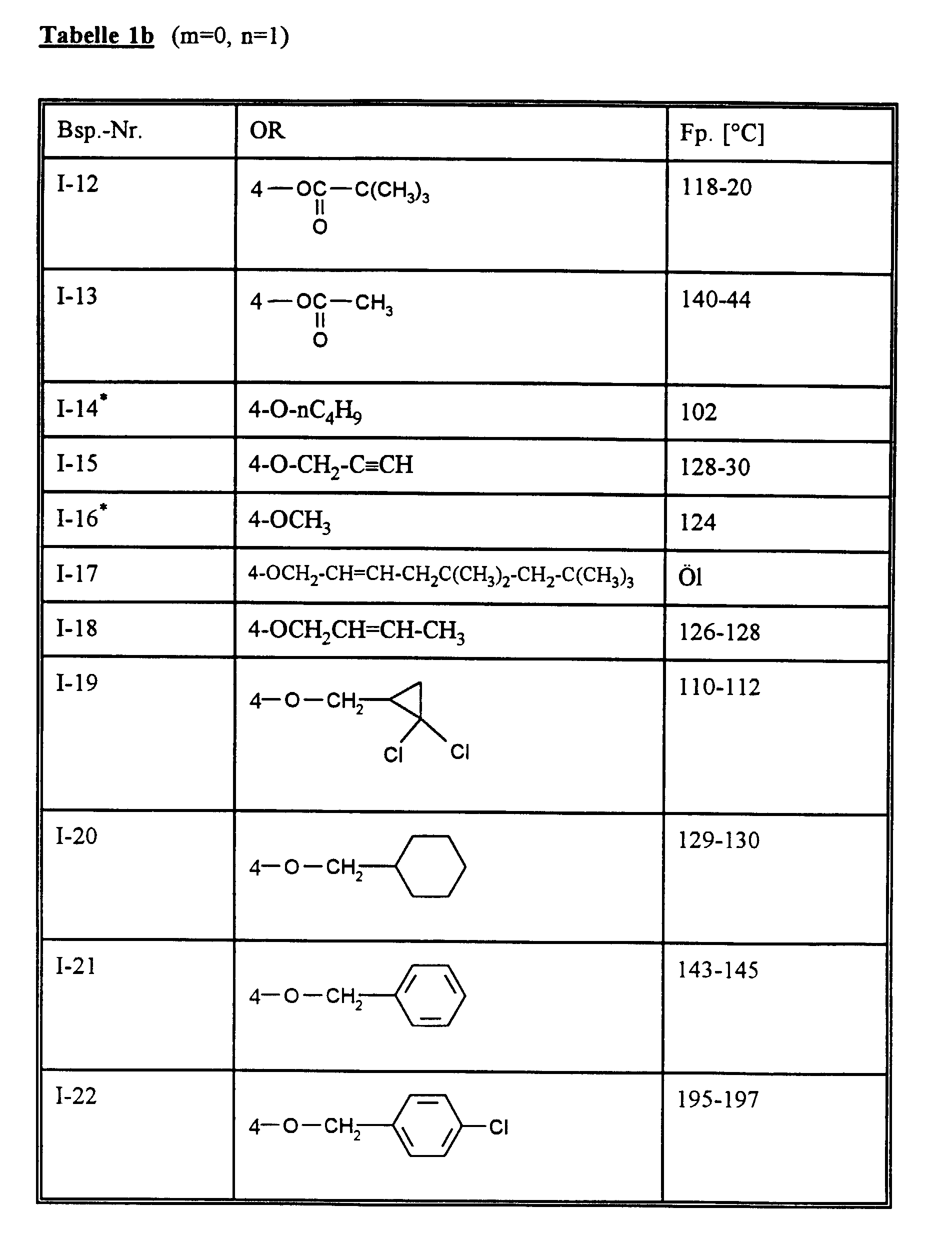

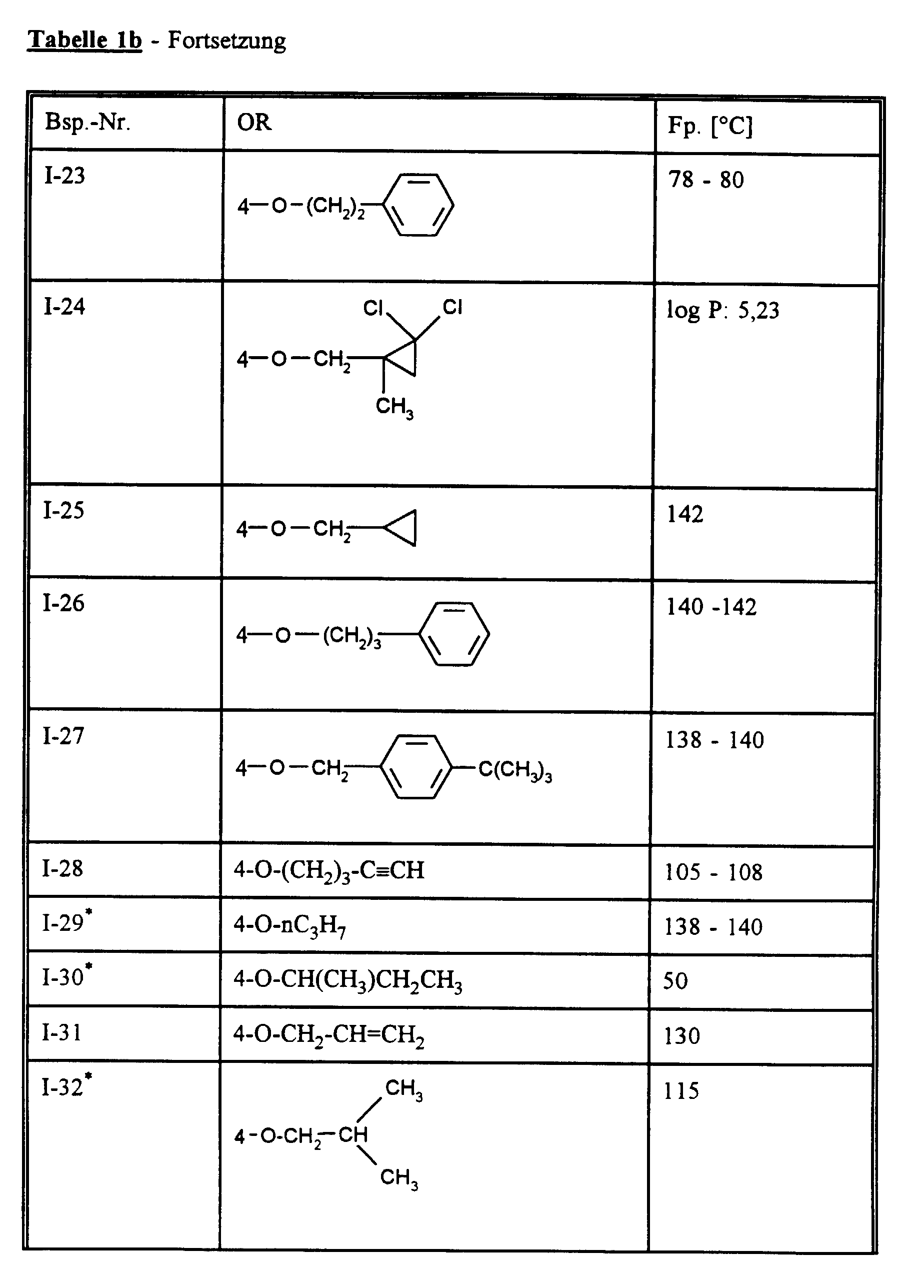

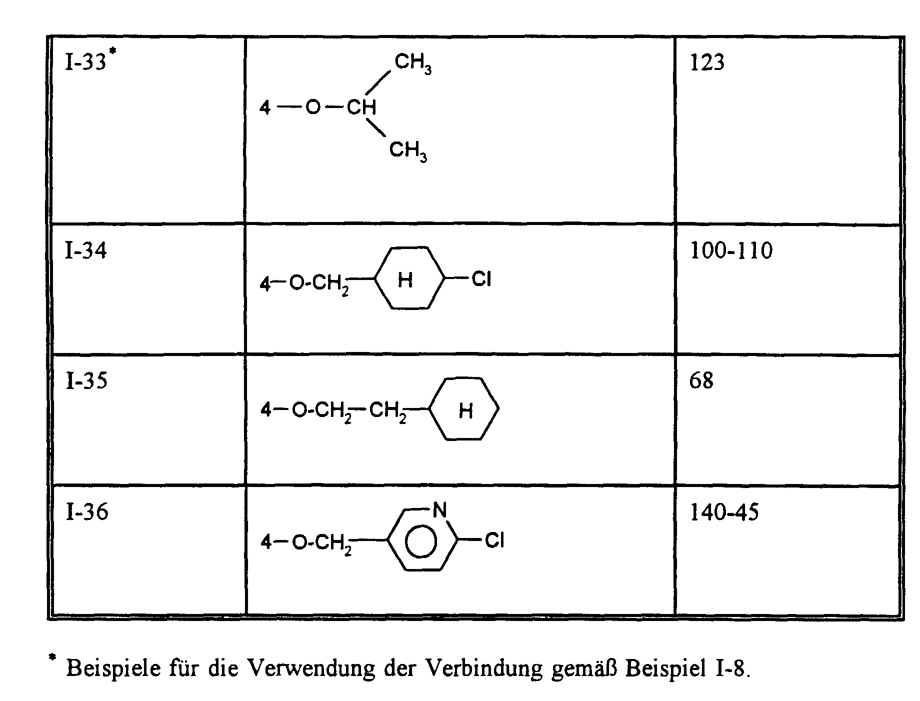

### Beispiel I-37

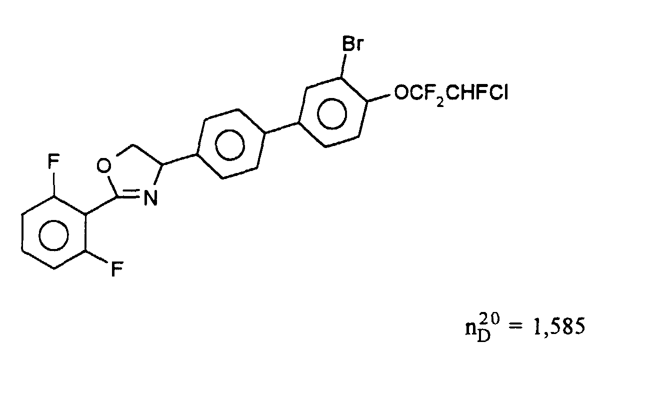

### Beispiel I-38

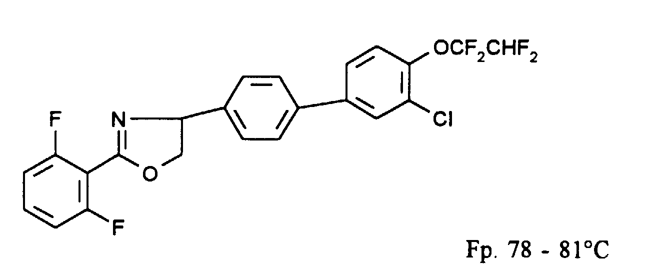

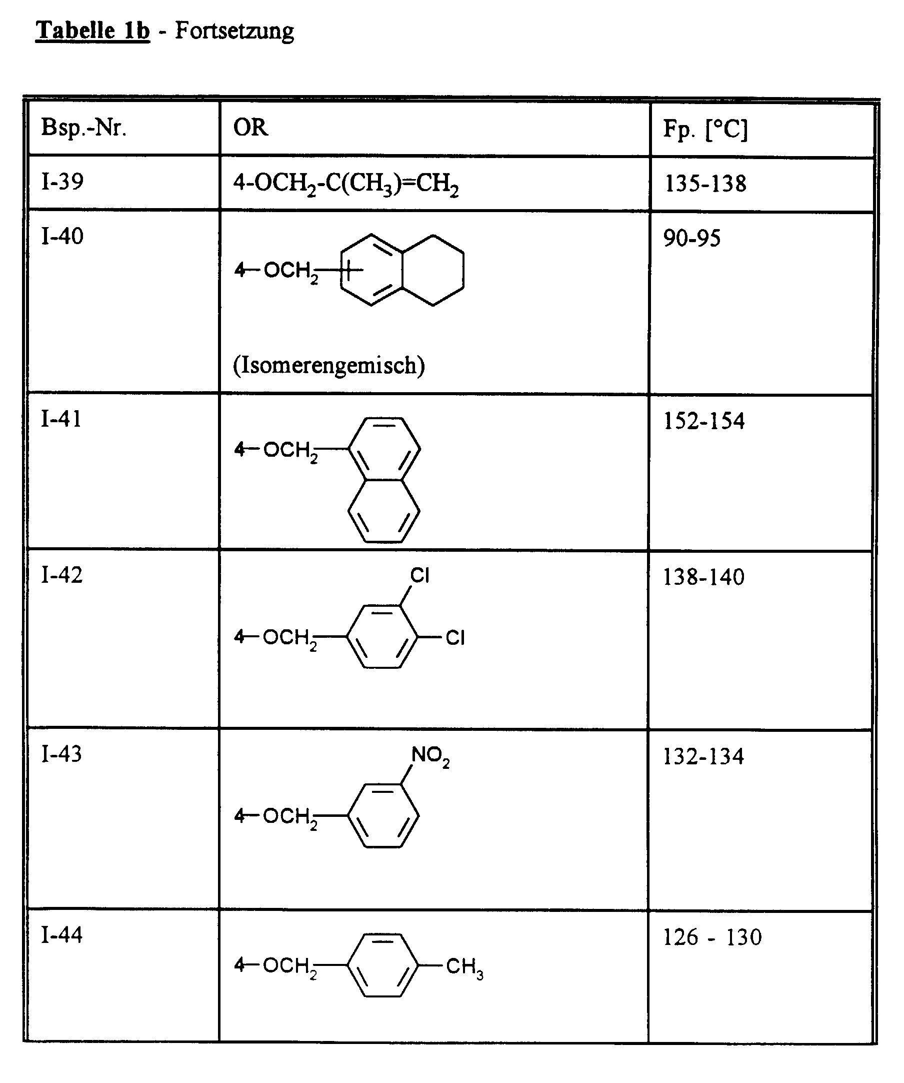

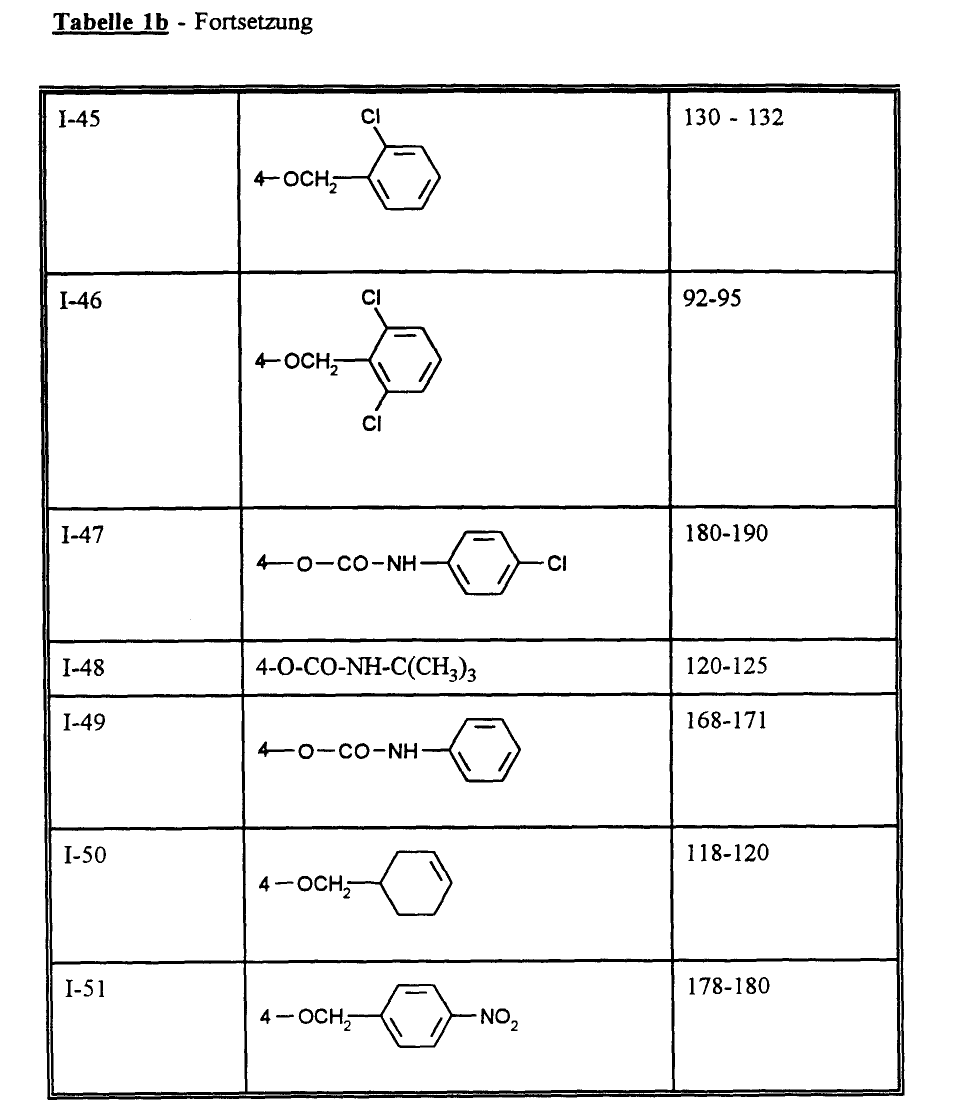

### Beispiel I-52

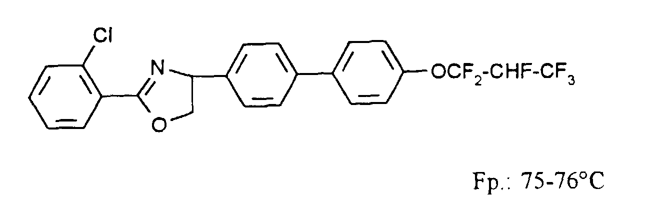

### Herstellung von Ausgangsverbindungen

### Beispiel (IIa-1)

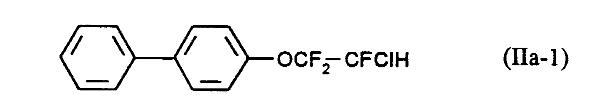

In einer Rührapparatur mit Gaseinleitungsrohr am Boden des Reaktionsgefäßes werden 250 g 4-Hydroxybiphenyl in 1 000 ml Aceton und 50 g gepulvertes Natriumhydroxid vorgelegt und die Reaktionsmischung zum Sieden erhitzt. Anschließend wird Trifluorchlorethylen bis zur Sättigung eingeleitet. Nach Abkühlen auf Raumtemperatur werden 2 000 ml Wasser zudosiert und die sich abscheidende organische Phase abgetrennt. Nach Trocknung wird das Produkt destilliert; Siedebereich: 170-174°C / 20 mbar. Ausbeute: 295 g.

### Beispiel (IIa-2)

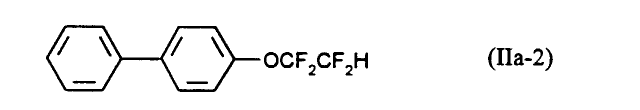

In einer Rührapparatur mit Gaseinleitungsrohr werden 250 g 4-Hydroxy-biphenyl, 1 000 ml N-Methylpyrrolidon und 50 g Natriumhydroxid gepulvert vorgelegt und auf 130°C erhitzt. Dann wird Tetrafluorethylen eingeleitet, bis kein Tetrafluorethylen mehr aufgenommen wird. Nach Abkühlen auf Raumtemperatur wird der Ansatz in 3 000 ml Wasser eingerührt und der Feststoff abgesaugt, mit Wasser gewaschen und im Vakuum getrocknet.

Die Auswaage beträgt 391 g 4-Tetrafluorethoxy-biphenyl = 98 % d. Th.; Schmelzpunkt: 65°C.

### Beispiel (IIa-3)

Analog werden 250 g 4-Hydroxybiphenyl mit Hexafluorpropen zu 4-Hexafluorpropoxy-biphenyl umgesetzt.

Ausbeute: 180 g; Siedepunkt: 96-98°C / 0,3 mbar, Schmelzpunkt: 60°C.

### Beispiel (IIb-1)

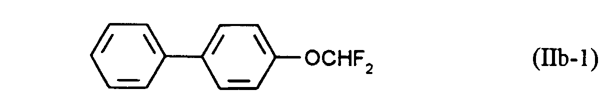

51,1 (0,3 mol) 4-Hydroxybiphenyl werden in 250 ml Dioxan gelöst und mit 38,3 ml 45 %iger Natronlauge versetzt. Man erhitzt auf 60°C und leitet dann bei dieser Temperatur 100 g (1,15 mol) Difluorchlormethan ein. Nach Abkühlen wird mit 75 ml Wasser versetzt, der unlösliche Niederschlag abgesaugt und die Wasserphase dreimal mit tert.-Butylmethylether extrahiert. Die vereinigten organischen Phasen werden getrocknet und eingeengt. Man erhält 55,9 g Rohprodukt, das noch 20,9 % Ausgangsphenol enthält, das mit Natronlauge entfernt wird.
Ausbeute: 40 g (60 % der Theorie)
Fp.: 35°C

### Beispiel (IIe-1)

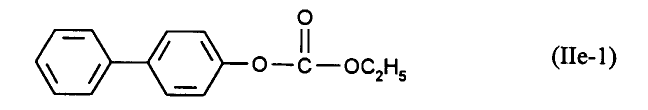

Zu 34 g (0,2 mol) 4-Hydroxybiphenyl und 28 ml Triethylamin in 200 ml Essigsäureethylester tropft man bei 10°C innerhalb von 15 Minuten 22 g (0,2 mol) Chlorameisensäureethylester. Man rührt noch 30 Minuten bei Raumtemperatur, saugt den Niederschlag ab und wäscht die flüssige Phase mit 200 ml Wasser, trocknet sie und engt im Vakuum ein. Der Rückstand wird mit 50 ml Diisopropylether aufgenommen und die Kristalle abgesaugt. Ausbeute 47,5 g (98,5 % der Theorie), Fp. 70°C.

### Beispiel (Ic-1)

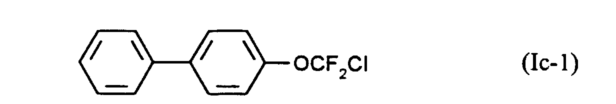

800 ml Benzol, 40 g Fe-Pulver und 350 g 4-Difluormethoxyanilin werden vorgelegt, bei 30-38°C wird eine Lösung von 1000 g Trichloressigsäure in 1600 ml Benzol in 5 Std. zugetropft. Gleichzeitig werden 210 g Natriumnitrit portionsweise zugegeben (alle 15 Minuten 12 g). Die Reaktion ist leicht exotherm, während der Zugabe Gasentwicklung. Nach Ende der Zugabe wird etwa 20 Std. bei Raumtemperatur nachgerührt. Anschließend wird im Maße der Gasentwicklung bis auf Rückflußtemperatur (75-76°C) erhitzt und so lange nachgerührt, bis die Gasentwicklung beendet ist (ca. 4 Std.). Der Ansatz wird abgekühlt, 2,4 Liter 5 %ige Salzsäure zugegeben und zunächst Benzol bis Innentemperatur 100°C abdestilliert. Anschließend wird eine Wasserdampfdestillation durchgeführt. Die organische Phase (flüssig) wird abgetrennt, mit Wasser gewaschen, getrocknet und destilliert.
Ausbeute: 109 g, Smp. 55°C
Siedepunkt: 135-140°C/26 mbar

### Beispiel (VII-1)

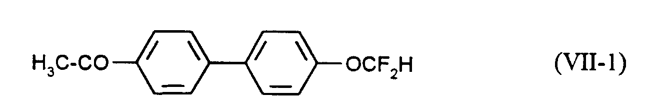

5,3 g (0,025 mol) 4-Acetyl-4'-hydroxybiphenyl werden in 35 ml Toluol vorgelegt. Zu der Suspension werden bei 60°C 6,7 g (0,075 mol) 45 %ige Natronlauge getropft. Anschließend werden 0,42 g (0,00125 mol) Tetrabutylphosphoniumbromid zugefügt und in 2 Stunden bei 95 bis 100°C 20 g (0,23 mol) Difluorchlormethan eingeleitet. Man kühlt ab, verdünnt mit Wasser und filtriert ab (0,4 g). Die Toluolphase wird abgetrennt und die Wasserphase zweimal mit Toluol extrahiert. Die vereinigten Toluolphasen werden mit Wasser gewaschen, getrocknet und eingeengt. Man erhält 5,9 g (83 % d.Th.) beige Kristalle vom Fp. 79-81°C.

### Beispiel (VIIa-1)

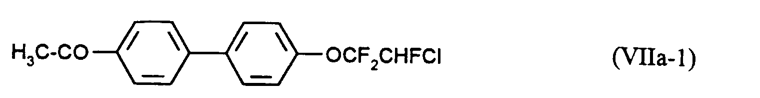

In eine Mischung aus 50 g 4-Acetyl-4'-hydroxybiphenyl, 400 ml Acetonitril, 60 ml Wasser und 10 g Kaliumhydroxid werden bis zur Sättigung bei 40°C Trifluorchlorethylen eingeleitet. Dann wird das Acetonitril entfernt und der Rückstand mit 100 ml Wasser verrührt. Das Festprodukt wird abgesaugt, getrocknet. Anschließend wird das Rohprodukt mit 20 ml heißem Cyclohexan verrührt und erneut filtriert.
Ausbeute: 45 g
Fp.: 92-94°C

### Beispiel (VIIa-2)

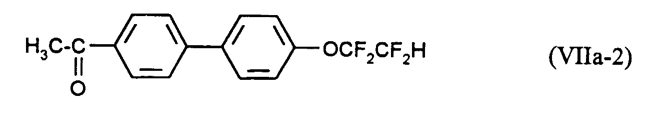

Analog Beispiel (VIIa-1) werden aus 96 g 4-Acetyl-4'-hydroxybiphenyl und Tetrafluorethylen 107 g Rohprodukt erhalten, die in Methyl-tert.-butylether gelöst und mit 300 ml 5 %iger Natronlauge extrahiert werden. Die Etherphase wird eingeengt.
Ausbeute: 58 g
Fp.: 95-97°C

### Beispiel (VIIa-3)

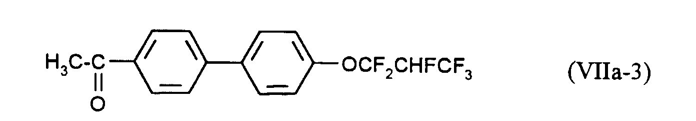

Analog Beispiel (VIIa-1) wurden aus 50 g 4-Acetyl-4'-hydroxybiphenyl und Hexafluorpropen 59 g Produkt erhalten.
Fp.: 86-87°C

### Beispiel (VIII-1)

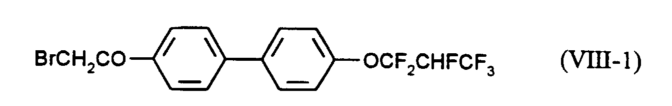

29 g (0,08 mol) 4-Hexafluorpropoxyphenylacetophenon gemäß Beispiel (VIIa-3) werden in 200 ml Methanol suspendiert und 13,4 g (0,084 mol) Brom bei 0°C zugetropft. Man rührt 12 Stunden nach und engt ein. Man erhält 41,3 g braune Kristalle, die an Kieselgel mit Methylenchlorid als Laufmittel gereinigt werden.
Ausbeute: 24,6 g (71 % der Theorie) gelbe Kristalle vom Fp. 69-71°C.

Analog und gemäß den allgemeinen Angaben zur Herstellung erhält man die folgenden Verbindungen der Formel (VIII):

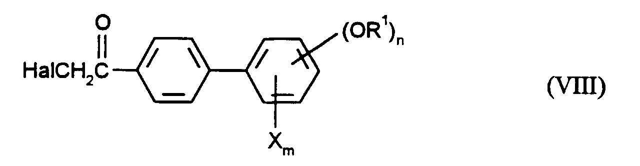

**Tabelle 2**

| (m = 0, n = 1) | | | | |
|---|---|---|---|---|
| Bsp.-Nr. | OR¹ | Hal | Fp. [°C] | Ausbeute (% d.Th.) |
| VIII-2 | 4-OCHF₂ | Br | 83-85 | 62 |
| VIII-3 | 4-OCF₂CHFCl | Br | 72-74 | 63 |
| VIII-4 | 4-OCF₂CF₂H | Br | 56-61°C | 85 |

### Beispiel (VIII-5)

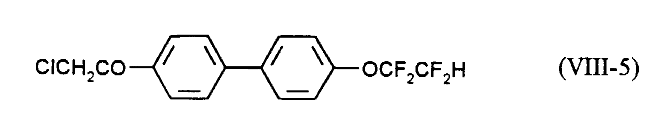

7 g (0,0525 mol) Aluminiumchlorid werden in 50 ml 1,2-Dichlorethan suspendiert. Dann wird bei 5 bis 10°C 5,9 g (0,0525 mol) Chloracetylchlorid zugetropft. Anschließend werden 13,5 g (0,05 mol) 4-Tetrafluorethoxybiphenyl in 10 ml Dichlorethan gelöst bei -10°C zugetropft. Man hält noch eine Stunde bei -10°C und rührt dann 12 Stunden bei Raumtemperatur nach. Das Reaktionsgemisch wird auf 150 ml Eiswasser gegossen, mit Essigester versetzt und die organische Phase abgetrennt. Man wäscht mit Bicarbonatlösung, dann zweimal mit Wasser, trocknet und engt ein. Das Rohprodukt wird über Kieselgel (Laufmittel Methylenchlorid) gereinigt. Man erhält 14,7 g hellbeige Kristalle (Ausbeute 85 % d.Th.) vom Fp. 74°C.

### Beispiel (X-1)

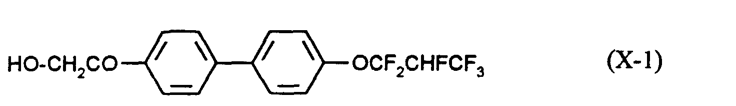

24,3 g (0,055 mol) Bromketon gemäß Beispiel (VIII-1) werden in 145 ml Ethanol/Wasser vorgelegt. Zu der Suspension gibt man 22,5 g (0,33 mol) Natriumformiat und erhitzt 12 Stunden zum Sieden. Das Ethanol wird z.T. abdestilliert, dann wird abfiltriert. Nach Waschen mit Wasser und Abfiltrieren wird getrocknet. Man erhält 20,7 g (93 % d.Th.) Kristalle vom Fp. 135°C.

Analog und gemäß den allgemeinen Angaben zur Herstellung erhält man die folgenden Verbindungen der Formel (X)

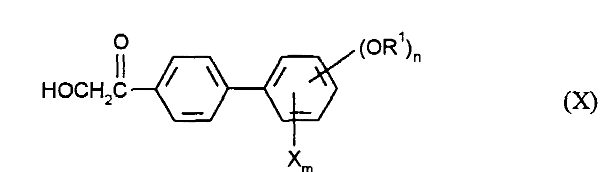

**Tabelle 3**

| (m = 0, n = 1) | | | |
|---|---|---|---|
| Bsp.-Nr. | OR¹ | Fp. [°C] | Ausbeute [% d.Th.) |
| X-2 | 4-OCHF₂ | 115-118 | 91 |
| X-3 | 4-OCF₂CHFCl | 160 | 97 |
| X-4 | 4-OCF₂CF₂H | 155-160 | 90 |

### Beispiel (XI-1)

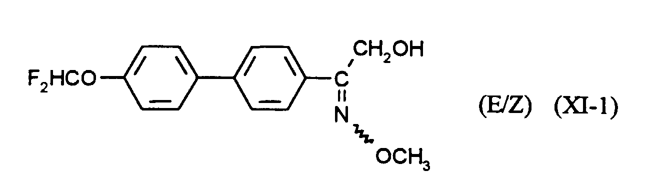

5 g (0,018 mol) 4-Difluormethoxyphenyl-ω-hydroxyacetophenon gemäß Beispiel (X-2) werden in 50 ml 1,2-Dimethoxyethan gelöst und mit 5 ml Wasser versetzt. Anschließend werden 1,85 g (0,0225 mol) Natriumacetat und 1,9 g (0,0225 mol) O-Methylhydroxylamin-Hydrochlorid zugegeben und über Nacht bei Raumtemperatur gerührt. Man gießt das Reaktionsgemisch auf 180 ml Eiswasser, saugt ab, wäscht mit Wasser und trocknet. Man erhält 5,4 g hellbeige Kristalle vom Fp. 65-81°C (E/Z-Gemisch: 43/57).

Analog und gemäß den allgemeinen Angaben zur Herstellung erhält man die folgenden Verbindungen der Formel (XI)

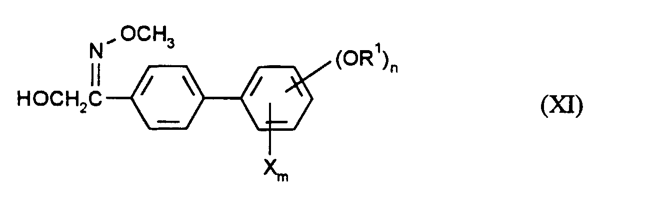

**Tabelle 4**

| (m = 0, n = 1) | | | |
|---|---|---|---|
| Bsp.-Nr. | OR¹ | Fp. [°C] | Ausbeute (% d. Th.) |
| XI-2 | 4-OCF₂CHFCl | *) | 89 |
| XI-3 | 4-OCF₂CHF₂ | *) | 83 |
| XI-4 | 4-OCF₂CHFCF₃ | *) | 82 |

| | | | |
|---|---|---|---|
| *) Da die entstehenden E/Z-Gemische einen sehr weiten Schmelzbereich besitzen, ist die Angabe des Schmelzpunktes nicht charakteristisch. | | | |

### Beispiel (XIII-1)

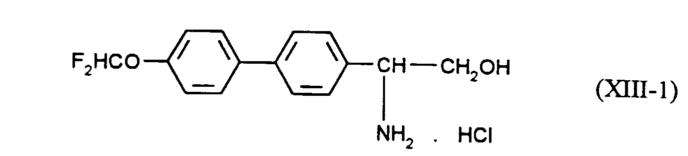

9,2 g (0,2425 mol) Natriumboranat werden in 250 ml absol. THF vorgelegt, dann werden bei 20°C innerhalb von 10 Minuten 27,6 g (0,2425 mol) Trifluoressigsäure in 25 ml THF zugetropft. Anschließend werden zum trüben, farblosen Reaktionsgemisch bei 15°C in 15 Minuten 14,9 g (0,0485 mol) der Oximether gemäß Beispiel (XI-1) gelöst in 25 ml THF zugetropft. Man rührt 2 Stunden bei Raumtemperatur, dann 2 h bei Siedetemperatur nach. Nach dem Abkühlen wird vorsichtig bei ca. 10°C mit 150 ml Wasser versetzt und 12 Stunden bei Raumtemperatur nachgerührt. Man filtriert ab und engt ein. Nach Verrühren mit 2n HCl werden die weißen Kristalle abgesaugt, mit Methylenchlorid gewaschen und getrocknet.

Man erhält 13 g weiße Kristalle vom Fp. 180°C (Zers.).
Ausbeute: 82 % d.Th.

### Beispiel (XIII-2)

Analog Beispiel (XIII-1) erhält man die Verbindung der Formel 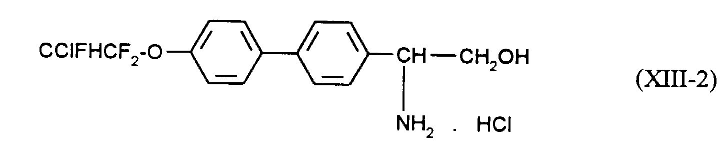 vom Fp. 197°C (Zersetzung).
Ausbeute: 91 % d.Th.

### Beispiel (XIII-3)

Analog Beispiel (XIII-1) erhält man die Verbindung der Formel 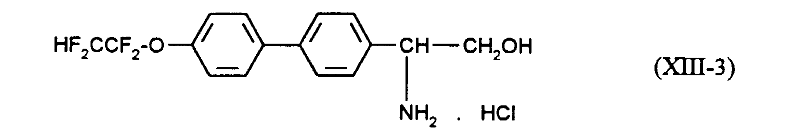 vom Fp. 250°C (Zersetzung).
Ausbeute: 53 % d.Th.

### Beispiel (XIV-1)

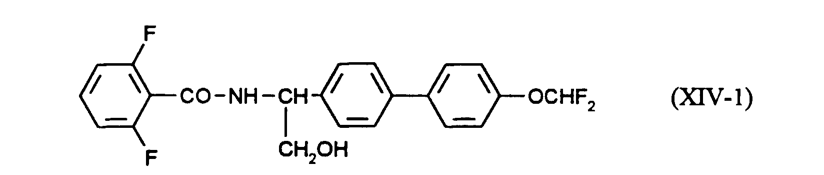

Zu einer Mischung aus 2,5 g (0,0086 mol) 2-Amino-2-phenyl-(4-difluormethoxyphenyl)-ethanol gemäß Beispiel (XIII-1) und 0,9 g (0,009 mol) Triethylamin in 50 ml THF werden 1,52 g (0,0086 mol) 2,6-Difluorbenzoylchlorid getropft (bei 10 bis 20°C). Man rührt 3 Stunden nach, engt ein und nimmt in Methylenchlorid auf, wäscht dreimal mit Wasser, trocknet und engt erneut ein.

Man erhält 3,3 g hellbeige Kristalle vom Fp. 146°C.
Ausbeute: 83,5 % d.Th.

Analog und gemäß den allgemeinen Angaben zur Herstellung erhält man die folgenden Verbindungen der Formel (XIV):

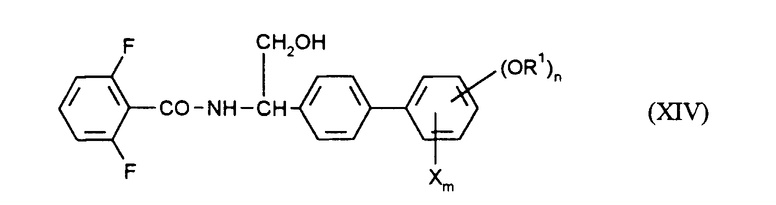

**Tabelle 5**

| (m = 0, n = 1) | | | |
|---|---|---|---|
| Bsp.-Nr. | OR¹ | Fp [°C] | Ausbeute [% d. Th.] |
| XIV-2 | 4-OCF₂CHFCl | 194-195 | 54 |
| XIV-3 | 4-OCF₂CHFCF₃ | 142-145 | 73 |
| XIV-4 | 4-OCF₂CF₂H | 184-186 | 81 |

### Beispiel (XVII-1)

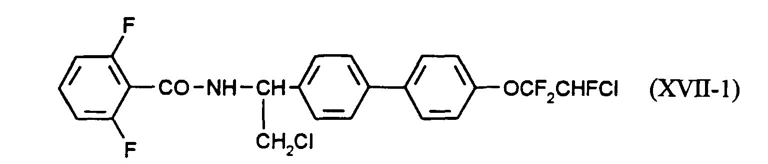

10,6 g (0,022 mol) Hydroxyethylamid gemaß Beispiel (XIV-2) werden in 100 ml trockenem Toluol suspendiert. Man tropft 10,5 g (0,088 Mol) Thionylchlorid ohne zu Kühlen zu, wobei eine klare gelbe Lösung entsteht. Man heizt auf 70°C und hält 2 Stunden bei dieser Temperatur. Nach Abkühlen auf 0°C filtriert man ab, wäscht mit wenig Toluol nach und trocknet.

Man erhält 8,8 g (79 % d. Th.) weiße Kristalle vom Fp. 190-192°C.

### Beispiel (XVII-2)

Analog Beispiel XVII-1 erhält man 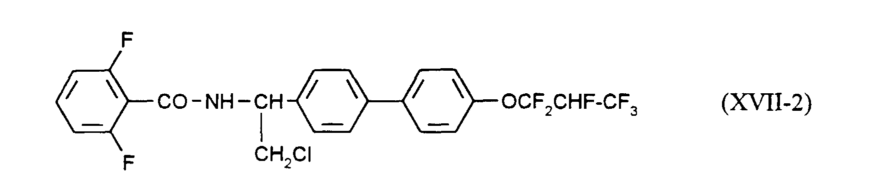 weiße Kristalle vom Fp. 161-164°C (83 % d. Th.)

### Beispiel (XVII-3)

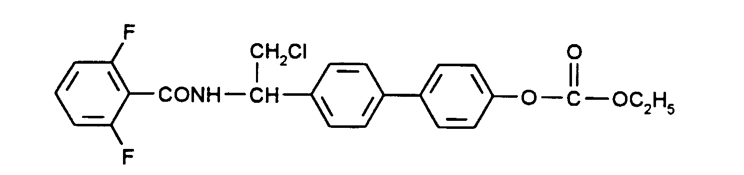

In das Gemisch von 53 g (0,213 mol) 2-(2,6-Difluorbenzoylamido)-2-methoxy-1-chlorethan, 48,4 g (0,2 mol) 4-Ethoxycarbonyloxybiphenyl und 12 ml Eisessig in 200 ml Methylenchlorid werden bei 5°C innerhalb von 30 Minuten 130,4 g (0,98 mol) Aluminiumchlorid portionsweise eingetragen. Dabei verfärbt sich der Ansatz über blau nach rotviolett. Man rührt eine Stunde bei 5°C und 1 Stunde bei 10°C, gibt das Reaktionsgemisch vorsichtig auf Eis, dekantiert die Suspension vorsichtig von Wasser ab, engt am Rotationsverdampfer ein, versetzt den Rückstand mit 50 ml Acetonitril und saugt die ausgefallenen Kristalle ab. Ausbeute 42,8 g (47 % der Theorie), Fp. 209°C.

Analog bzw. gemäß den allgemeinen Angaben zur Herstellung erhält man die folgenden Verbindungen der Formel (XVII):

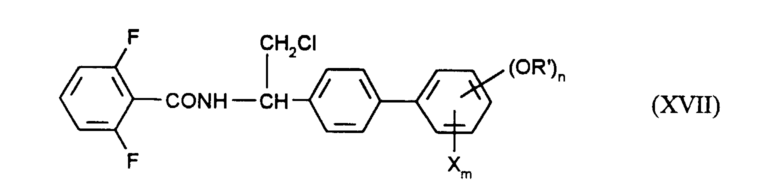

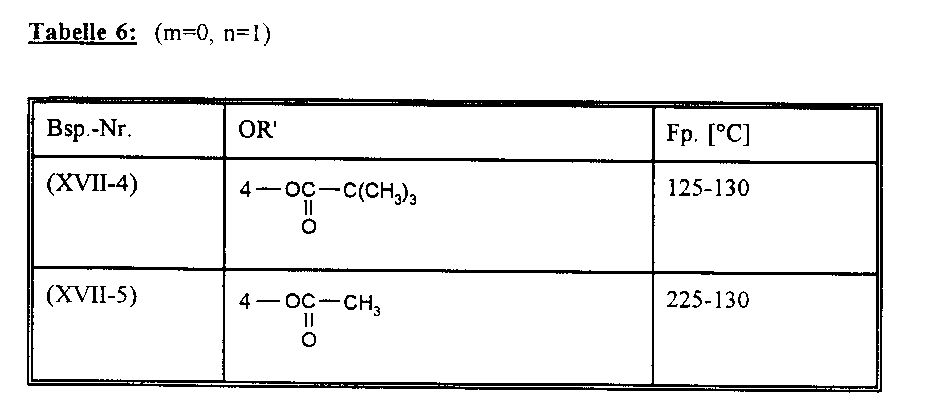

### Beispiel für die Verwendung der Verbindung gemäß Beispiel (I-8)

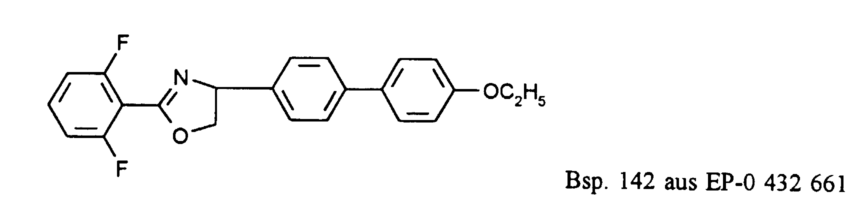

Zu 1,75 g der Verbindung gemäß Beispiel (I-8) in 10 ml Dimethylformamid tropft man zunächst 0,22 ml (0,005 mol) 45 %ige NaOH-Lösung und dann bei 0°C 0,5 g (0,005 mol) Ethylbromid. Man läßt 2 Stunden bei Raumtemperatur rühren, gibt auf 50 ml Eiswasser und saugt die Kristalle ab. Das Filtrat wird mit Essigsäureethylester extrahiert, die Essigsäureethylesterphase getrocknet und im Vakuum eingeengt. Die Kristalle werden vereinigt, mit 20 ml Diisopropylester verrührt und abgesaugt. Ausbeute 1,1 g (58 % der Theorie), Fp. 148 bis 150°C.

In den folgenden Anwendungsbeispielen wurde die aus der EP-A-0 432 661 bekannte Verbindung der Formel (A) 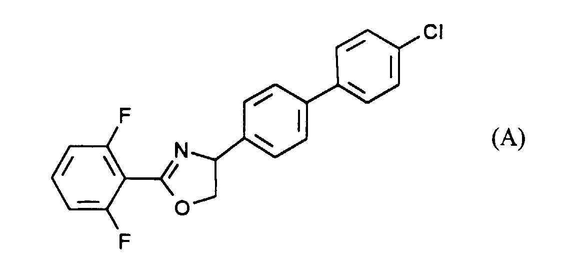 als Vergleichssubstanz eingesetzt.

### Anwendungsbeispiele

### Beispiel A

| Plutella-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel (I-2) bei einer beispielhaften Wirkstoffkonzentration von 0,01 % eine Abtötung von 100 % nach 3 Tagen und die Verbindung gemäß Herstellungsbeispiel I-11 bei einer beispielhaften Wirkstoffkonzentration von 0,1 % eine Abtötung von 100 % nach 7 Tagen.

### Beispiel B

| Spodoptera-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel (I-11) bei einer beispielhaften Wirkstoff-Konzentration von 0,1 % eine Abtötung von 100 % nach 7 Tagen.

### Beispiel C

| Tetranychus-Test (OP-resistent / Spritzbehandlung) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Stadien der gemeinen Spinnmilbe (Tetranychus urticae) befallen sind, werden mit einer Wirkstoffzubereitung der gewünschten Konzentration gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test bewirkte z.B. die Verbindung gemäß Herstellungsbeispiel I-2 bei einer beispielhaften Wirkstoffkonzentration von 0,000032 % eine Abtötung von 95 % nach 14 Tagen, während die bekannte Verbindung (A) eine Abtötung von lediglich 80 % bewirkte.

### Beispiel D

| Nymphenhäutungstest an mehrwirtigen Zecken | |
|---|---|
| Testtiere | Amblyomma variegatum, vollgesogene Nymphenstadien |
| | |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

10 vollgesogene Nymphen werden in die zu testende Wirkstoffzubereitung 1 Minuten getaucht. Die Tiere werden auf mit Filterscheiben bestückte Petrischalen (Ø 9,5 cm) überführt und abgedeckt. Nach 5-6 Wochen Aufbewahrung in einem klimatisierten Raum wird die Häutungsrate bestimmt.

Dabei bedeutet 100 %, daß sich alle Tiere normal gehäutet haben; 0 % bedeutet, daß sich keine Tiere normal gehäutet haben.

In diesem Test bewirke z.B. die Verbindung gemäß Herstellungsbeispiel I-2 bei einer beispielhaften Wirkstoff-Konzentration von 1000 ppm eine Häutungsrate von 0 %.

### Beispiel E

| Blowfly-Larven-Test / Entwicklungshemmende Wirkung | |
|---|---|
| Testtiere | Lucilia cuprina-Larven |
| | |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

Etwa 20 Lucilia cuprina-Larven werden in ein Teströhrchen gebracht, welches ca. 1 cm³ Pferdefleisch und 0,5 ml der zu testenden Wirkstoffzubereitung enthält. Nach 24 und 48 Stunden wird die Wirksamkeit der Wirkstoffzubereitung ermittelt. Die Teströhrchen werden in Becher mit Sand-bedecktem Boden überführt. Nach weiteren 2 Tagen werden die Teströhrchen entfernt und die Puppen ausgezählt.

Die Wirkung der Wirkstoffzubereitung wird nach der Zahl der geschlüpften Fliegen nach 1,5-facher Entwicklungsdauer und unbehandelter Kontrolle beurteilt. Dabei bedeutet 100 %, daß keine Fliegen geschlüpft sind; 0 % bedeutet, daß alle Fliegen normal geschlüpft sind.

In diesem Test hatte z.B. die Verbindung gemäß Herstellungsbeispiel I-2 bei einer beispielhaften Wirkstoffkonzentration von 1000 ppm eine Wirkung von 100 %.

### Beispiel F

| Nephotettix-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen I-7, I-8 und I-11 bei einer beispielhaften Wirkstoffkonzentration von 0,1 % eine Abtötung von mindestens 90 % nach 6 Tagen.

## Patentansprüche

1. Verbindungen der Formel (I) 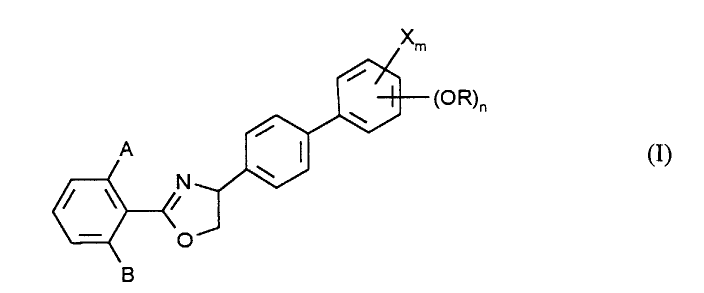 in welcher
A für Wasserstoff, Fluor oder Chlor steht,
B für Fluor oder Chlor steht,
R für R¹ oder R² steht, wobei
R¹ für C₁-C₆-Halogenalkyl oder C₃-C₆-Halogencycloalkyl mit jeweils mindestens einem Fluoratom und zusätzlich mindestens einem Wasserstoff- oder Chloratom oder für C₄-C₆-Halogencycloalkenyl mit mindestens einem Fluoratom steht,
R² für Wasserstoff, C₃-C₁₂-Alkenyl, C₃-C₁₂-Alkinyl, für gegebenenfalls durch C₁-C₄-Alkyl, C₂-C₄-Alkenyl, C₂-C₄-Halogenalkenyl, Phenyl, Halogenphenyl, Styryl oder Halogenstyryl substituiertes C₃-C₆-Cycloalkyl,
für gegebenenfalls durch Halogen, C₁-C₄-Alkyl, C₂-C₄-Alkenyl, C₂-C₄-Halogenalkenyl, Phenyl, Halogenphenyl, Styryl oder Halogenstyryl substituiertes C₃-C₆-Cycloalkyl-C₁-C₄-alkyl,
für gegebenenfalls durch Halogen oder C₁-C₄-Alkyl substituiertes C₄-C₆-Cycloalkenyl-C₁-C₄-alkyl,
für gegebenenfalls durch C₁-C₄-Alkyl substituiertes C₄-C₆-Cycloalkenyl,
für jeweils gegebenenfalls einfach bis vierfach, gleich oder verschieden durch Nitro, Halogen, C₁-C₁₂-Alkyl, C₁-C₁₂-Halogenalkyl, C₁-C₁₂-Alkoxy oder C₁-C₁₂-Halogenalkoxy substituiertes Phenyl-C₁-C₆-alkyl, Naphthyl-C₁-C₃-alkyl oder TetrahydronaphthylC₁-C₃-alkyl,
für gegebenenfalls einfach oder zweifach, gleich oder verschieden durch Nitro, Halogen, C₁-C₁₂-Alkyl, C₁-C₁₂-Halogenalkyl, C₁-C₁₂-Alkoxy oder C₁-C₁₂-Halogenalkoxy substituiertes 5- oder 6-gliedriges Heteroaryl-C₁-C₄-alkyl mit 1 oder 2 gleichen oder verschiedenen Heteroatomen aus der Reihe Stickstoff, Sauerstoff und Schwefel oder für den Rest COR³ steht,
worin
R³ für C₁-C₁₂-Alkyl, C₁-C₁₂-Alkoxy, C₃-C₁₂-Alkenyl, C₃-C₁₂-Alkenyloxy, für jeweils gegebenenfalls durch Halogen, C₁-C₄-Alkyl, C₁-C₄-Halogenalkyl, C₂-C₄-Alkenyl, C₂-C₄-Halogenalkenyl substituiertes C₃-C₁₀-Cycloalkyl, C₃-C₁₀-Cycloalkyloxy oder C₃-C₁₀-Cycloalkyl-C₁-C₆-alkyloxy,
für jeweils gegebenenfalls einfach bis vierfach, gleich oder verschieden durch Halogen, C₁-C₁₂-Alkyl, C₁-C₁₂-Halogenalkyl, C₁-C₁₂-Alkoxy oder C₁-C₁₂-Halogenalkoxy substituiertes Phenyl oder Naphthyl oder für den Rest NR⁴R⁵ steht,
in welchem
R⁴ für Wasserstoff oder C₁-C₁₂-Alkyl steht und
R⁵ für C₁-C₁₂-Alkyl, C₁-C₁₂-Halogenalkyl, für jeweils gegebenenfalls durch Halogen, C₁-C₄-Alkyl, C₁-C₄-Halogenalkyl oder C₂-C₄-Halogenalkenyl substituiertes C₃-C₁₀-Cycloalkyl oder C₃-C₁₀-Cycloalkyl-C₁-C₆-alkyl oder für jeweils gegebenenfalls einfach bis vierfach, gleich oder verschieden durch Halogen, C₁-C₁₂-Alkyl, C₁-C₁₂-Halogenalkyl, C₁-C₁₂-Alkoxy, C₁-C₁₂-Halogenalkoxy substituiertes Phenyl oder Phenyl-C₁-C₆-alkyl steht,
X für Halogen, C₁-C₆-Alkyl oder C₁-C₆-Alkoxy steht,
m für 0, 1 oder 2 steht und
n für 1 oder 2 steht,
ausgenommen 2-(2,6-Difluorphenyl)-4-[4'-(2,2,2-trifluorethoxy)-biphenyl-4]-2-oxazolin und 2-(2-Chlor-6-fluorphenyl)-4-[4'-(2,2,2-trifluorethoxy)-biphenyl-4]-2-oxazolin.

2. Verbindungen der Formel (I) gemäß Anspruch 1, in welcher
A für Wasserstoff, Fluor oder Chlor steht,
B für Fluor oder Chlor steht,
R für R¹ oder R² steht, wobei
R¹ für C₁-C₃-Halogenalkyl oder C₄-C₅-Halogencycloalkyl mit jeweils mindestens einem Fluoratom und zusätzlich mindestens einem Wasserstoff- oder Chloratom oder für C₄-C₆-Halogencycloalkenyl mit mindestens einem Fluoratom steht und
R² für Wasserstoff, C₃-C₁₂-Alkenyl, C₃-C₅-Alkinyl, für gegebenenfalls durch C₁-C₄-Alkyl, C₂-C₄-Alkenyl, C₂-C₃-Halogenalkenyl, Phenyl, Halogenphenyl, Styryl oder Halogenstyryl substituiertes C₃-C₆-Cycloalkyl,
für gegebenenfalls durch Halogen, C₁-C₄-Alkyl, C₂-C₄-Alkenyl, C₂-C₃-Halogenalkenyl, Phenyl, Halogenphenyl, Styryl oder Halogenstyryl substituiertes C₃-C₆-Cycloalkyl-C₁-C₄-alkyl,
für gegebenenfalls durch Halogen substituiertes C₄-C₆-Cycloalkenylmethyl,
für C₄-C₆-Cycloalkenyl,
für jeweils gegebenenfalls einfach oder zweifach, gleich oder verschieden durch Nitro, Halogen, C₁-C₄-Alkyl, C₁-C₄-Halogenalkyl, C₁-C₄-Alkoxy oder C₁-C₄-Halogenalkoxy substituiertes Phenyl-C₁-C₆-alkyl, Naphthylmethyl, Tetrahydronaphthylmethyl, Pyridyl-, Furanyl-, Thiazolyl-, Oxazolyl- oder Isoxazolyl-C₁-C₃-alkyl oder für den Rest COR³ steht,
R³ für C₁-C₆-Alkyl, C₁-C₆-Alkoxy, C₃-C₆-Alkenyl, C₃-C₆-Alkenyloxy, für jeweils gegebenenfalls durch Fluor, Chlor, C₁-C₃-Alkyl, C₁-C₂-Halogenalkyl, C₂-C₃-Halogenalkenyl substituiertes C₃-C₆-Cycloalkyl, C₃-C₆-Cycloalkyloxy oder C₃-C₆-Cycloalkyl-C₁-C₂-alkyloxy, für gegebenenfalls einfach oder zweifach, gleich oder verschieden durch Halogen, C₁-C₄-Alkyl, C₁-C₃-Halogenalkyl, C₁-C₄-Alkoxy oder C₁-C₄-Halogenalkoxy substituiertes Phenyl oder für den Rest NHR⁵ steht,
R⁵ für C₁-C₄-Alkyl oder für jeweils gegebenenfalls einfach oder zweifach, gleich oder verschieden durch Fluor, Chlor, Brom, C₁-C₄-Alkyl, C₁-C₄-Halogenalkyl oder C₁-C₄-Halogenalkoxy substituiertes Phenyl oder Benzyl steht,
X für Fluor, Chlor oder Brom steht,
m für 0 oder 1 steht und
n für 1 steht,
ausgenommen 2-(2,6-Difluorphenyl)-4-[4'-(2,2,2-trifluorethoxy)-biphenyl-4]-2-oxazolin und 2-(2-Chlor-6-fluorphenyl)-4-[4'-(2,2,2-trifluorethoxy)-biphenyl-4]-2-oxazolin.

3. Verbindungen der Formel (I) gemäß Anspruch 1, in welcher
A für Wasserstoff, Fluor oder Chlor steht,
B für Fluor oder Chlor steht,
R für R¹ oder R² steht, wobei
R¹ für eine der Gruppen -CHF₂, -CClF₂,
-CF₂CHFCl, -CF₂CH₂F, -CF₂CHF₂, -CF₂CCl₃, -CF₂CHFCF₃, -CH₂CF₃, -CH₂CF₂CHF₂, -CH₂CF₂CF₃, 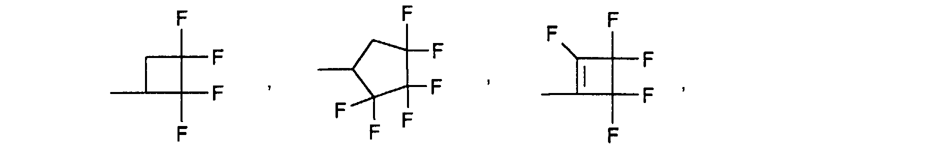 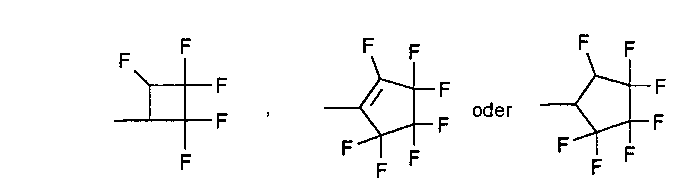 steht und
R² für Wasserstoff, für Propenyl, Butenyl, Pentenyl, Hexenyl, Propinyl, Butinyl, Pentinyl,
für eine der Cycloalkylalkylgruppierungen: 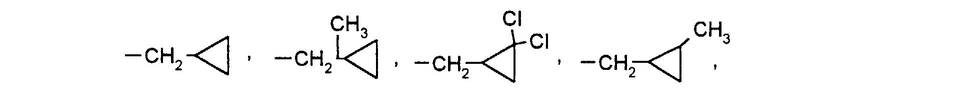 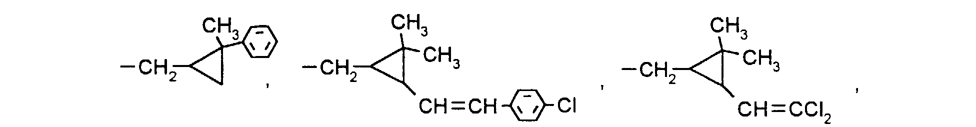 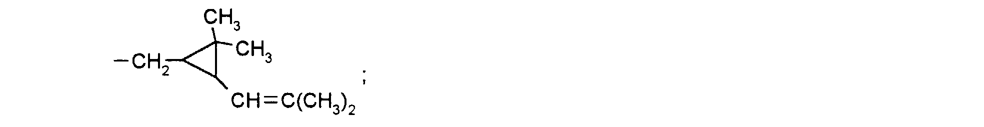 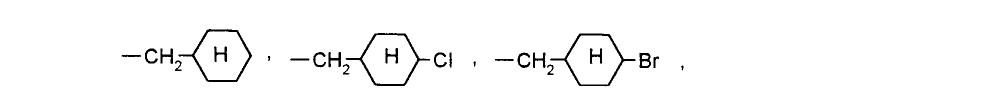 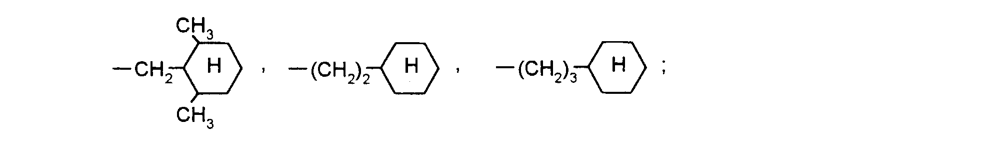 für die Cycloalkenylalkylgruppierung: 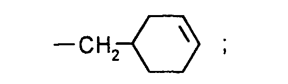 für eine der Phenylalkylgruppierungen: 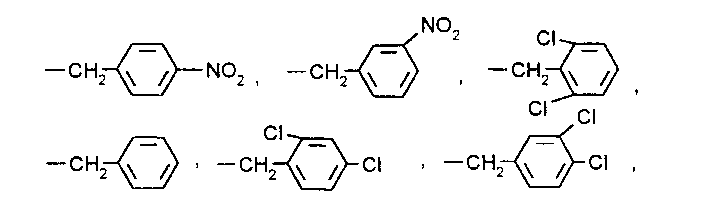 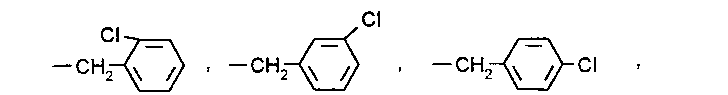 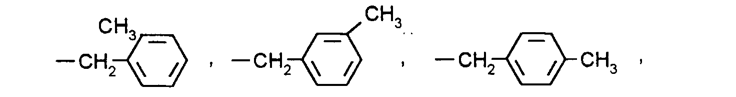 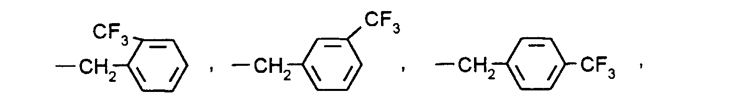 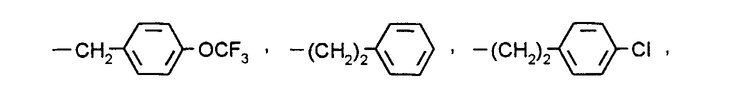 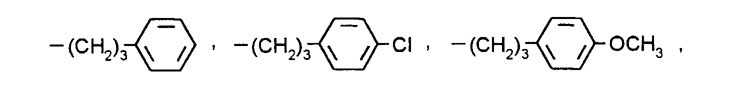 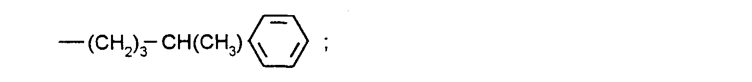 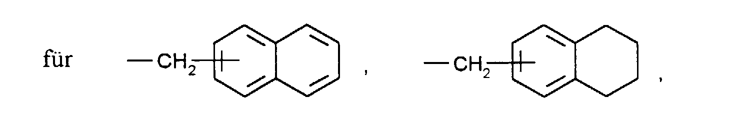 für eine der Heteroarylalkylgruppierungen: 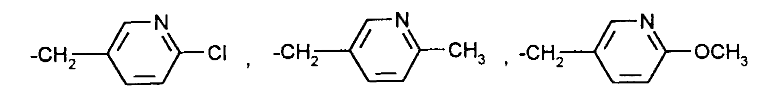 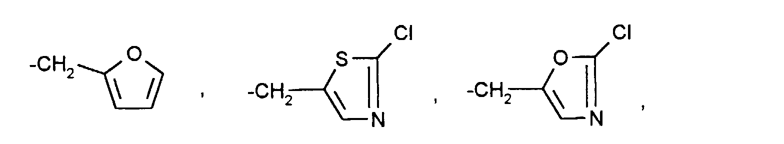 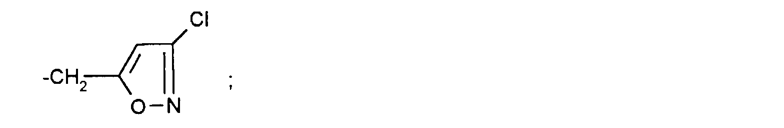 oder für den Rest -COR³ steht,
R³ für Methyl, Ethyl, Propyl;
für Methoxy, Ethoxy, Propoxy, Butoxy;
für Cyclopropyl, Cyclohexyl;
für Cyclohexyloxy;
für Phenyl, 2-Chlorphenyl, 3-Chlorphenyl, 2,6-Difluorphenyl, 2-Trifluormethoxyphenyl, 4-Trifluormethoxyphenyl; 2,4-Dichlorphenyl;
3,4-Dichlorphenyl; oder für den Rest -NHR⁵ steht,
R⁵ für Methyl, Ethyl oder gegebenenfalls einfach durch Chlor substituiertes Phenyl steht,
X für Fluor, Chlor oder Brom steht,
m für 0 oder 1 steht und
n für 1 steht,
ausgenommen 2-(2,6-Difluorphenyl)-4-[4'-(2,2,2-trifluorethoxy)-biphenyl-4]-2-oxazolin und 2-(2-Chlor-6-fluorphenyl)-4-[4'-(2,2,2-trifluorethoxy)-biphenyl-4]-2-oxazolin.

4. Verfahren zur Herstellung der Verbindungen der Formel (I) gemäß Anspruch 1, dadurch gekennzeichnet, daß man
A) in einer ersten Stufe zum Erhalt von Verbindungen der Formel (II) 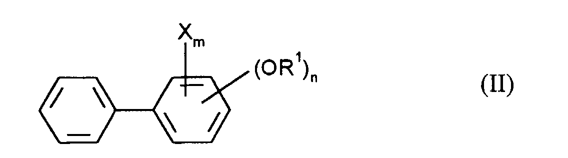 in welcher
R¹, X, m und n die in Anspruch 1 angegebene Bedeutung haben,
α) zum Erhalt von Verbindungen der Formel (IIa) 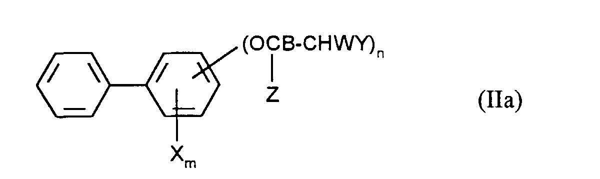 in welcher
W und Y unabhängig voneinander für Fluor, Chlor oder Trifluormethyl stehen,
B für Wasserstoff oder Fluor steht,
Z für Fluor steht oder
W und Z gemeinsam für -(CF₂)₁ stehen,
worin
l für 2, 3 oder 4 steht,
n für 1 oder 2 steht und
m für 0, 1 oder 2 steht,
ein Hydroxybiphenyl der Formel (III) 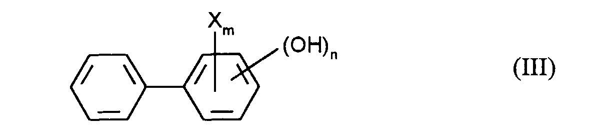 in welcher
X, m und n die oben angegebene Bedeutung haben,
mit einer Verbindung der Formel (IV) 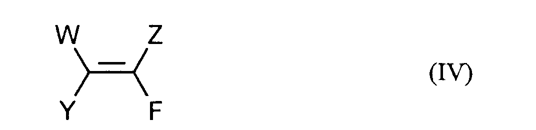 in welcher
W, Y und Z die oben angegebene Bedeutung haben,
gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert,
oder
β) zum Erhalt von Verbindungen der Formel (IIb) 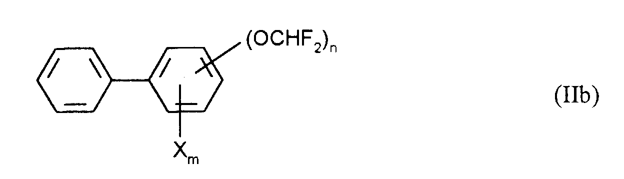 in welcher
X, m und n die oben angegebene Bedeutung haben,
Hydroxybiphenyle der obengenannten Formel (III) mit einem Difluorhalogenmethan der Formel (V)
CHF₂Hal (V)
in welcher
Hal für Chlor oder Brom steht,
gegebenenfalls in Gegenwart einer Base, gegebenenfalls in Gegenwart eines Katalysators und gegebenenfalls in Gegenwart eines Verdünnungsmittels umsetzt,
oder
γ) zum Erhalt von Verbindungen der Formel (IIc) 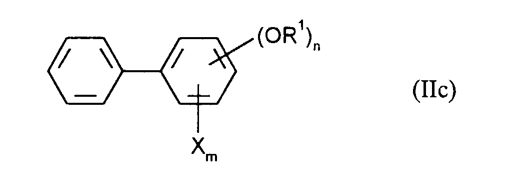 in welcher
X, R¹, m und n die oben angegebene Bedeutung haben,
Aminophenolderivate der Formel (VI) 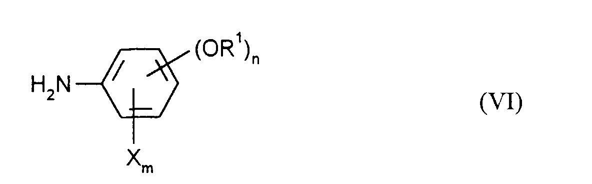 in welcher
X, R¹, m und n die oben angegebene Bedeutung haben,
diazotiert und das entstandene Diazoniumsalz mit Benzol in Gegenwart von Säure und Eisenpulver oder in Gegenwart einer Base und jeweils gegebenenfalls in Gegenwart eines Verdünnungsmittels umsetzt,
oder
δ) zum Erhalt von Verbindungen der Formel (IId) 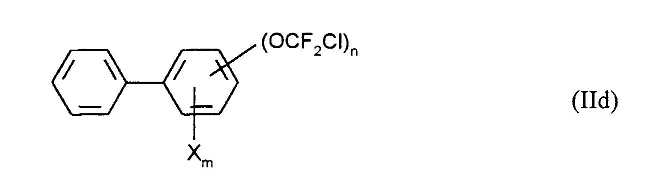 in welcher
X, m und n die oben angegebene Bedeutung haben,
ein Hydroxybiphenyl der obengenannten Formel (III) mit Tetrachlorkohlenstoff in Gegenwart von Fluorwasserstoffsäure, gegebenenfalls in Gegenwart eines Verdünnungmittels umsetzt,
B) in einer zweiten Stufe zum Erhalt von Verbindungen der Formel (VII) 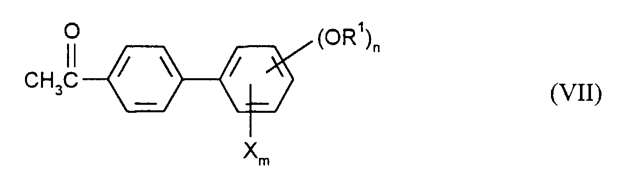 in welcher
X, R¹, m und n die oben angegebene Bedeutung haben,
α) die nach Verfahren A) erhältlichen Verbindungen der Formel (II) in Gegenwart einer Säure oder Lewis-Säure und in Gegenwart eines Verdünnungsmittels mit Acetylchlorid umsetzt,
oder
β) zum Erhalt von Verbindungen der Formel (VIIa) 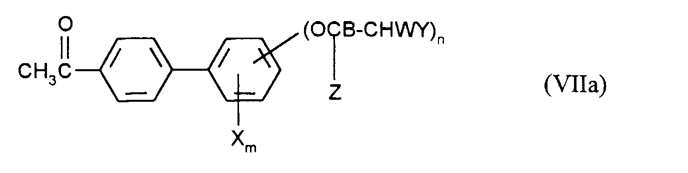 in welcher
B, X, m, n, W, Y und Z die oben angegebene Bedeutung haben,
ein Hydroxybiphenylderivat der Formel (IIIa) 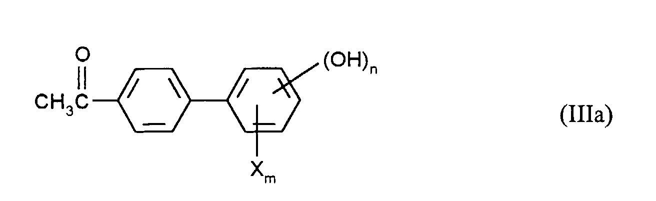 in welcher
X, m und n die oben angegebene Bedeutung haben,
mit einer Verbindung der Formel (IV) 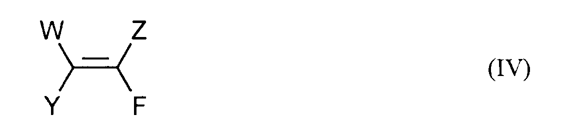 in welcher
W, Y und Z die oben angegebene Bedeutung haben,
gegebenenfalls in Gegenwart einer Base, gegebenenfalls in Gegenwart eines Katalysators und gegebenenfalls in Gegenwart eines Lösungsmittels umsetzt und gegebenenfalls anschließend hydriert,
oder
γ) zum Erhalt von Verbindungen der Formel (VIIb) 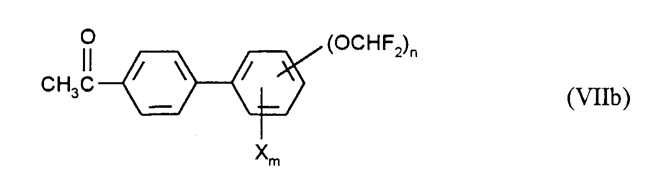 in welcher
X, m und n die oben angegebene Bedeutung haben,
ein Hydroxybiphenylderivat der oben gezeigten Formel (IIIa) mit einem Difluorhalogenmethan der oben gezeigten Formel (V) gegebenenfalls in Gegenwart einer Base, gegebenenfalls in Gegenwart eines Katalysators und gegebenenfalls in Gegenwart eines Verdünnungsmittels umsetzt,
C) in einer dritten Stufe zum Erhalt von Verbindungen der Formel (VIII) 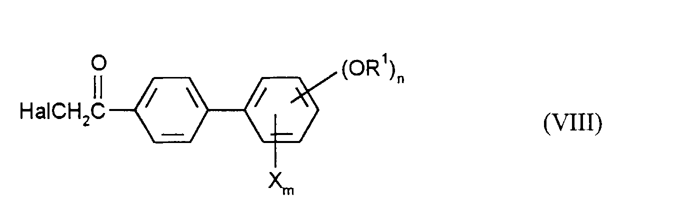 in welcher
X, R¹, m und n die oben angegebene Bedeutung haben und
Hal für Chlor oder Brom steht,
α) die nach Verfahren B) erhältlichen Verbindungen der oben gezeigten Formel (VIII) gegebenenfalls in Gegenwart eines Verdünnungsmittels chloriert oder bromiert,
oder
β) die nach Verfahren A) erhältlichen Verbindungen der oben gezeigten Formel (II) mit Halogenacetylchloriden der Formel (IX)
HalCH₂COCl (IX)
in welcher
Hal für Chlor oder Brom steht,
in Gegenwart einer Säure oder Lewis-Säure und in Gegenwart eines Verdünnungsmittels umsetzt,
D) in einer vierten Stufe zum Erhalt von Verbindungen der Formel (X) 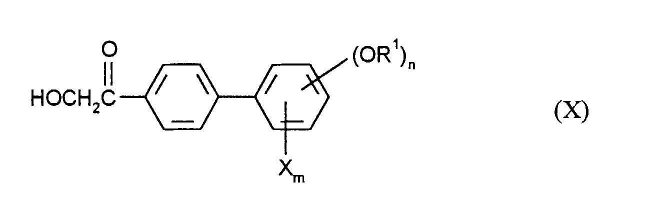 in welcher
X, R¹, m und n die oben angegebene Bedeutung haben,
die nach Verfahren C) erhältlichen Verbindungen der oben gezeigten Formel (VIII) mit einem Salz der Ameisensäure, gegebenenfalls in Gegenwart eines Katalysators umsetzt,
E) in einer fünften Stufe zum Erhalt von Verbindungen der Formel (XI) 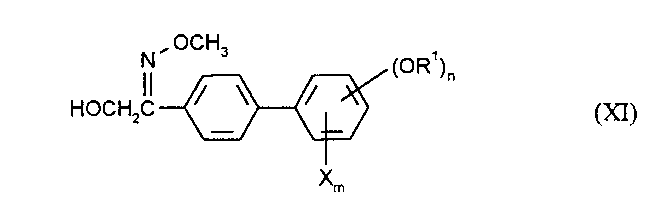 in welcher
X, R¹, m und n die oben angegebene Bedeutung haben,
die nach Verfahren D) erhältlichen Verbindungen der oben gezeigten Formel (X) mit der Verbindung der Formel (XII)
H₂N-OCH₃ (XII)
gegebenenfalls in Gegenwart eines Verdünnungsmittels umsetzt,
F) in einer sechsten Stufe zum Erhalt von Verbindungen der Formel (XIII) 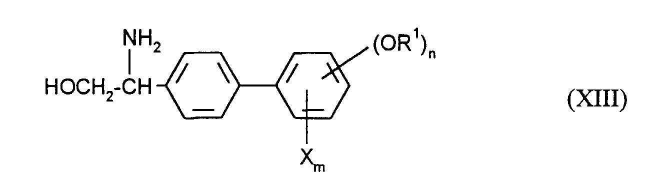 in welcher
R¹, X, m und n die oben angegebene Bedeutung hat,
die nach Verfahren E) erhältlichen Verbindungen der oben gezeigten Formel (XI) mit einem Reduktionsmittel in Gegenwart einer Säure und gegebenenfalls in Gegenwart eines Verdünnungsmittels reduziert,
G) in einer siebten Stufe zum Erhalt von Verbindungen der Formel (XIV) 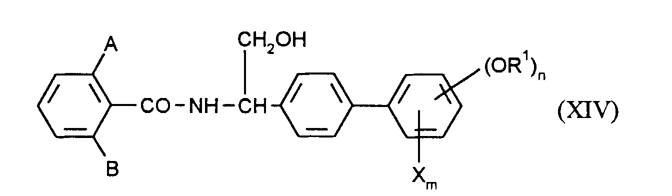 in welcher
A, B, R¹, X, m und n die in Anspruch 1 angegebene Bedeutung haben und
entweder
α) die nach Verfahren F) erhältlichen Verbindungen der oben gezeigten Formel (XIII) mit einem 2-A,6-B-Benzoylchlorid, gegebenenfalls in Gegenwart einer Base und gegebenenfalls in Gegenwart eines Verdünnungsmittels umsetzt
oder
β) zunächst die nach Verfahren A) erhältlichen Verbindungen der oben gezeigten Formel (II) mit einer Verbindung der Formel (XV) 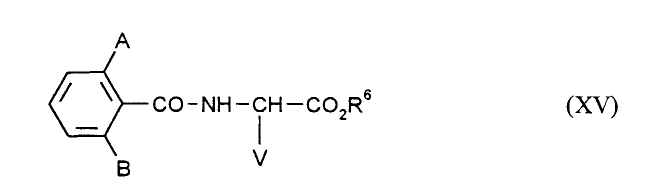 in welcher
A und B die oben angegebene Bedeutung haben,
V für Chlor, Hydroxy oder C₁-C₄-Alkoxy steht und
R⁶ für Wasserstoff oder Alkyl steht,
in Gegenwart eines sauren Katalysators und gegebenenfalls in Gegenwart eines Verdünnungsmittels umsetzt und
die so erhaltenen Verbindungen der Formel (XVI) 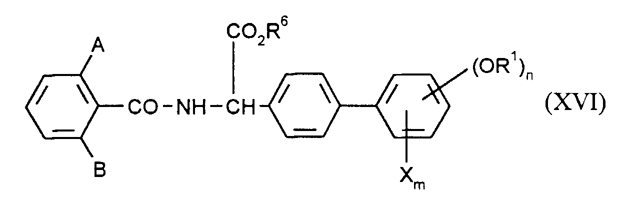 in welcher
A, B, R¹ und R⁶ die oben angegebene Bedeutung haben,
in Gegenwart eines Reduktionsmittels und in Gegenwart eines Verdünnungsmittels reduziert,
H) in einer achten Stufe zum Erhalt von Verbindungen der Formel (XVII) 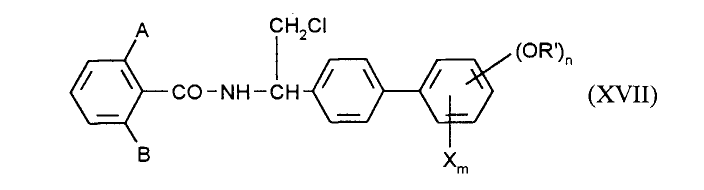 in welcher
A, B, X, m und n die oben angegebene Bedeutung haben,
R' für R¹ oder COR³ steht, worin
R¹ und R³ die in Anspruch 1 genannte Bedeutung haben
entweder
α) die nach Verfahren G) erhältlichen Verbindungen der oben gezeigten Formel (XIV) mit einem Chlorierungsmittel, gegebenenfalls in Gegenwart eines Verdünnungsmittels umsetzt
oder
β) die nach Verfahren A) erhältlichen Verbindungen der oben gezeigten Formel (II) mit einer Verbindung der Formel (IIe) 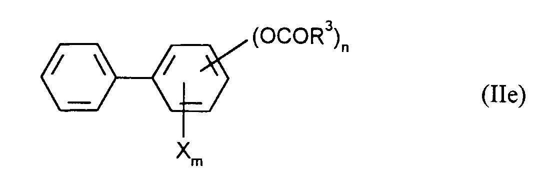 in welcher
R³, X, n und m die oben angegebene Bedeutung haben,
mit einer Verbindung der Formel (XVIII) 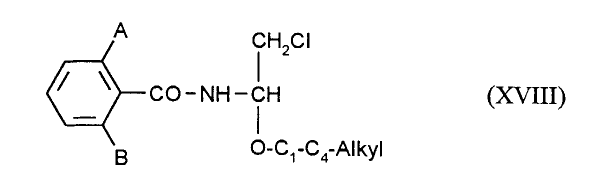 in welcher
A und B die oben angegebene Bedeutung haben,
in Gegenwart eines sauren Katalysators und gegebenenfalls in Gegenwart eines Verdünnungsmittels umsetzt,
und
I) in einer neunten Stufe die nach Verfahren H) erhältlichen Verbindungen der oben gezeigten Formel (XVII) in Gegenwart einer Base, gegebenenfalls in Gegenwart eines Katalysators und gegebenenfalls in Gegenwart eines Verdünnungsmittels cyclisiert,
und gegebenenfalls
J) die dabei für R = COR³ erhaltenen Verbindungen der Formel (Ia) 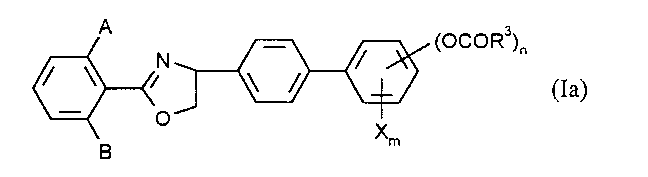 in welcher
A, B, R³, X, n und m die oben angegebene Bedeutung haben
zu Verbindungen der Formel (Ib) 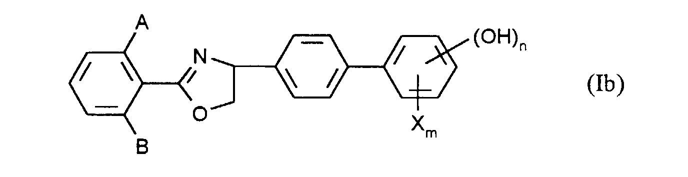 in welcher
A, B X, n und m die oben angegebene Bedeutung haben
verseift und diese anschließend gegebenenfalls
K
α) mit einer Verbindung der Formel (XIX)
HalCOR³ (XIX)
in welcher
R³ die obengenannte Bedeutung hat und
Hal für Halogen steht, gegebenenfalls in Gegenwart eines Verdünnungsmittels und gegebenenfalls in Gegenwart einer Base umsetzt, oder
β) mit einer Verbindung der Formel (IV) 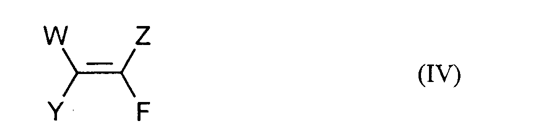 in welcher
W, Y und Z die oben angegebene Bedeutung haben,
gegebenenfalls in Gegenwart einer Base, gegebenenfalls in Gegenwart eines Katalysators und gegebenenfalls in Gegenwart eines Verdünnungsmittels umsetzt und gegebenenfalls anschließend hydriert,
oder
γ) mit einem Difluorhalogenmethan der Formel (V)
CHF₂Hal (V)
in welcher
Hal für Chlor oder Brom steht,
gegebenenfalls in Gegenwart einer Base, gegebenenfalls in Gegenwart eines Katalysators und gegebenenfalls in Gegenwart eines Verdünnungsmittels umsetzt,
oder
δ) mit Tetrachlorkohlenstoff in Gegenwart von Fluorwasserstoffsäure, gegebenenfalls in Gegenwart eines Verdünnungsmittels umsetzt, oder
ε) mit einer Verbindung der Formel (XX)
M-R² (XX)
in welcher
R² die obengenannte Bedeutung hat und
M für eine Abgangsgruppe steht, gegebenenfalls in Gegenwart eines Verdünnungsmittels und gegebenenfalls in Gegenwart einer Base umsetzt.

5. Verbindungen der Formel (II) 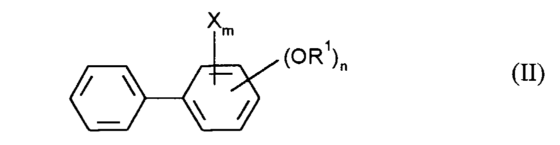 in welcher
R¹, X, m und n die in Anspruch 1 angegebene Bedeutung haben,
ausgenommen die Verbindungen 4-(2,2,2-Trifluorethoxy)biphenyl, 4-(1,1-Difluorethoxy)biphenyl, 4-(1,1-Difluor-n-propoxy)biphenyl und 4-(2-Fluorethoxy)biphenyl.

6. Verbindungen der Formel (VII) 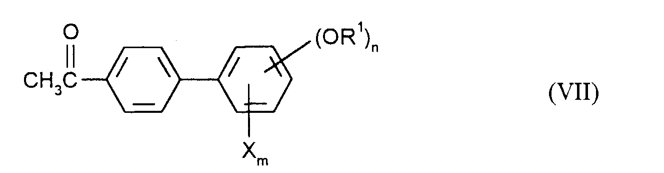 in welcher
X, R¹, m und n die in Anspruch 1 angegebene Bedeutung haben.

7. Verbindungen der Formel (VIII) 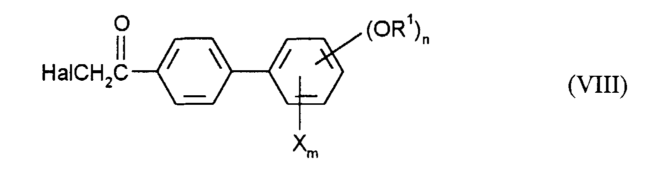 in welcher
X, R¹, m und n die in Anspruch langegebene Bedeutung haben und
Hal für Chlor oder Brom steht.

8. Verbindungen der Formel (X) 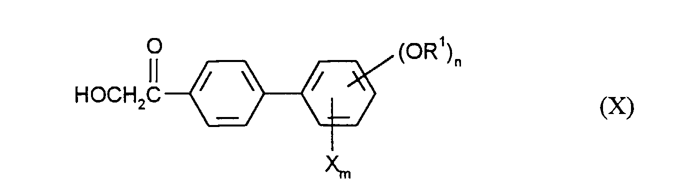 in welcher
X, R¹, m und n die in Anspruch 1 angegebene Bedeutung haben.

9. Verbindungen der Formel (XI) 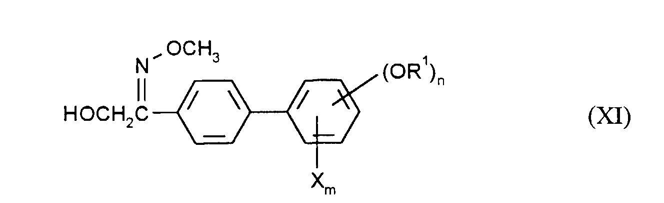 in welcher
X, R¹, m und n die in Anspruch 1 angegebene Bedeutung haben.

10. Verbindungen der Formel (XIII) 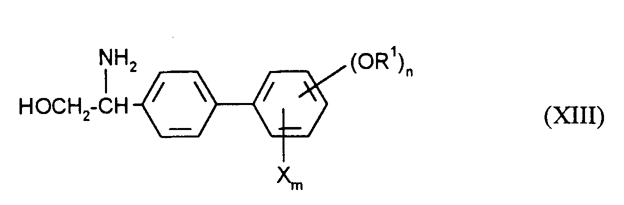 in welcher
R¹, X, m und n die in Anspruch 1 angegebene Bedeutung haben.

11. Verbindungen der Formel (XIV) 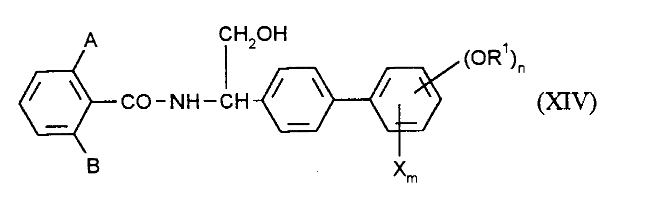 in welcher
A, B, R¹, X, m und n die in Anspruch 1 angegebene Bedeutung haben.

12. Verbindungen der Formel (XVI) 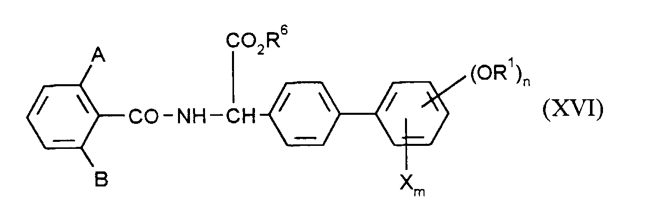 in welcher
A, B, R¹, X, m und n die in Anspruch 1 angegebene Bedeutung haben und
R⁶ für Alkyl steht.

13. Verbindungen der Formel (XVII) 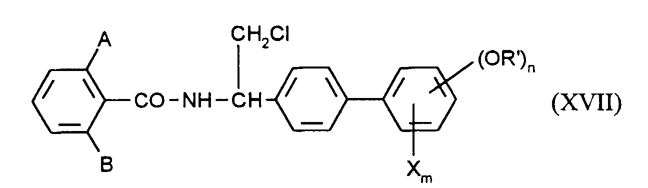 in welcher
A, B, X, m und n die in Anspruch 1 angegebene Bedeutung haben,
R' für R¹ oder COR³ steht, worin
R¹ und R³ die in Anspruch 1 genannte Bedeutung haben.

14. Schädlingsbekämpfungsmittel, gekennzeichnet durch einen Gehalt an mindestens einer Verbindung der Formel (I) gemäß Anspruch 1.

15. Verwendung von Verbindungen der Formel (I) gemäß Anspruch 1 zur Bekämpfung von Schädlingen.

16. Verfahren zur Bekämpfung von Schädlingen, dadurch gekennzeichnet, daß man Verbindungen der Formel (I) gemäß Anspruch 1 auf Schädlinge und/oder ihren Lebensraum einwirken läßt.

17. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

18. Verwendung von Verbindungen der Formel (I) gemäß Anspruch 1 zur Herstellung von Schädlingsbekämpfungsmitteln.

## Claims

1. Compounds of the formula (I) 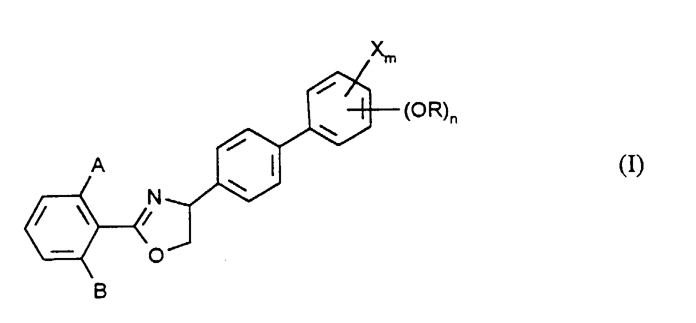 in which
A represents hydrogen, fluorine or chlorine,
B represents fluorine or chlorine,
R represents R¹ or R², where
R¹ represents C₁-C₆-halogenoalkyl or C₃-C₆-halogenocycloalkyl, each of which has at least one fluorine atom and additionally at least one hydrogen or chlorine atom, or represents C₄-C₆-halogenocycloalkenyl having at least one fluorine atom,
R² represents hydrogen, C₃-C₁₂-alkenyl, C₃-C₁₂-alkinyl, or represents C₃-C₆-cycloalkyl which is optionally substituted by C₁-C₄-alkyl, C₂-C₄-alkenyl, C₂-C₄-halogenoalkenyl, phenyl, halogenophenyl, styryl or halogenostyryl,
or represents C₃-C₆-cycloalkyl-C₁-C₄-alkyl which is optionally substituted by halogen, C₁-C₄-alkyl, C₂-C₄-alkenyl, C₂-C₄-halogenoalkenyl, phenyl, halogenophenyl, styryl or halogenostyryl,
or represents C₄-C₆-cycloalkenyl-C₁-C₄-alkyl which is optionally substituted by halogen or C₁-C₄-alkyl,
or represents C₄-C₆-cycloalkenyl which is optionally substituted by C₁-C₄-alkyl,
or represents phenyl-C₁-C₆-alkyl, naphthyl-C₁-C₃-alkyl or tetrahydronaphthyl-C₁-C₃-alkyl, each of which is optionally monosubstituted to tetrasubstituted by identical or different substituents from the series consisting of nitro, halogen, C₁-C₁₂-alkyl, C₁-C₁₂-halogenoalkyl, C₁-C₁₂-alkoxy or C₁-C₁₂-halogenoalkoxy,
or represents five- or six-membered heteroaryl-C₁-C₄-alkyl which has 1 or 2 identical or different hetero atoms from the series consisting of nitrogen, oxygen and sulphur and is optionally monosubstituted or disubstituted by identical or different substituents from the series consisting of nitro, halogen, C₁-C₁₂-alkyl, C₁-C₁₂-halogenoalkyl, C₁-C₁₂-alkoxy or C₁-C₁₂-halogenoalkoxy,
or represents the radical COR³
in which
R³ is C₁-C₁₂-alkyl, C₁-C₁₂-alkoxy, C₃-C₁₂-alkenyl, C₃-C₁₂-alkenyloxy or represents C₃-C₁₀-cycloalkyl, C₃-C₁₀-cycloalkyloxy or C₃-C₁₀-cycloalkyl-C₁-C₆-alkyloxy, each of which is optionally substituted by halogen, C₁-C₄-alkyl, C₁-C₄-halogenoalkyl, C₂-C₄-alkenyl or C₂-C₄-halogenoalkenyl,
or represents phenyl or naphthyl, each of which is optionally monosubstituted or tetrasubstituted by identical or different substituents from the series consisting of halogen, C₁-C₁₂-alkyl, C₁-C₁₂-halogenoalkyl, C₁-C₁₂-alkoxy or C₁-C₁₂-halogenoalkoxy, or represents the radical NR⁴R⁵,
in which
R⁴ represents hydrogen or C₁-C₁₂-alkyl and
R⁵ represents C₁-C₁₂-alkyl, C₁-C₁₂-halogenoalkyl or represents C₃-C₁₀-cycloalkyl or C₃-C₁₀-cycloalkyl-C₁-C₆-alkyl, each of which is optionally substituted by halogen, C₁-C₄-alkyl, C₁-C₄-halogenoalkyl or C₂-C₄-halogenoalkenyl, or represents phenyl or phenyl-C₁-C₆-alkyl, each of which is optionally monosubstituted to tetrasubstituted by identical or different substituents from the series consisting of halogen, C₁-C₁₂-alkyl, C₁-C₁₂-halogenoalkyl, C₁-C₁₂-alkoxy or C₁-C₁₂-halogenoalkoxy,
X represents halogen, C₁-C₆-alkyl or C₁-C₆-alkoxy,
m represents 0, 1 or 2 and
n represents 1 or 2,
with the exception of 2-(2,6-difluorophenyl)-4-[4'-(2,2,2-trifluoroethoxy)-biphenyl-4]-2-oxazoline and 2-(2-chloro-6-fluorophenyl)-4-[4'-(2,2,2-trifluoro-ethoxy)-biphenyl-4]-2-oxazoline.

2. Compounds of the formula (I) according to Claim 1, in which
A represents hydrogen, fluorine or chlorine,
B represents fluorine or chlorine,
R represents R¹ or R², where
R¹ represents C₁-C₃-halogenoalkyl or C₄-C₅-halogenocycloalkyl having in each case at least one fluorine atom and additionally at least one hydrogen or chlorine atom, or represents C₄-C₆-halogenocycloalkenyl having at least one fluorine atom, and
R² represents hydrogen, C₃-C₁₂-alkenyl, C₃-C₅-alkinyl or represents C₃-C₆-cycloalkyl which is optionally substituted by C₁-C₄-alkyl, C₂-C₄-alkenyl, C₂-C₃-halogenoalkenyl, phenyl, halogenophenyl, styryl or halogenostyryl,
or represents C₃-C₆-cycloalkyl-C₁-C₄-alkyl which is optionally substituted by halogen, C₁-C₄-alkyl, C₂-C₄-alkenyl, C₂-C₃-halogenoalkyl, phenyl, halogenophenyl, styryl or halogenostyryl,
or represents C₄-C₆-cycloalkenylmethyl which is optionally substituted by halogen,
or represents C₄-C₆-cycloalkenyl,
or represents phenyl-C₁-C₆-alkyl, naphthylmethyl, tetrahydronaphthylmethyl, pyridyl-, furanyl-, thiazolyl-, oxazolyl- or isoxazolyl-C₁-C₃-alkyl, each of which is optionally monosubstituted or disubstituted by identical or different substituents from the series consisting of nitro, halogen, C₁-C₄-alkyl, C₁-C₄-halogenoalkyl, C₁-C₄-alkoxy or C₁-C₄-halogenoalkoxy, or represents the radicals COR³,
R³ represents C₁-C₆-alkyl, C₁-C₆-alkoxy, C₃-C₆-alkenyl, C₃-C₆-alkenyloxy, or represents C₃-C₆-cycloalkyl, C₃-C₆-cycloalkyloxy or C₃-C₆-cycloalkyl-C₁-C₂-alkyloxy, each of which is optionally substituted by fluorine, chlorine, C₁-C₃-alkyl, C₁-C₂-halogenoalkyl or C₂-C₃-halogenoalkenyl,
or represents phenyl which is optionally monosubstituted or disubstituted by identical or different substituents from the series consisting of halogen, C₁-C₄-alkyl, C₁-C₃-halogenoalkyl, C₁-C₄-alkoxy or C₁-C₄-halogenoalkoxy, or represents the radical NHR⁵,
R⁵ represents C₁-C₄-alkyl, or represents phenyl or benzyl, each of which is optionally monosubstituted or disubstituted by identical or different substituents from the series consisting of fluorine, chlorine, bromine, C₁-C₄-alkyl, C₁-C₄-halogenoalkyl or C₁-C₄-halogenoalkoxy,
X represents fluorine, chlorine or bromine,
m represents 0 or 1 and
n represents 1,
with the exception of 2-(2,6-difluorophenyl)-4-[4'-(2,2,2-trifluoroethoxy)-biphenyl-4]-2-oxazoline and 2-(2-chloro-6-fluorophenyl)-4-[4'-(2,2,2-trifluoroethoxy)-biphenyl-4]-2-oxazoline.

3. Compounds of the formula (I) according to Claim 1 in which
A represents hydrogen, fluorine or chlorine,
B represents fluorine or chlorine,
R represents R¹ or R² where
R¹ represents one of the groups -CHF₂, -CClF₂,
-CF₂CHFCl, -CF₂CH₂F, -CF₂CHF₂, -CF₂CCl₃, -CF₂CHFCF₃, -CH₂CF₃, -CH₂CF₂CHF₂, -CH₂CF₂CF₃, 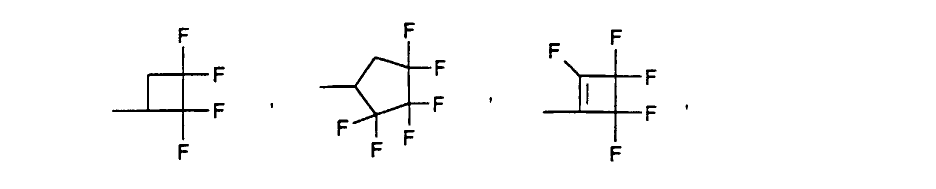 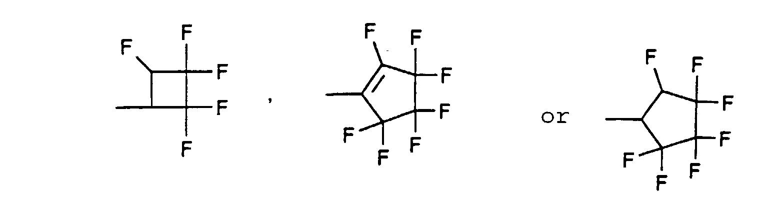 and
R² represents hydrogen, or represents propenyl, butenyl, pentenyl, hexenyl, propinyl, butinyl, pentinyl,
or represents one of the cycloalkylalkyl groups: 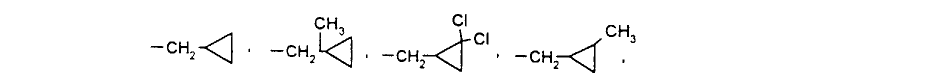 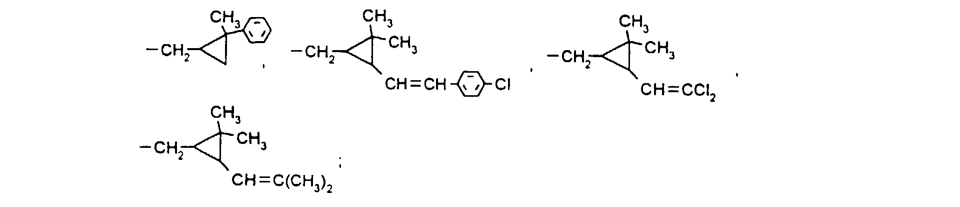 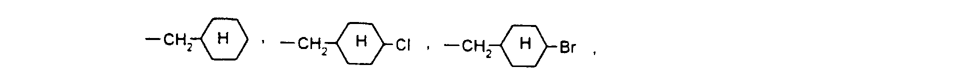 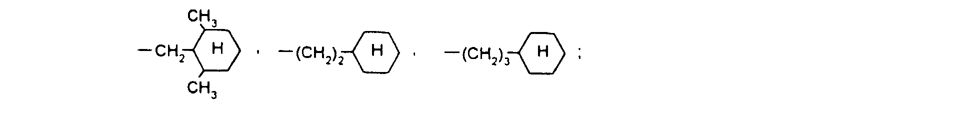 or represents the cycloalkenylalkyl group: 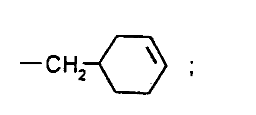 or represents one of the phenylalkyl groups: 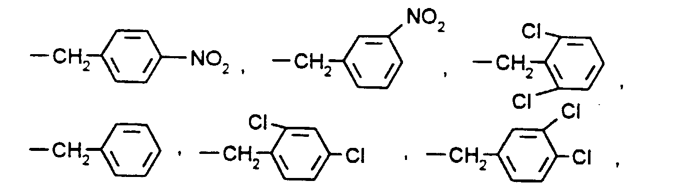 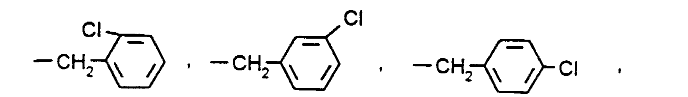 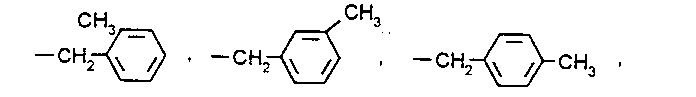 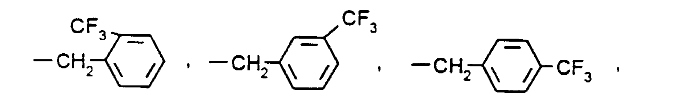 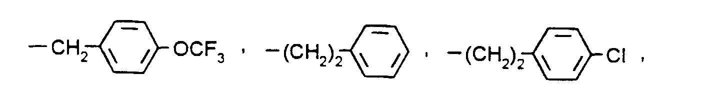 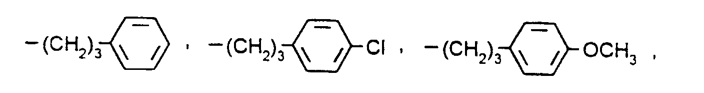 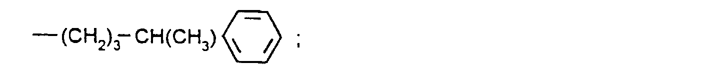 or represents 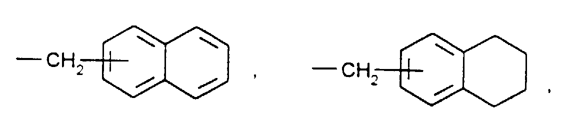 or represents one of the heteroarylalkyl groups: 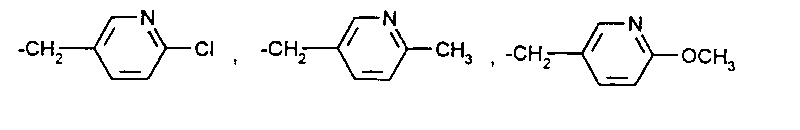 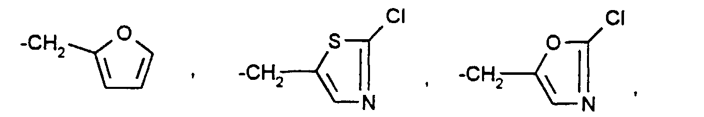 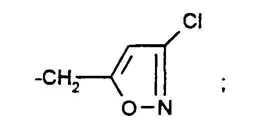 or represents the radical -COR³,
R³ represents methyl, ethyl, propyl; or represents methoxy, ethoxy, propoxy, butoxy; or represents cyclopropyl, cyclohexyl;
or represents cyclohexyloxy;
or represents phenyl, 2-chlorophenyl, 3-chlorophenyl, 2,6-difluorophenyl, 2-trifluoromethoxyphenyl, 4-trifluoromethoxyphenyl; 2,4-dichlorophenyl; 3,4-dichlorophenyl;
or represents the radical -NHR⁵,
R⁵ represents methyl, ethyl or represents phenyl which is optionally monosubstituted by chlorine,
X represents fluorine, chlorine or bromine,
m represents 0 or 1 and
n represents 1,
with the exception of 2-(2,6-difluorophenyl)-4-[4'-(2,2,2-trifluoroethoxy)-biphenyl-4]-2-oxazoline and 2-(2-chloro-6-fluorophenyl)-4-[4'-(2,2,2-trifluoroethoxy)-biphenyl-4]-2-oxazoline.

4. Process for the preparation of the compounds of the formula (I) according to Claim 1, characterized in that,
A) in a first step, to obtain compounds of the formula (II) 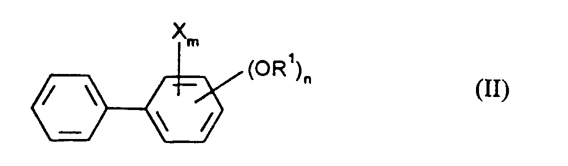 in which
R¹, X, m and n have the meaning given in Claim 1,
α) to obtain compounds of the formula (IIa) 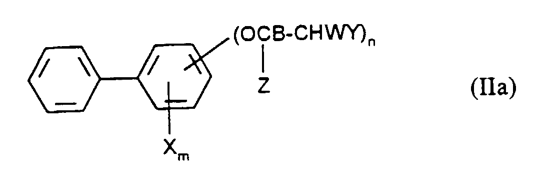 in which
W and Y independently of one another represent fluorine, chlorine or trifluoromethyl,
B represents hydrogen or fluorine,
Z represents fluorine or
W and Z together represent -(CF₂)₁
in which
l represents 2, 3 or 4,
n represents 1 or 2 and
m represents 0, 1 or 2,
a hydroxybiphenyl of the formula (III) 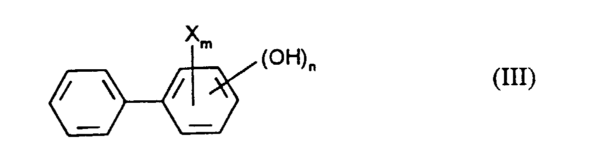 in which
X, m and n have the abovementioned meaning
is reacted with a compound of the formula (IV) 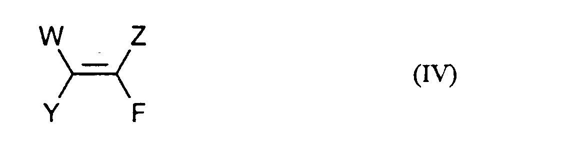 in which
W, Y and Z have the abovementioned meaning,
if appropriate in the presence of a base,
if appropriate in the presence of a catalyst and if appropriate in the presence of a diluent, and, if appropriate, the product is subsequently hydrogenated,
or,
β) to obtain compounds of the formula (IIb) 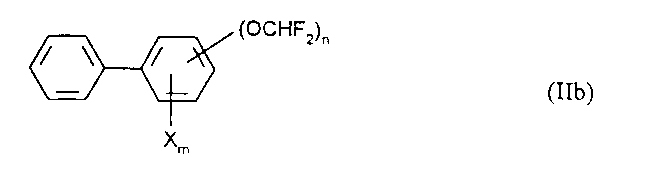 in which
X, m and n have the abovementioned meaning,
hydroxybiphenyls of the abovementioned formula (III) are reacted with a difluorohalogenomethane of the formula (V)
CHF₂Hal (V)
in which
Hal represents chlorine or bromine,
if appropriate, in the presence of a base, if appropriate in the presence of a catalyst and if appropriate in the presence of a diluent,
or,
γ) to obtain compounds of the formula (IIc) 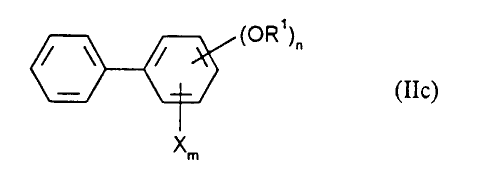 in which
X, R¹, m and n have the abovementioned meaning,
aminophenol derivatives of the formula (VI) 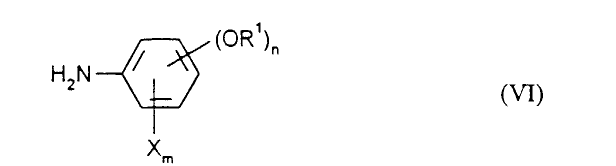 in which
X, R¹, m and n have the abovementioned meaning
are diazotized and the resulting diazonium salt is reacted with benzene in the presence of acid and iron powder or in the presence of a base and in each case, if appropriate, in the presence of a diluent,
or,
δ) to obtain compounds of the formula (IId) 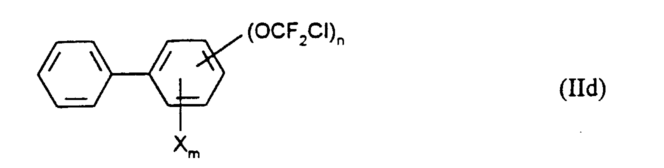 in which
X, m and n have the abovementioned meaning,
a hydroxybiphenyl of the abovementioned formula (III) is reacted with carbon tetrachloride in the presence of hydrofluoric acid, if appropriate in the presence of a diluent,
B) in a second step, to obtain compounds of the formula (VII) 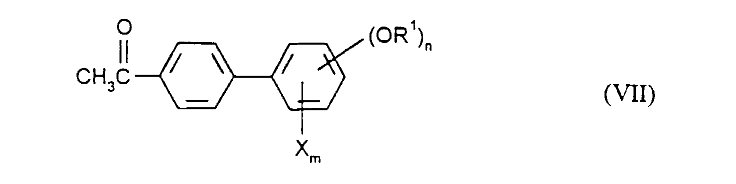 in which
X, R¹, m and n have the abovementioned meaning,
α) the compounds of the formula (II) which can be obtained by the process A) are reacted with acetyl chloride in the presence of an acid or Lewis acid and in the presence of a diluent,
or,
β) to obtain compounds of the formula (VIIa) 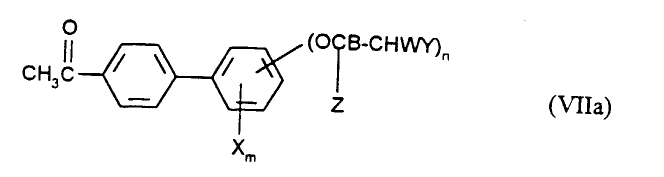 in which
B, X, m, n, W, Y and Z have the abovementioned meaning,
a hydroxybiphenyl derivative of the formula (IIIa) 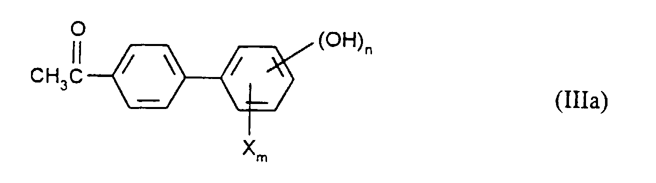 in which
X, m and n have the abovementioned meaning,
is reacted with a compound of the formula (IV) 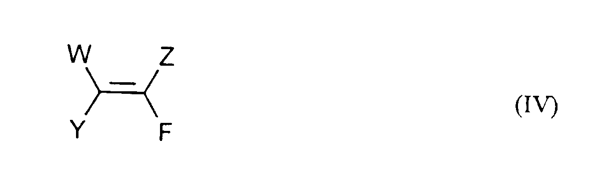 in which
W, Y and Z have the abovementioned meaning,
if appropriate in the presence of a base, if appropriate in the presence of a catalyst and if appropriate in the presence of a solvent, and, if appropriate, the product is subsequently hydrogenated,
or,
γ) to obtain compounds of the formula (VIIb) 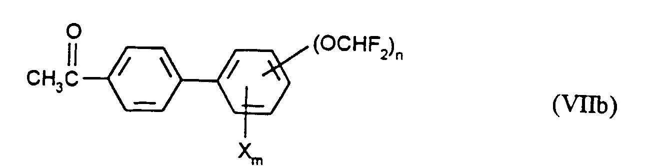 in which
X, m and n have the abovementioned meaning,
a hydroxybiphenyl derivative of the above-shown formula (IIIa) is reacted with a difluorohalogenomethane of the above-shown formula (V), if appropriate in the presence of a base, if appropriate in the presence of a catalyst and if appropriate in the presence of a diluent,
C) in a third step, to obtain compounds of the formula (VIII) 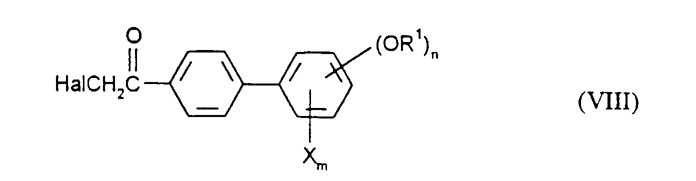 in which
X, R¹, m and n have the abovementioned meaning and
Hal represents chlorine or bromine,
α) the compounds of the above-shown formula (VII), which can be obtained by process B), are chlorinated or brominated, if appropriate in the presence of a diluent,
or
β) the compounds of the above-shown formula (II), which can be obtained by process A), are reacted with halogenoacetyl chlorides of the formula (IX)
HalCH₂COCl (IX)
in which
Hal represents chlorine or bromine
in the presence of an acid or Lewis acid and in the presence of a diluent,
D) in a fourth step, to obtain compounds of the formula (X) 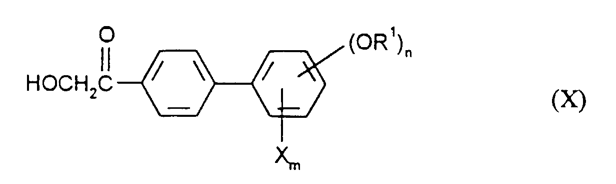 in which
X, R¹, m and n have the abovementioned meaning,
the compounds of the above-shown formula (VIII), which can be obtained by process C), are reacted with a salt of formic acid, if appropriate in the presence of a catalyst,
E) in a fifth step, to obtain compounds of the formula (XI) 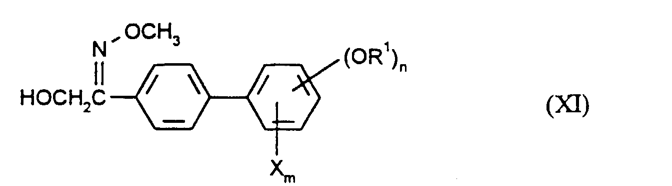 in which
X, R¹, m and n have the abovementioned meaning,
the compounds of the above-shown formula (X), which can be obtained by process D), are reacted with the compound of the formula (XII)
H₂N-OCH₃ (XII),
if appropriate in the presence of a diluent,
F) in a sixth step, to obtain compounds of the formula (XIII) 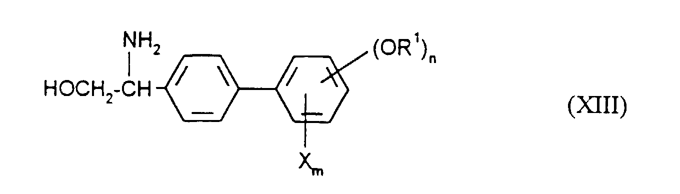 in which
R¹, X, m and n have the abovementioned meaning,
the compounds of the above-shown formula (XI), which can be obtained by process E), are reduced with a reducing agent in the presence of an acid, and, if appropriate, in the presence of a diluent,
G) in a seventh step, to obtain compounds of the formula (XIV) 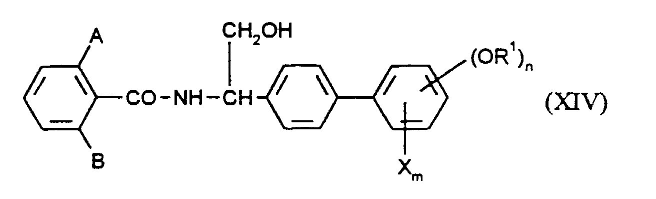 in which
A, B, R¹, X, m and n have the meaning given in Claim 1,
either
α) the compounds of the above-shown formula (XIII), which can be obtained by process F), are reacted with a 2-A,6-B-benzoyl chloride, if appropriate in the presence of a base and if appropriate in the presence of a diluent,
or
β) the compounds of the above-shown formula (II), which can be obtained by process A), are first reacted with a compound of the formula (XV) 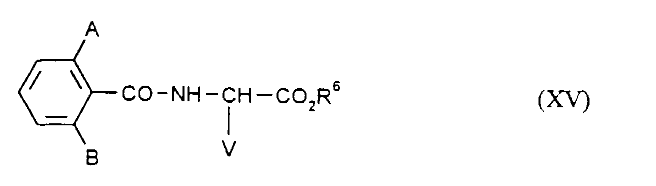 in which
A and B have the abovementioned meaning,
V represents chlorine, hydroxyl or C₁-C₄-alkoxy and
R⁶ represents hydrogen or alkyl
in the presence of an acidic catalyst and, if appropriate, in the presence of a diluent, and
the resulting compounds of the formula (XVI) 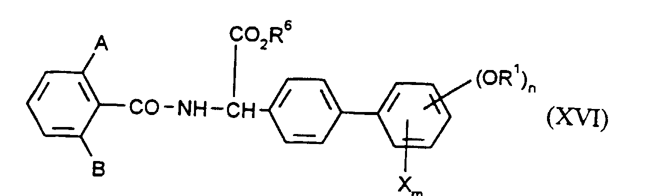 in which
A, B, R¹ and R⁶ have the abovementioned meaning,
are reduced in the presence of a reducing agent and in the presence of a diluent,
H) in an eighth step, to obtain compounds of the formula (XVII) 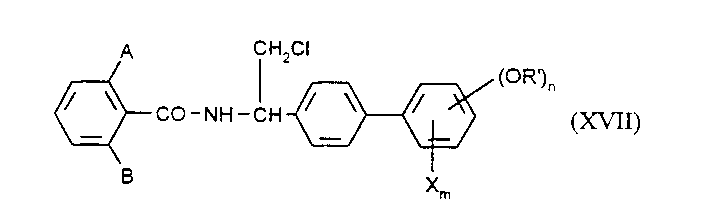 in which
A, B, X, m and n have the abovementioned meaning and
R' represents R¹ or COR³, where
R¹ and R³ have the meaning given in Claim 1,
either
α) the compounds of the above-shown formula (XIV), which can be obtained by process G), are reacted with a chlorinating agent, if appropriate in the presence of a diluent,
or
β) the compounds of the above-shown formula (II), which can be obtained by process A), are reacted with a compound of the formula (IIe) 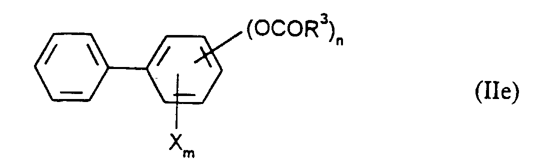 in which
R³, X, n and m have the abovementioned meaning,
with a compound of the formula (XVIII) 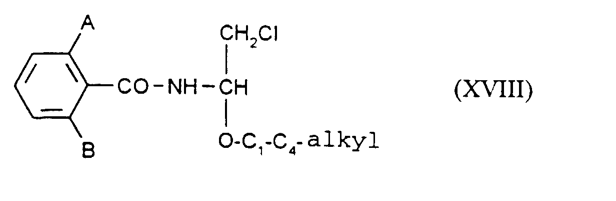 in which
A and B have the abovementioned meaning
in the presence of an acidic catalyst and, if appropriate, in the presence of a diluent, and
I) in a ninth step, the compounds of the above-shown formula (XVII) which can be obtained by process H), are cyclized in the presence of a base, if appropriate in the presence of a catalyst, and if appropriate in the presence of a diluent,
and, if appropriate,
J) the compounds of the formula (Ia) 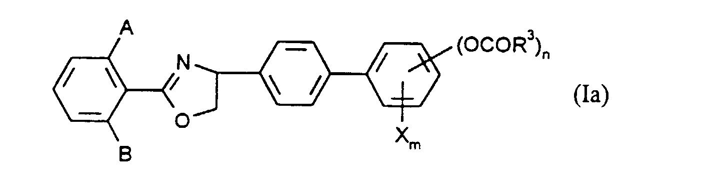 in which
A, B, R³, X, n and m have the abovementioned meaning
which are obtained when R = COR³
are hydrolyzed to give compounds of the formula (Ib) 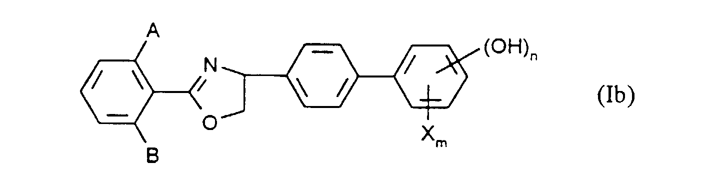 in which
A, B, X, n and m have the abovementioned meaning
and, if appropriate, these are subsequently
K
α) reacted with a compound of the formula (XIX)
HalCOR³ (XIX)
in which
R³ has the abovementioned meaning and
Hal represents halogen, if appropriate in the presence of a diluent, and if appropriate in the presence of a base, or
β) reacted with a compound of the formula (IV) 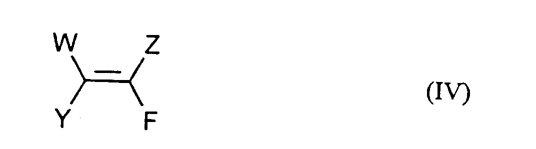 in which
W, Y and Z have the abovementioned meaning,
if appropriate in the presence of a base, if appropriate in the presence of a catalyst and if appropriate in the presence of a diluent and, if appropriate, the product is subsequently hydrogenated,
or
γ) reacted with a difluorohalogenomethane of the formula (V)
CHF₂Hal (V)
in which
Hal represents chlorine or bromine,
if appropriate in the presence of a base, if appropriate in the presence of a catalyst and if appropriate in the presence of a diluent,
or
δ) reacted with carbon tetrachloride in the presence of hydrofluoric acid, if appropriate in the presence of a diluent, or
ε) reacted with a compound of the formula (XX)
M-R² (XX)
in which
R² has the abovementioned meaning and
M represents a leaving group, if appropriate in the presence of a diluent and if appropriate in the presence of a base.

5. Compounds of the formula (II) 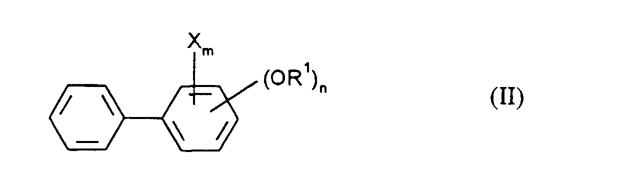 in which
R¹, X, m and n have the meaning given in Claim 1,
with the exception of the compounds 4-(2,2,2-trifluoroethoxy)biphenyl, 4-(1,1-difluoroethoxy)-biphenyl, 4-(1,1-difluoro-n-propoxy)biphenyl and 4-(2-fluoroethoxy)biphenyl.

6. Compounds of the formula (VII) 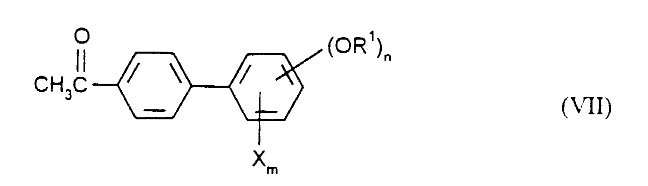 in which
X, R¹, m and n have the meaning given in Claim 1.

7. Compounds of the formula (VIII) 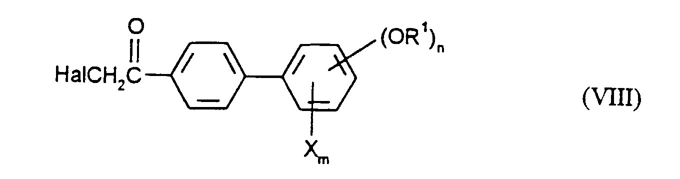 in which
X, R¹, m and n have the meaning given in Claim 1 and
Hal represents chlorine or bromine.

8. Compounds of the formula (X) 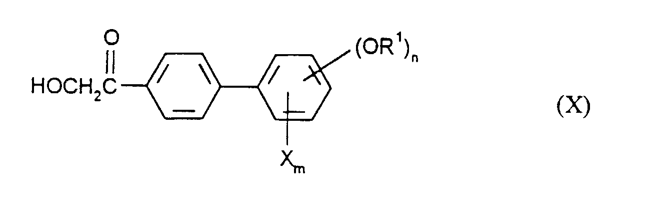 in which
X, R¹, m and n have the meaning given in Claim 1.

9. Compounds of the formula (XI) 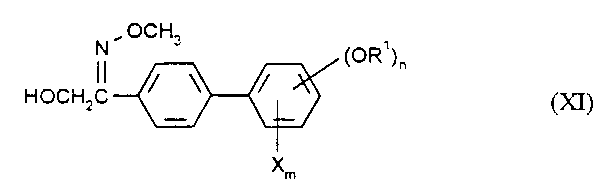 in which
X, R¹, m and n have the meaning given in Claim 1.

10. Compounds of the formula (XIII) 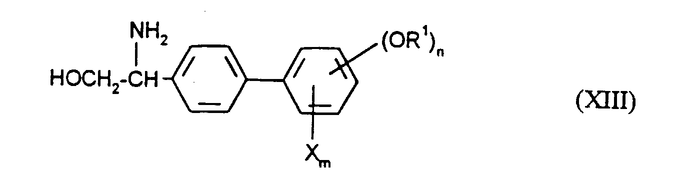 in which
R¹, X, m and n have the meaning given in Claim 1.

11. Compounds of the formula (XIV) 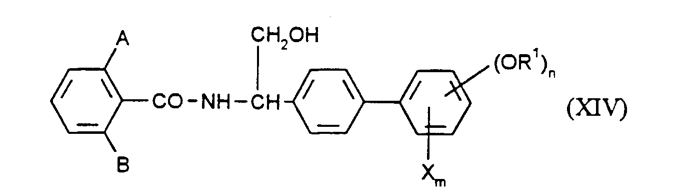 in which
A, B, R¹, X, m and n have the meaning given in Claim 1.

12. Compounds of the formula (XVI) 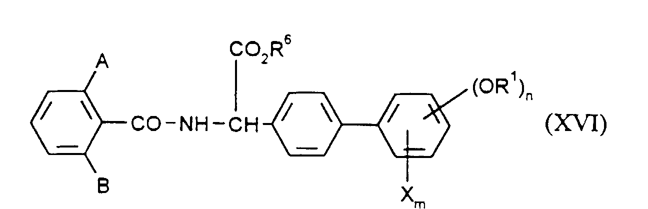 in which
A, B, R¹, X, m and n have the meaning given in Claim 1 and
R⁶ represents alkyl.

13. Compounds of the formula (XVII) 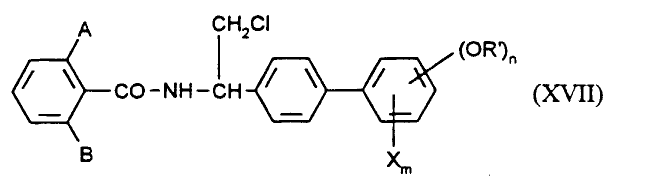 in which
A, B, X, m and n have the meaning given in Claim 1 and
R' represents R¹ or COR³, where
R¹ and R³ have the meaning given in Claim 1.

14. Pesticides, characterized in that they comprise at least one compound of the formula (I) according to Claim 1.

15. Use of compounds of the formula (I) according to Claim 1 for controlling pests.

16. Method of controlling pests, characterized in that compounds of the formula (I) according to Claim 1 are allowed to act on pests and/or their environment.

17. Process for the preparation of pesticides, characterized in that compounds of the formula (I) according to Claim 1 are mixed with extenders and/or surfactants.

18. Use of compounds of the formula (I) according to Claim 1 for the preparation of pesticides.

## Revendications

1. Composés de formule (I) 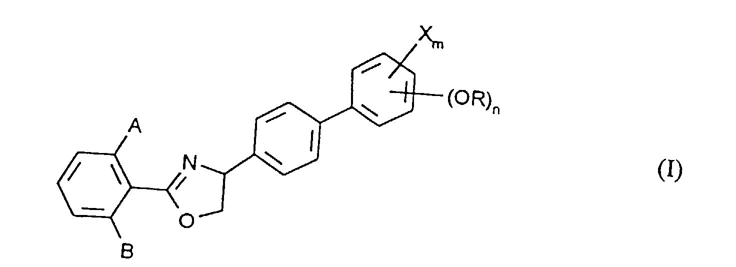 dans laquelle
A représente de l'hydrogène, du fluor ou du chlore,
B est du fluor ou du chlore,
R représente R¹ ou R²,
R¹ étant un groupe halogénalkyle en C₁ à C₆ ou halogénocycloalkyle en C₃ à C₆ ayant dans chaque cas au moins un atome de fluor et en outre au moins un atome d'hydrogène ou de chlore ou un groupe halogénocycloalcényle en C₄ à C₆ ayant au moins un atome de fluor,
R² représentant de l'hydrogène, un groupe alcényle en C₃ à C₁₂, alcynyle en C₃ à C₁₂,
un groupe cycloalkyle en C₃ à C₆ éventuellement substitué par un radical alkyle en C₁ à C₄, alcényle en C₂ à C₄, halogénalcényle en C₂ à C₄, phényle, halogénophényle, styryle ou halogénostyryle,
un groupe (cycloalkyle en C₃ à C₆)-(alkyle en C₁ à C₄) substitué, le cas échéant, par un halogène, un radical alkyle en C₁ à C₄, alcényle en C₂ à C₄, halogénalcényle en C₂ à C₄, phényle, halogénophényle, styryle ou halogénostyryle,
un groupe (cycloalcényle en C₄ à C₆)-(alkyle en C₁ à C₄) éventuellement substitué par un halogène ou un radical alkyle en C₁ à C₄,
un groupe cycloalcényle en C₄ à C₆ éventuellement substitué par un radical alkyle en C₁ à C₄,
un groupe phényl-(alkyle en C₁ à C₆), naphtyl-(alkyle en C₁ à C₃) ou tétrahydronaphtyl-(alkyle en C₁ à C₃) portant chacun, le cas échéant, 1 à 4 substituants, identiques ou différents, nitro, halogéno, alkyle en C₁ à C₁₂, halogénalkyle en C₁ à C₁₂, alkoxy en C₁ à C₁₂ ou halogénalkoxy en C₁ à C₁₂,
un groupe hétéroaryl-(alkyle en C₁ à C₄) pentagonal ou hexagonal ayant 1 ou 2 hétéro-atomes identiques ou différents de la série azote, oxygène et soufre, portant, le cas échéant, 1 ou 2 substituants, identiques ou différents, nitro, halogéno, alkyle en C₁ à C₁₂, halogénalkyle en C₁ à C₁₂, alkoxy en C₁ à C₁₂ ou halogénalkoxy en C₁ à C₁₂, ou le reste COR³,
dans lequel
R³ est un groupe alkyle en C₁ à C₁₂, alkoxy en C₁ à C₁₂, alcényle en C₃ à C₁₂, alcényloxy en C₃ à C₁₂, un groupe cycloalkyle en C₃ à C₁₀, cycloalkyloxy en C₃ à C₁₀ ou (cycloalkyle en C₃ à C₁₀)-(alkyloxy en C₁ à C₆) dont chacun est éventuellement substitué par un halogène, un radical alkyle en C₁ à C₄, halogénalkyle en C₁ à C₄, alcényle en C₂ à C₄, halogénalcényle en C₂ à C₄,
un groupe phényle ou naphtyle dont chacun porte, le cas échéant, 1 à 4 substituants, identiques ou différents, halogéno, alkyle en C₁ à C₁₂, halogénalkyle en C₁ à C₁₂, alkoxy en C₁ à C₁₂ ou halogénalkoxy en C₁ à C₁₂ ou le reste NR⁴R⁵
dans lequel
R⁴ est de l'hydrogène ou un groupe alkyle en C₁ à C₁₂ et
R⁵ est un groupe alkyle en C₁ à C₁₂, halogénalkyle en C₁ à C₁₂, un groupe cycloalkyle en C₃ à C₁₀ ou (cycloalkyle en C₃ à C₁₀)-(alkyle en C₁ à C₆) dont chacun est substitué, le cas échéant par un halogène, un radical alkyle en C₁ à C₄, halogénalkyle en C₁ à C₄ ou halogénalcényle en C₂ à C₄ ou un groupe phényle ou phényl-(alkyle en C₁ à C₆) dont chacun est substitué, le cas échéant, 1 à 4 fois identiques ou différentes par un halogène, un radical alkyle en C₁ à C₁₂, halogénalkyle en C₁ à C₁₂, alkoxy en C₁ à C₁₂, halogénalkoxy en C₁ à C₁₂,
X est un halogène, un groupe alkyle en C₁ à C₆ ou alkoxy en C₁ à C₆,
m a la valeur 0, 1 ou 2 et
n a la valeur 1 ou 2,
excepté la 2-(2,6-difluorophényl)-4-[4'-(2,2,2-trifluoréthoxy)-biphényl-4]-2-oxazoline et la 2-(2-chloro-6-fluorophényl)-4-[4'-(2,2,2-trifluoréthoxy)-biphényl-4]-2-oxazoline.

2. Composés de formule (I) suivant la revendication 1, dans lesquels
A est de l'hydrogène, du fluor ou du chlore,
B est du fluor ou du chlore,
R représente R¹ ou R²,
R¹ étant un groupe halogénalkyle en C₁ à C₃ ou halogénocycloalkyle en C₄ ou C₅ ayant chacun au moins un atome de fluor et en outre au moins un atome d'hydrogène ou de chlore ou un groupe halogénocycloalcényle en C₄ à C₆ ayant au moins un atome de fluor, et
R² étant de l'hydrogène, un groupe alcényle en C₃ à C₁₂, alcynyle en C₃ à C₅, un groupe cycloalkyle en C₃ à C₆ éventuellement substitué par un radical alkyle en C₁ à C₄, alcényle en C₂ à C₄, halogénalcényle en C₂ ou C₃, phényle, halogénophényle, styryle ou halogénostyryle,
un groupe (cycloalkyle en C₃ à C₆)-(alkyle en C₁ à C₄) éventuellement substitué par un halogène, un radical alkyle en C₁ à C₄, alcényle en C₂ à C₄, halogénalcényle en C₂ ou C₃, phényle, halogénophényle, styryle ou halogénostyryle,
un groupe (cycloalcényle en C₄ à C₆)-(méthyle) éventuellement substitué par un halogène,
un groupe cycloalcényle en C₄ à C₆,
un groupe phényl-(alkyle en C₁ à C₆), naphtylméthyle, tétrahydronaphtylméthyle, pyridyl-, furanyl-, thiazolyl-, oxazolyl- ou isoxazolyl-(alkyle en C₁ à C₃) dont chacun est éventuellement substitué 1 ou 2 fois identiques ou différentes par un radical nitro, halogéno, alkyle en C₁ à C₄, halogénalkyle en C₁ à C₄, alkoxy en C₁ à C₄ ou halogénalkoxy en C₁ à C₄, ou le reste COR³,
R³ est un groupe alkyle en C₁ à C₆, alkoxy en C₁ à C₆, alcényle en C₃ à C₆, alcényloxy en C₃ à C₆, un groupe cycloalkyle en C₃ à C₆, cycloalkyloxy en C₃ à C₆ ou (cycloalkyle en C₃ à C₆)-(alkyloxy en C₁ ou C₂) dont chacun est éventuellement substitué par du fluor, du chlore, un radical alkyle en C₁ à C₃, halogénalkyle en C₁ ou C₂, halogénalcényle en C₂ ou C₃,
un groupe phényle portant, le cas échéant, un ou deux substituants identiques ou différents, halogéno, alkyle en C₁ à C₄, halogénalkyle en C₁ à C₃, alkoxy en C₁ à C₄ ou halogénalkoxy en C₁ à C₄, ou le reste NHR⁵,
R⁵ est un groupe alkyle en C₁ à C₄ ou représente un groupe phényle ou benzyle dont chacun est éventuellement substitué une ou deux fois identiques ou différentes par du fluor, du chlore, du brome, un radical alkyle en C₁ à C₄, halogénalkyle en C₁ à C₄ ou halogénalkoxy en C₁ à C₄,
X représente du fluor, du chlore, du brome,
m a la valeur 0 ou 1 et
n est égal à 1,
excepté la 2-(2,6-difluorophényl)-4-[4'-(2,2,2-trifluoréthoxy)-biphényl-4]-2-oxazoline et la 2-(2-chloro-6-fluorophényl)-4-[4'-(2,2,2-trifluoréthoxy)-biphényl-4]-2-oxazoline.

3. Composés de formule (I) suivant la revendication 1, dans lesquels
A représente de l'hydrogène, du fluor ou du chlore,
B est du fluor ou du chlore,
R représente R¹ ou R²,
R¹ étant l'un des groupes -CHF₂, -CClF₂,
-CF₂CHFCl,-CF₂CH₂F, -CF₂CHF_{2,} -CF₂CCl₃, -CF₂CHFCF₃,
-CH₂CF₃, -CH₂CF₂CHF₂, -CH₂CF₂CF₃, 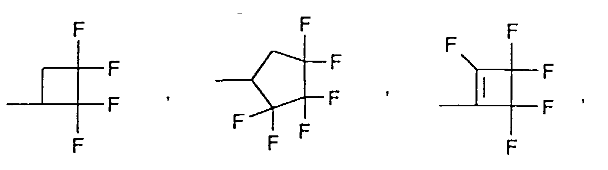 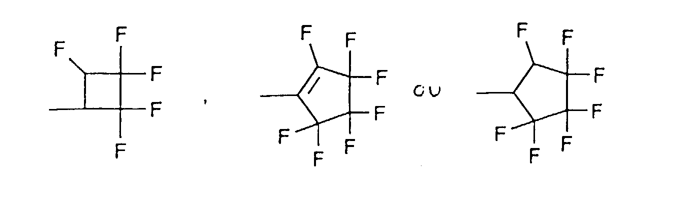 et
R² représentant de l'hydrogène, un groupe propényle, butényle, pentényle, hexényle, propynyle, butynyle, pentynyle,
l'un des groupements cycloalkylalkyle : 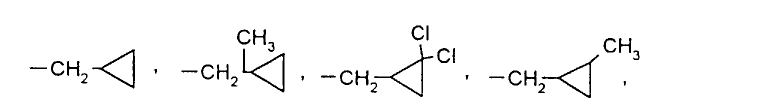 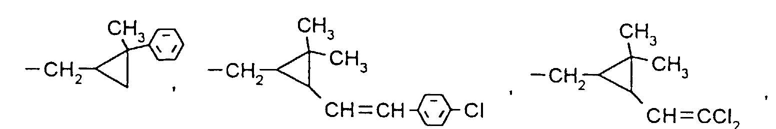 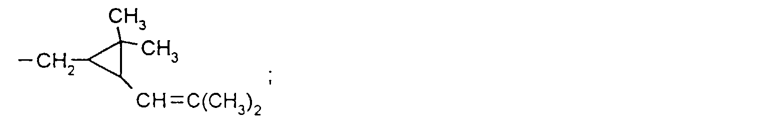 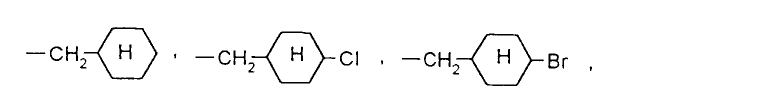 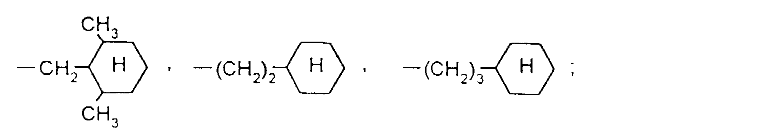 le groupement cycloalcénylalkyle 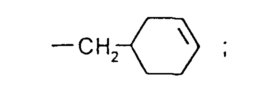 l'un des groupements phénylalkyle : 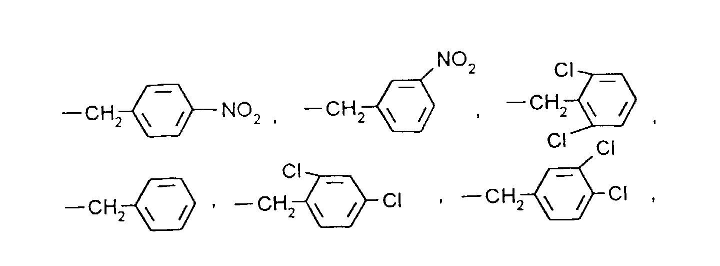 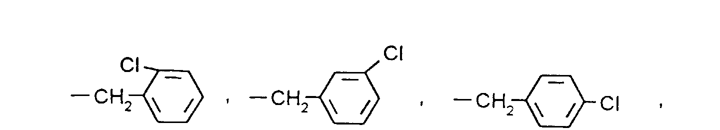 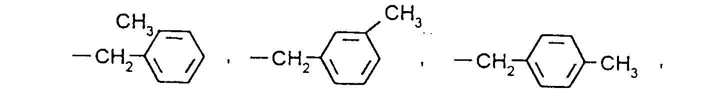 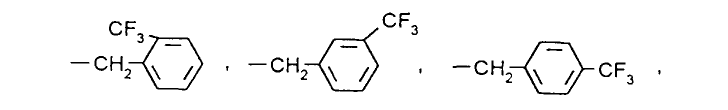 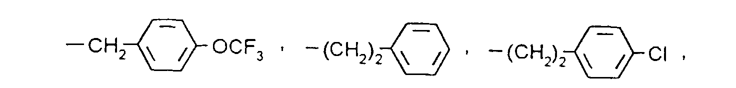 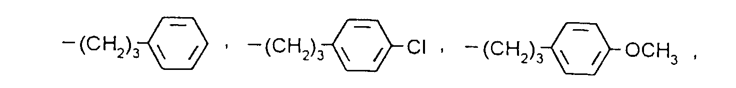 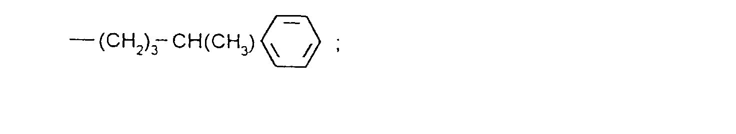 un groupement 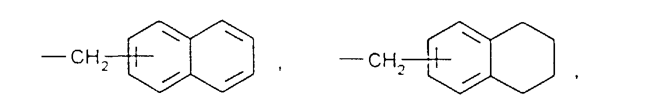 l'un des groupements hétéroarylalkyle : 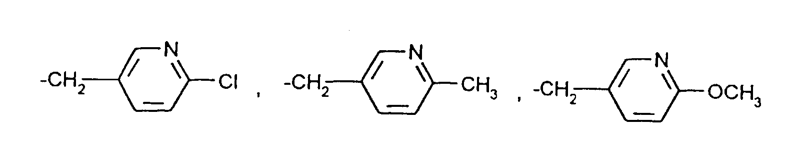 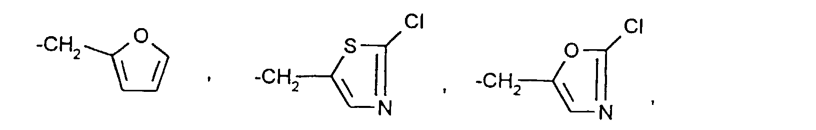 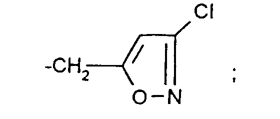 ou le reste -COR³,
R³ est un groupe méthyle, éthyle, propyle ;
un groupe méthoxy, éthoxy, propoxy, butoxy ;
un groupe cyclopropyle, cyclohexyle ;
un groupe cyclohexyloxy ;
un groupe phényle, 2-chlorophényle, 3-chlorophényle, 2,6-difluorophényle, 2-trifluorométhoxyphényle, 4-trifluorométhoxyphényle, 2,4-dichlorophényle, 3,4-dichlorophényle ;
ou le reste -NHR⁵,
R⁵ est un groupe méthyle, éthyle ou un groupe phényle éventuellement substitué une fois par du chlore,
X est du fluor, du chlore ou du brome,
m a la valeur 0 ou 1 et
n est égal à 1,
excepté la 2-(2,6-difluorophényl)-4-[4'-(2,2,2-trifluoréthoxy)-biphényl-4]-2-oxazoline et la 2-(2-chloro-6-fluorophényl)-4-[4'-(2,2,2-trifluoréthoxy)-biphényl-4]-2-oxazoline.

4. Procédé de production des composés de formule (I) suivant la revendication 1, caractérisé en ce que
A) dans une première étape pour l'obtention de composés de formule (II) 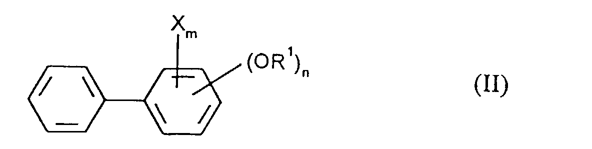 dans laquelle
R¹, X, m et n on la définition indiquée dans la revendication 1,
α) pour l'obtention de composés de formule (IIa) 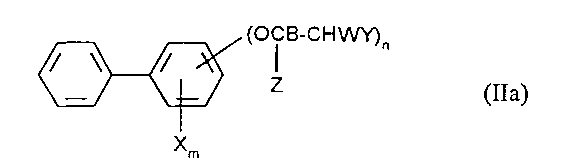 dans laquelle
W et Y représentent, indépendamment l'un de l'autre, du fluor, du chlore ou un groupe trifluorométhyle,
B est de l'hydrogène ou du fluor,
Z est du fluor ou
W et Z forment ensemble un groupe -(CF₂)₁,
dans lequel
l a la valeur 2, 3 ou 4,
n a la valeur ou 2 et
m a la valeur 0, 1 ou 2,
on fait réagir un hydroxybiphényle de formule (III) 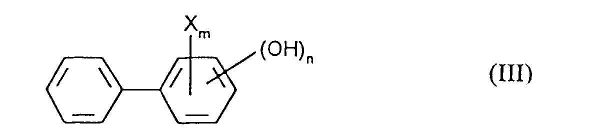 dans laquelle
X, m et n ont la définition indiquée ci-dessus, avec un composé de formule (IV) 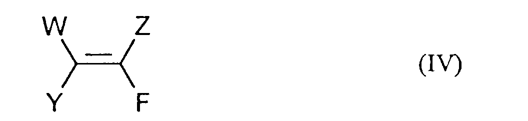 dans laquelle
W, Y et Z ont la définition indiquée ci-dessus, le cas échéant en présence d'une base, en la présence éventuelle d'un catalyseur et, le cas échéant, en présence d'un diluant, puis on hydrogène éventuellement le produit de réaction,
ou bien
β) pour obtenir des composés de formule (IIb) 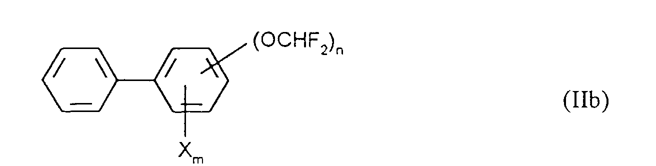 dans laquelle
X, m et n ont la définition indiquée ci-dessus, on fait réagir des hydroxybiphényles de la formule (III) indiquée ci-dessus avec un difluorhalogénométhane de formule (V)
CHF₂Hal (V)
dans laquelle
Hal représente du chlore ou du brome,
le cas échéant en présence d'une base, éventuellement en présence d'un catalyseur et, le cas échéant, en présence d'un diluant,
ou bien
γ) pour obtenir des composés de formule (IIc) 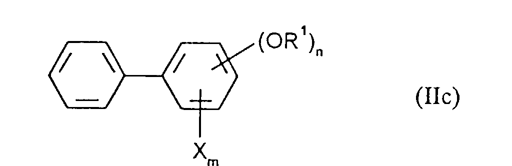 dans laquelle
X, R¹, m et n ont la définition indiquée ci-dessus,
on diazote des dérivés d'aminophénol de formule (VI) 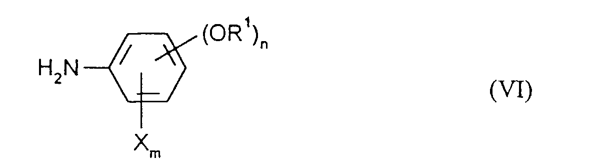 dans laquelle
X, R¹, m et n ont la définition indiquée ci-dessus,
et on fait réagir le sel de diazonium formé avec du benzène en présence d'un acide et de fer en poudre ou en présence d'une base et dans chaque cas, éventuellement, en présence d'un diluant,
ou bien
δ) pour obtenir des composés de formule (IId) 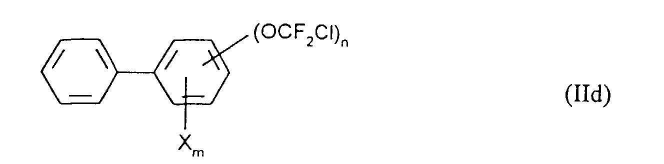 dans laquelle
X, m et n ont la définition indiquée ci-dessus,
on fait réagir un hydroxybiphényle de la formule (III) indiquée ci-dessus avec du tétrachlorure de carbone en présence d'acide fluorhydrique, éventuellement en présence d'un diluant,
B) dans une seconde étape, pour l'obtention de composés de formule (VII) 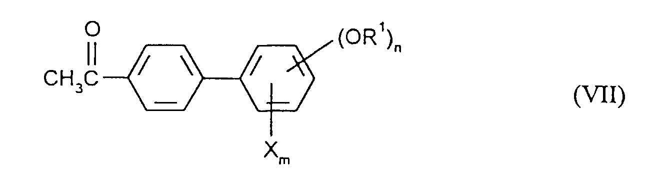 dans laquelle
X, R¹, m et n ont la définition indiquée ci-dessus,
α) on fait réagir avec le chlorure d'acétyle les composés de formule (II) obtenus selon le procédé A) en présence d'un acide ou d'un acide de Lewis et, le cas échéant, d'un diluant,
ou bien
β) pour obtenir des composés de formule (VIIa) 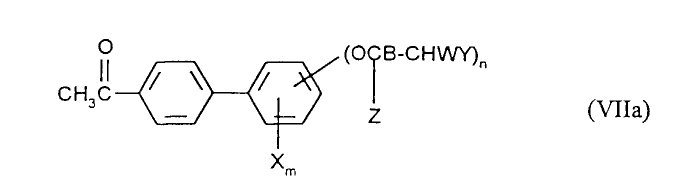 dans laquelle
B, X, m, n, W, Y et Z ont la définition indiquée ci-dessus,
on fait réagir un dérivé d'hydroxybiphényle de formule (IIIa) 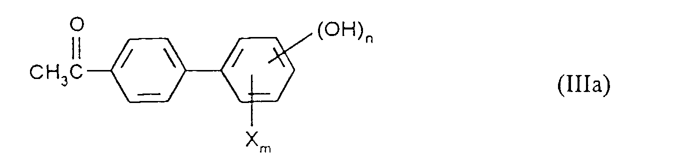 dans laquelle
X, m et n ont la définition indiquée ci-dessus, avec un composé de formule (IV) 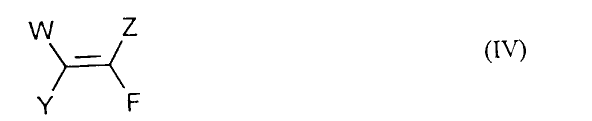 dans laquelle
W, Y et Z ont la définition indiquée ci-dessus,
le cas échéant en présence d'une base, en la présence éventuelle d'un catalyseur et, le cas échéant, en présence d'un solvant, puis on hydrogène éventuellement le produit de réaction,
ou bien
γ) pour obtenir des composés de formule (VIIb) 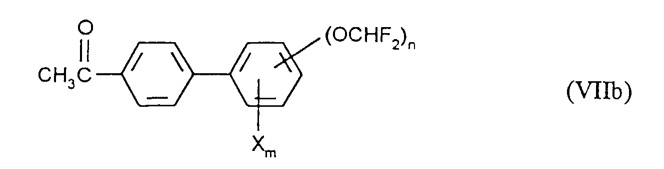 dans laquelle
X, m et n ont la définition indiquée ci-dessus,
on fait réagir un dérivé d'hydroxybiphényle de formule (IIIa) indiquée ci-dessus avec un difluorhalogénométhane de formule (V) indiquée ci-dessus, le cas échéant en présence d'une base, éventuellement en présence d'un catalyseur et en la présence éventuelle d'un diluant,
C) dans une troisième étape, pour obtenir des composés de formule (VIII) 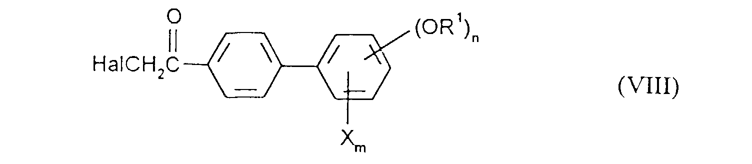 dans laquelle
X, R¹, m et n ont la définition indiquée ci-dessus et
Hal représente du chlore ou du brome,
α) on chlore ou on brome les composés, obtenus selon le procédé B), de formule (VIII) indiquée ci-dessus, le cas échéant en présence d'un diluant,
ou bien
β) on fait réagir les composés de formule (II) indiquée ci-dessus, obtenus selon le procédé A), avec des chlorures d'halogénacétyle de formule (IX)
HalCH₂COCl (IX)
dans laquelle
Hal représente du chlore ou du brome,
en présence d'un acide ou d'un acide de Lewis et en présence d'un diluant,
D) dans une quatrième étape, pour obtenir des composés de formule (X) 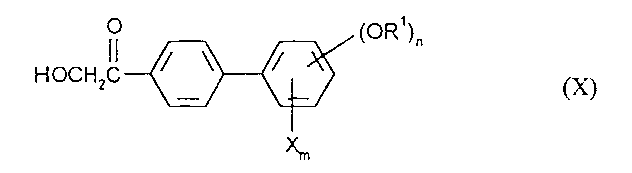 dans laquelle
X, R¹, m et n ont la définition indiquée ci-dessus,
on fait réagir les composés, obtenus selon le procédé C), de formule (VIII) indiquée ci-dessus avec un sel d'acide formique, éventuellement en présence d'un catalyseur,
E) dans une cinquième étape, pour obtenir des composés de formule (XI) 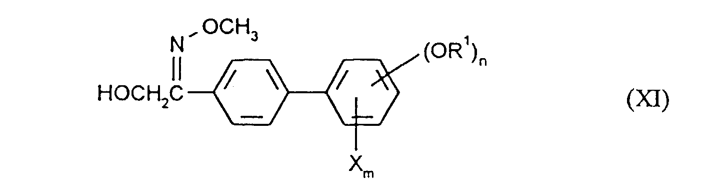 dans laquelle
X, R¹, m et n ont la définition indiquée ci-dessus,
on fait réagir les composés, obtenus selon le procédé D), de formule (X) indiquée ci-dessus,
avec le composé de formule (XII)
H₂N-OCH₃ (XII)
le cas échéant en présence d'un diluant,
F) dans une sixième étape pour obtenir des composés de formule (XIII) 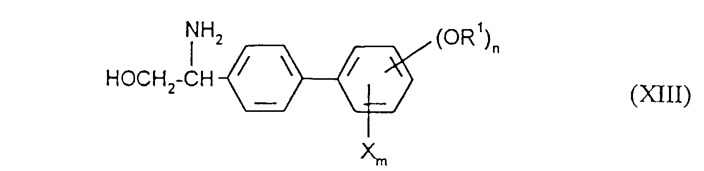 dans laquelle
R¹, X, m et n ont la définition indiquée ci-dessus,
on réduit les composés, obtenus selon le procédé E), de formule (XI) indiquée ci-dessus avec un agent réducteur en présence d'un acide et, le cas échéant, en présence d'un diluant,
G) dans une septième étape, pour obtenir des composés de formule (XIV) 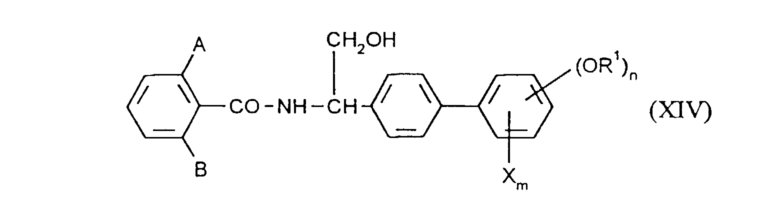 dans laquelle
A, B, R¹, X, m et n ont la définition indiquée ci-dessus,
α) on fait réagir les composés, obtenus selon le procédé F), de formule (XIII) indiquée ci-dessus avec un chlorure de 2-A,6-B-benzoyle, éventuellement en présence d'une base et, le cas échéant en présence d'un diluant
ou bien
β) on fait réagir tout d'abord les composés, obtenus selon le procédé A), de formule (II) indiquée ci-dessus avec un composé de formule (XV) 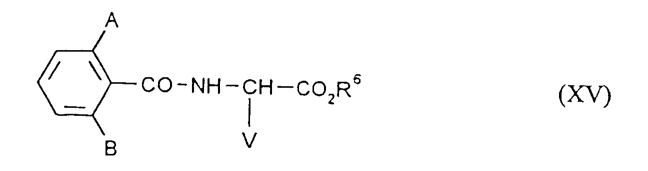 dans laquelle
A et B ont la définition indiquée ci-dessus,
V représente du chlore, un groupe hydroxy ou alkoxy en C₁ à C₄ et
R⁶ est de l'hydrogène ou un groupe alkyle,
en présence d'un catalyseur acide et, le cas échéant, en présence d'un diluant et
on réduit les composés ainsi obtenus de formule (XVI) 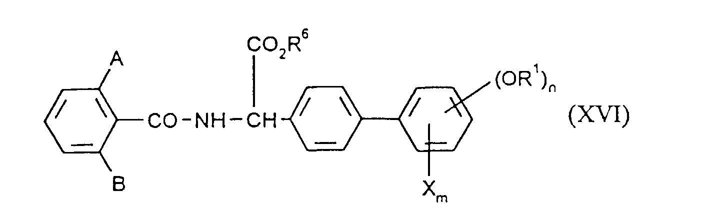 dans laquelle
A, B, R¹ et R⁶ ont la définition indiquée ci-dessus, en présence d'un agent réducteur et en présence d'un diluant,
H) dans une huitième étape pour l'obtention de composés de formule (XVII) 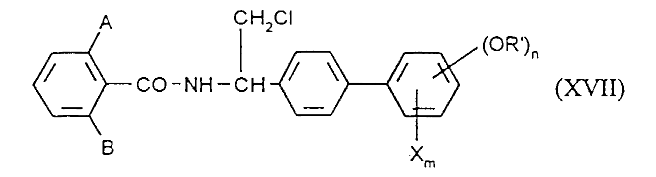 dans laquelle
A, B, X, m et n ont la définition indiquée ci-dessus,
R' représente R¹ ou un groupe COR³, où
R¹ et R³ ont la définition indiquée dans la revendication 1
α) on fait réagir les composés, obtenus selon le procédé G), de formule (XIV) indiquée ci-dessus avec un agent de chloration, éventuellement en présence d'un diluant
ou bien
β) on fait réagir les composés, obtenus selon le procédé A), de formule (II) indiquée ci-dessus avec un composé de formule (IIe) 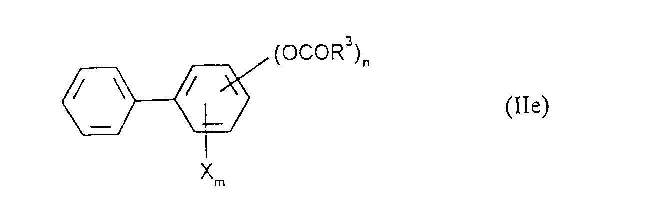 dans laquelle
R³, X, n et m ont la définition indiquée ci-dessus,
avec un composé de formule (XVIII) 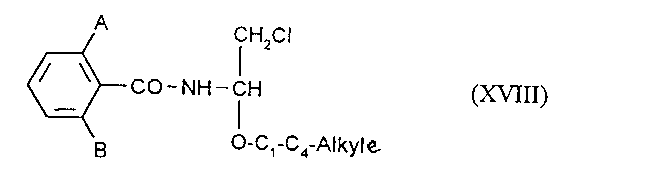 dans laquelle
A et B ont la définition indiquée ci-dessus
en présence d'un catalyseur acide et, le cas échéant, en présence d'un diluant,
et
I) dans une neuvième étape, on cyclise les composés, obtenus selon le procédé H), de formule (XVII) indiquée ci-dessus en présence d'une base, le cas échéant en présence d'un catalyseur et en la présence éventuelle d'un diluant,
et le cas échéant
J) on saponifie les composés que l'on obtient alors pour R = COR³, de formule (Ia) 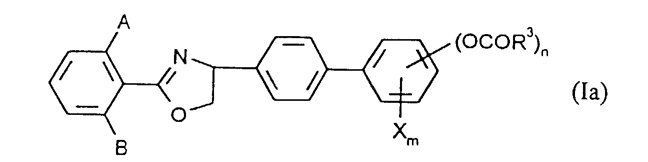 dans laquelle
A, B, R³, X, n et m ont la définition indiquée ci-dessus,
en composés de formule (Ib) 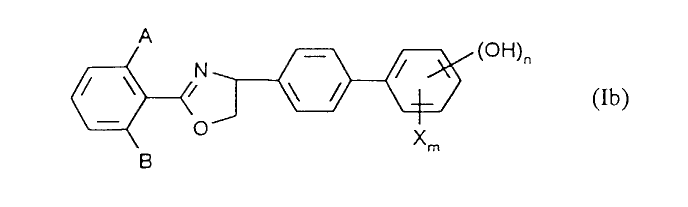 dans laquelle
A, B, X, n et m ont la définition indiquée ci-dessus,
puis, le cas échéant,
K
α) on fait réagir ces composés avec un composé de formule (XIX)
HalCOR³ (XIX)
dans laquelle
R³ a la définition indiquée ci-dessus et
Hal représente un halogène, le cas échéant en présence d'un diluant et en la présence éventuelle d'une base, ou bien
β) on les fait réagir avec un composé de formule (IV) 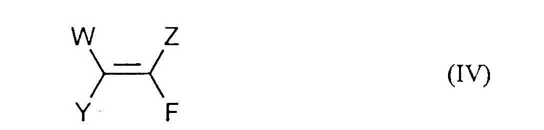 dans laquelle
W, Y et Z ont la définition indiquée ci-dessus,
le cas échéant en présence d'une base, en la présence éventuelle d'un catalyseur et, le cas échéant, en présence d'un diluant, puis on hydrogène éventuellement le produit de réaction,
ou bien
γ) on les fait réagir avec un difluorhalogénométhane de formule (V)
CHF₂Hal (V)
dans laquelle
Hal représente du chlore ou du brome,
éventuellement en présence d'une base, le cas échéant en présence d'un catalyseur et la présence éventuelle d'un diluant,
ou bien
δ) on les fait réagir avec du tétrachlorure de carbone en présence d'acide fluorhydrique, le cas échéant en présence d'un diluant, ou bien
ε) on les fait réagir avec un composé de formule (XX)
M-R² (XX)
dans laquelle
R² a la définition indiquée ci-dessus et
M représente un groupe partant, éventuellement en présence d'un diluant et en la présence éventuelle d'une base.

5. Composés de formule (II) 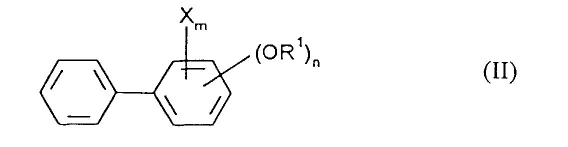 dans laquelle
R¹, X, m et n ont la définition indiquée dans la revendication 1,
excepté les composés 4-(2,2,2-trifluoréthoxy)biphényle, 4-(1,1-difluoréthoxy)biphényle, 4-(1,1-difluoro-n-propoxy)biphényle et 4-(2-fluoréthoxy)biphényle.

6. Composés de formule (VII) 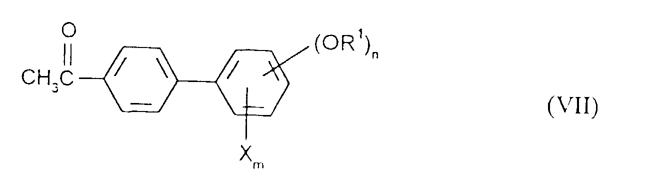 dans laquelle
X, R¹, m et n ont la définition indiquée dans la revendication 1.

7. Composés de formule (VIII) 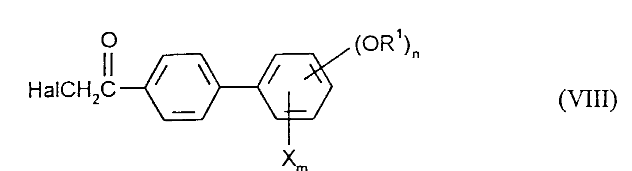 dans laquelle
X, R¹, m et n ont la définition indiquée dans la revendication 1 et
Hal représente du chlore ou du brome.

8. Composés de formule (X) 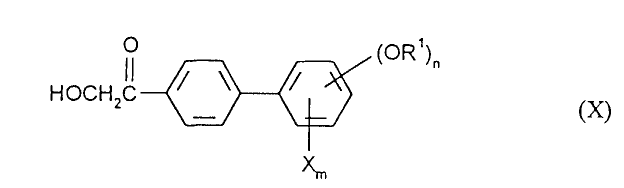 dans laquelle
X, R¹, m et n ont la définition indiquée dans la revendication 1.

9. Composés de formule (XI) 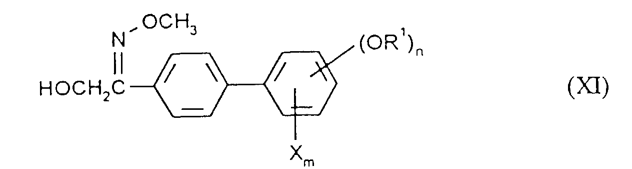 dans laquelle
X, R¹, m et n ont la définition indiquée dans la revendication 1.

10. Composés de formule (XIII) 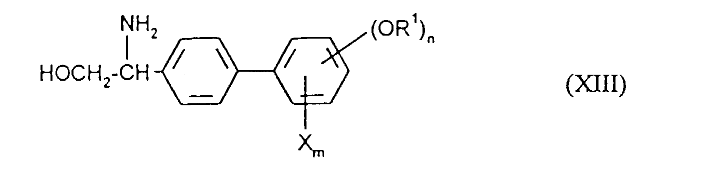 dans laquelle
R¹, X, m et n ont la définition indiquée dans la revendication 1.

11. Composés suivant la revendication (XIV) 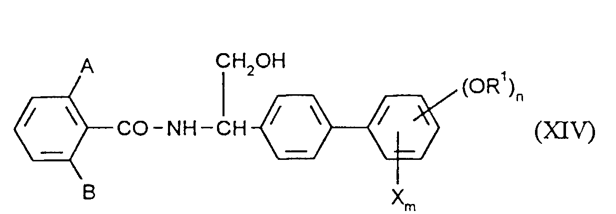 dans laquelle
A, B, R¹, X, m et n ont la définition indiquée dans la revendication 1.

12. Composés de formule (XVI) 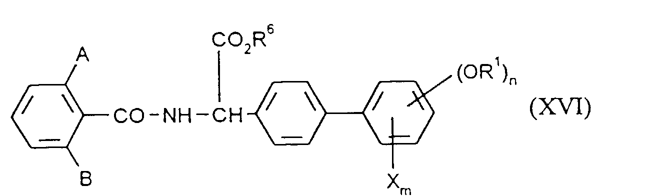 dans laquelle
A, B, R¹, X, m et n ont la définition indiquée dans la revendication 1 et
R⁶ est un groupe alkyle.

13. Composés de formule (XVII) 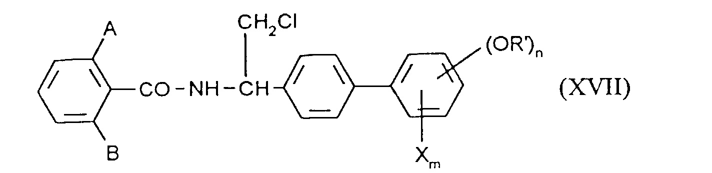 dans laquelle
A, B, X, m et n ont la définition indiquée dans la revendication 1,
R' représente R¹ ou COR³, où
R¹ et R³ ont la définition indiquée dans la revendication 1.

14. Compositions pesticides, caractérisées par une teneur en au moins un composé de formule (I) suivant la revendication 1.

15. Utilisation de composés de formule (I) suivant la revendication 1 pour combattre des parasites.

16. Procédé pour combattre des parasites, caractérisé en ce qu'on fait agir des composés de formule (I) suivant la revendication 1 sur des parasites et/ou sur leur milieu.

17. Procédé de préparation de compositions pesticides, caractérisé en ce qu'on mélange des composés de formule (I) suivant la revendication 1 avec des diluants et/ou des agents tensio-actifs.

18. Utilisation de composés de formule (I) suivant la revendication 1 pour la préparation de compositions pesticides.
